# EUROPEAN PATENT SPECIFICATION

(11) **EP 1 327 145 B1**
(45) Date of publication and mention of the grant of the patent: **11.03.2009**
(21) Application number: 01986346.3
(22) Date of filing: 05.10.2001
(51) Int. Cl.: G01N 33/53

(54) **CELLS HAVING A SPECTRAL SIGNATURE, AND METHODS OF PREPARATION AND USE THEREOF**
ZELLEN MIT SPEKTRALER SIGNATUR SOWIE VERFAHREN ZU IHRER HERSTELLUNG UND NUTZUNG
CELLULES DOTEES D'UNE SIGNATURE SPECTRALE ET PROCEDES DE PREPARATION ET UTILISATION DESDITES CELLULES

(30) Priority: 06.10.2000 US 238677 P; 15.08.2001 US 312558 P
(43) Date of publication of application: 16.07.2003
(73) Proprietor: Life Technologies Corporation, Carlsbad, CA 92008 (US)
(72) Inventor: BRUCHEZ, Marcel, P., Fremont, CA 94536 (US); DANIELS, R., Hugh, Mountain View, CA 94040 (US); DIAS, Jennifer, Dublin, CA 94568 (US); MATTHEAKIS, Larry, C., Cupertino, CA 95014 (US); LIU, Jianquan, Fremont, CA 94538 (US)
(74) Representative: Couchman, Jonathan Hugh
(86) International application number: PCT/US2001/031410
(87) International publication number: WO 2002/029410

(56) References cited:
- EP-A- 0 990 903
- US-A- 5 990 479
- US-A- 6 051 386
- BRUCHEZ M JR ET AL: "Semiconductor nanocrystals as fluorescent biological labels" SCIENCE, AMERICAN ASSOCIATION FOR THE ADVANCEMENT OF SCIENCE,, US, vol. 281, 25 September 1998 (1998-09-25), pages 2013-2016, XP002125872 ISSN: 0036-8075
- CHAN WCW ET AL: "Quantum Dot bioconjugates for ultrasensitive nonisotopic detection" SCIENCE, AMERICAN ASSOCIATION FOR THE ADVANCEMENT OF SCIENCE,, US, vol. 281, 25 September 1998 (1998-09-25), pages 2016-2018, XP002125871 ISSN: 0036-8075
- LACOSTE T D ET AL: "SUPER RESOLUTION MOLECULAR RULER USING SINGLE QUANTUM DOTS" BIOPHYSICAL JOURNAL, NEW YORK, US, US, vol. 78, January 2000 (2000-01), page 402A XP000933548 ISSN: 0006-3495

## Description

### TECHNICAL FIELD

The application relates to semiconductor nanocrystal probes for biological applications, and methods of screening modulators of receptors using encoded cells.

### CROSS REFERENCE TO RELATED APPLICATIONS

### BACKGROUND OF THE INVENTION

Multiplexed assay formats are necessary to meet the demands of today's high-throughput screening methods, and to match the demands that combinatorial chemistry is putting on the established discovery and validation systems for pharmaceuticals. In addition, the ever-expanding repertoire of genomic information is rapidly necessitating very efficient, parallel and inexpensive assay formats. The requirements for all of these multiplexed assays are ease of use, reliability of results, a high-throughput format, and extremely fast and inexpensive assay development and execution.

For these high-throughput techniques, a number of assay formats are currently available. Each of these formats has limitations, however. By far the most dominant high-throughput technique is based on the separation of different assays into different regions of space. The 96-well plate format is the workhorse in this arena. In 96-well plate assays, the individual wells (which are isolated from each other by walls) are charged with different components, the assay is performed and then the assay result in each well measured. The information about which assay is being run is carried with the well number, or the position on the plate, and the result at the given position determines which assays are positive. These assays can be based on chemiluminescence, scintillation, fluorescence, absorbance, scattering, or colorimetric measurements, and the details of the detection scheme depend on the reaction being assayed. Assays have been reduced in size to accommodate 1536 wells per plate, though the fluid delivery and evaporation of the assay solution at this scale are significantly more problematic. High-throughput formats based on multi-well arraying require complex robotics and fluid dispensing systems to function optimally. The dispensing of the appropriate solutions to the appropriate bins on the plate poses a challenge from both an efficiency and a contamination standpoint, and pains must be taken to optimize the fluidics for both properties. Furthermore, the throughput is ultimately limited by the number of wells that one can put adjacent on a plate, and the volume of each well. Arbitrarily small wells have arbitrarily small volumes, resulting in a signal that scales with the volume, shrinking proportionally to R³. The spatial isolation of each well, and thereby each assay, comes at the cost of the ability to run multiple assays in a single well. Such single-well multiplexing techniques are not widely used, due in large part to the inability to "demultiplex" or resolve the results of the different assays in a single well. However, such multiplexing would obviate the need for high-density well assay formats.

Each of the current techniques for ultra-high-throughput assay formats suffers from severe limitations. The present invention relates to methods for encoding spectra, which are readable with a single light source for excitation, into cells, which can be used in highly multiplexed assays.

The methods of the invention for encoding spectra can be used, for example, for screening for drug candidates, such as agonists or antagonists of receptors, for identifying new receptors, or for obtaining functional information pertaining to receptors, such as orphan G-protein coupled receptors (GPCRs). GPCRs represent one of the most important families of drug targets. G protein-mediated signaling systems have been identified in many divergent organisms, such as mammals and yeast. GPCRs respond to, among other extracellular signals, neurotransmitters, hormones, odorants and light. GPCRs are thought to represent a large superfamily of proteins that are characterized by the seven distinct hydrophobic regions, each about 20-30 amino acids in length, that forms the transmembrane domain. The amino acid sequence is not conserved across the entire superfamily, but each phylogenetically related subfamily contains a number of highly conserved amino acid motifs that can be used to identify and classify new members. Individual GPCRs activate particular signal transduction pathways, although at least ten different signal transduction pathways are known to be activated via GPCRs. For example, the beta 2-adrenergic receptor (pAR) is a prototype mammalian GPCR. In response to agonist binding, βAR receptors activate a G protein (Gₛ) which in turn stimulates adenylate cyclase and cyclic adenosine monophosphate production in the cell.

It has been postulated that members of the GPCR superfamily desensitize via a common mechanism involving G protein-coupled receptor kinase (GRK) phosphorylation followed by arrestin binding. The protein β-arrestin regulates GPCR signal transduction by binding agonist-activated receptors that have been phosphorylated by G protein receptor kinases. The β-arrestin protein remains bound to the GPCR during receptor internalization. The interaction between a GPCR and β-arrestin can be measured using several methods. In one example, the β-arrestin protein is fused to green fluorescent protein to create a protein fusion (Barak et al. (1997) J. Biol. Chem. 272(44):27497-500). The agonist-dependent binding of β-arrestin to a GPCR can be visualized by fluorescence microscopy. Microscopy can also be used to visualize the subsequent trafficking of the GPCR/β-arrestin complex to clathrin coated pits. Other methods for measuring binding of β-arrestin to a GPCR in live cells include techniques such as FRET (fluorescence resonance energy transfer), BRET (bioluminescent energy transfer) or enzyme complementation (Rossi et al. (1997) Proc. Natl Acad. Sci. USA 94(16):8405-10).

At present, there are nearly 400 GPCRs whose natural ligands and function are known. These known GPCRs, named for their endogenous ligands, have been classified into five major categories: Class-A Rhodopsin-like; Class-B Secretin-like; Class-C Metabotropic glutamate/pheromone; Class-D Fungal pheromone; Class-E cAMP (dictyostelium). Representative members of Class-A are the amine receptors (e.g., muscarinic, nicotinic, adrenergic, adenosine, dopamine, histamine and serotonin), the peptide receptors (e.g., angiotensin, bradykinin, chemokines, endothelin and opioid), the hormone receptors (e.g., follicle stimulating, lutropin and thyrotropin), and the sensory receptors, including rhodopsin (light), olfactory (smell) and gustatory (taste) receptors. Representatives of Class-B include secretin, calcitonin, gastrin and glucagon receptors. Much less is known about Classes C-E.

Many available therapeutic drugs in use today target GPCRs, as they mediate vital physiological responses, including vasodilation, heart rate, bronchodilation, endocrine secretion, and gut peristalsis (Wilson and Bergsma (2000) Phar. News 7: 105-114). For example, ligands to β-adrenergic receptors are used in the treatment of anaphylaxis, shock, hypertension hypotension, asthma and other conditions. Additionally, diseases can be caused by the occurrence of spontaneous activation of GPCRs, where a GPCR cellular response is generated in the absence of a ligand. Drugs that are antagonists of GPCRs decrease this spontaneous activity (a process known as inverse agonism) are important therapeutic agents. Examples of commonly prescribed GPCR-based drugs include Atenolon (Tenormin^{®}), Albuterol (Ventolin^{®}), Ranitidine (Zantac^{®}), Loratadine (Claritin^{®}), Hydrocodone (Vicodin^{®}), Theophylline (TheoDur^{®}), and Fluoxetine (Prozac).

Due to the therapeutic importance of GPCRs, methods for the rapid screening of compounds for GPCR ligand activity are desirable. Additionally, there is a need for methods of screening orphan GPCRs for interactions with known and putative GPCR ligands in order to characterize such receptors. The present invention meets these and other needs.

Bruchez M, Jr. et al., Science 281, 2013-2016 (1998) describe the use of semiconductor nanocrystals as fluorescent probes in biological staining and diagnostics. In order to establish the utility of nanocrystals for biological staining, 3T3 mouse fibroblast cells were fluorescently labelled using two different CdSe-CdS core-shell nanocrystals enclosed in a silica shell. The two nanocrystal types respectively emitted green and red fluorescence. In contrast to conventional multicolour dye imaging, both colours were seen at the same time. The red and green labels were clearly spectrally resolved to the eye and to a colour Polaroid^{®} camera.

### SUMMARY OF THE INVENTION

Methods for encoding cells with semiconductor nanocrystals are provided. In one aspect, a method is provided comprising the ability to separately identify individual populations of cells in a mixture of different types of cells which is highly advantageous for many applications. This method is especially useful for identifying a population of cells derived from an initial sample of one or more cells via its unique spectral code after several cell divisions. The method facilitates analysis of many otherwise identical cells which only differ by the presence or absence of one or more genes and which are subjected to a functional assay.

The ability to detect populations of cells derived from a few precursors by virtue of their spectral code greatly facilitates the high-throughput analysis of many systems. It allows the identification of populations that have multiplied in a particular environment in the absence of any further experimental processing. The number of cells bearing the diluted code can be determined using various spectral scanning devices.

Many specific binding interactions can only occur when at least one of the binding partners is In its 'natural' environment. This environment is often the membrane of a cell. Therefore to have a method to simultaneously interrogate multiple populations of cell that are of different lineages or are expressing different binding partners for a molecule of interest requires an ability to separately encode those cells. This invention describes a method by which this is done using SCNCs, and combinations of SCNCs with other fluorescent species, or otherwise detectable species. This is useful in, for example, high throughput cell based screening systems. One example is the analysis of G-protein coupled receptors and their binding partners - these receptors span lipid bilayers 7 times and can only bind their partners when in this conformation.

The invention provides a method of distinguishably identifying a population of cells in a mixture of different types of cells, comprising: providing a cell, contacting the cell with a semiconductor nanocrystal (SCNC) under conditions in which the semiconductor nanocrystal is associated with the cell to provide a distinguishably labeled cell, and identifying in a mixture of different types of cells a population of cells derived from the labeled cell via their unique spectral code after it has multiplied in the mixture.

The invention further provides a method of identifying a cell in a mixed population of cells, comprising mixing a composition comprising a cell encoded with a semiconductor nanocrystal (SCNC) with a cell distinct therefrom to form a mixed population, culturing the mixed population, such that there is derived from the encoded cell a population of cells after several cell divisions, applying an excitation source to the mixed population, and detecting the SCNC via its unique spectral code to identify the encoded cells.

The methods are particularly useful in multiplex settings where a plurality of different cell types are encoded and assayed for a phenotype. The large number of distinguishable semiconductor nanocrystals, fluorphores and combinations thereof can be employed to simultaneously analyze differently spectrally encoded cells.

### BRIEF DESCRIPTION OF THE DRAWINGS

Figure 1 is a pictorial representation illustrating a method for introducing semiconductor nanocrystals (SCNCs), to which have been conjugated cellular target-specific ligands, into live cells using peptides that facilitate passage into cells.
Figure 2 is a pictorial representation illustrating the use SCNCs as a marker for identifying-microinjected cells in which SCNCs are microinjected either alone or together with other molecules of interest to allow color-coded identification of a particular microinjected cell.
Figure 3 is a pictorial representation illustrating the use of SCNCs as markers in multicolor immunofluorescent staining in which (A) represents a co-injected protein detected by indirect fluorescence with antibody conjugated to Fluo-4 or SCNC-5, (B) represents a nucleus stained with Fluo-3 or SCNC-4, (C) represents the cell marked with SCNC-1, (D) represents actin cytoskeleton stained with Fluo-1 or SCNC-2 conjugated to phalloidin, and (E) represents microtubules stained with Fluo-2 or SCNC-3 conjugated to tubulin.
Figure 4 is a pictorial representation illustrating a method for introducing SCNCs into live cells in which the SCNC is enclosed in a liposome which contains proteins to trigger receptor mediated endocytosis and acid-induced fusogenic proteins.
Figure 5 depicts a bioluminescence resonance energy transfer experiment using semiconductor nanocrystals linked to a prospective binding partner for a protein of interest; this conjugate is introduced into cells expressing a fusion protein between the protein of interest and a luciferase to determine if fluorescence transfer occurs from the luciferase to the semiconductor nanocrystal *in vivo.*
Figure 6 depicts the conjugation of semiconductor nanocrystals to different types of proteins for use in affinity targeting of cells and subcellular structures.
Figure 7 depicts the toxicity screening in a single well of a single compound against a plurality of cell types encoded through the techniques described herein.
Figure 8 depicts a predictive *in silico* biodistribution and toxicity model that integrates high throughput histological information regarding prospective targets with a compound's proteome-wide selectivity against those targets.
Figure 9 lists some of the wide range of applications for cells encoded with semiconductor nanocrystals.
Figure 10 is a fluorescence micrograph of CHO cells and SCNCs incubated in the presence (Fig. 10A) or absence (Fig 10B) of Chariot reagent as described in Example 1.

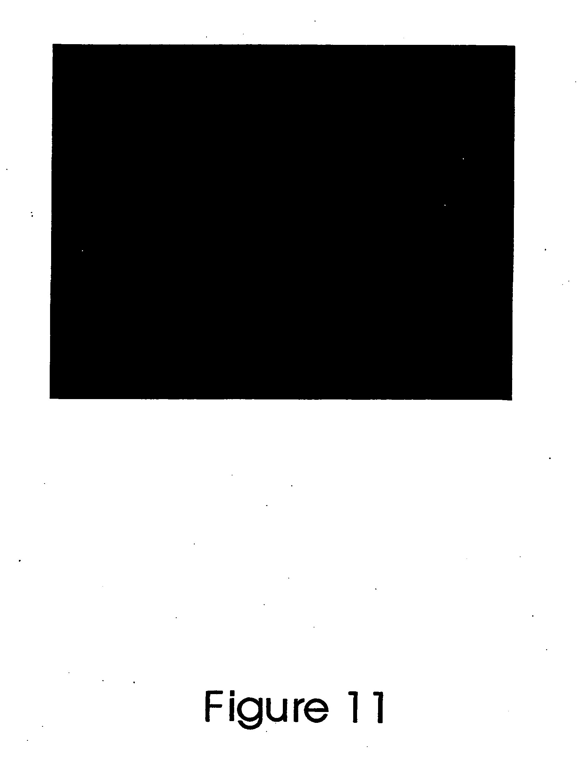

Figure 11 is a fluorescence micrograph of CHO cells incubated with 40 nM noncrosslinked polymer SCNC as described in Example 2.
Figure 12 is a fluorescence micrograph of SKBR3 breast cancer cells and green SCNCs transfected using BioPORTER reagent as described in Example 3. Cells were also stained with herceptin antibody.
Figure 13 is a fluorescence micrograph of CHO cells cotransfected with red polymer crosslinked SCNCs and EGFP/rac DNA as described in Example 4. Shown is the image using a 535 nm emission filter (Fig. 13A), a 625 nm emission filter (Fig. 13B), and the two images overlayed (Fig. 13C).
Figure 14 is a graphical representation of spectra (raw, Fig. 14A; normalized, Fig 14B) and of four individual CHO cells encoded with green SCNCs using Chariot reagent as described in Example 5.
Figure 15 is a graphical representation of spectra (raw, Fig. 15A; normalized, Fig 15B) of five individual CHO cells encoded with red SCNCs using Chariot reagent as described in Example 5.
Figure 16 is a graphical representation of spectra (raw, Fig. 16A; normalized, Fig 16B) of five individual CHO cells encoded with green and red SCNCs using Chariot reagent as described in Example 5.
Figure 17 is a pictorial representation illustrating the simultaneous single-plate screening of a plurality of different encoded cells for their ability to grow under selective conditions as described in Example 7.
Figure 18 is a graphical representation of isoproterenol dose responses of encoded or unencoded CHO cells expressing the MI muscarinic receptor.
Figure 19 illustrates the non-competed (19A) and competition binding of 1 µM CGP 12177 (19B) to encoded CHO cells expressing the β2 adrenergic receptor.

### DETAILED DESCRIPTION OF THE INVENTION

### DEFINITIONS

Before the present invention is described in detail, it is to be understood that this invention is not limited to the particular methodology, devices, or compositions described, as such methods, devices, or compositions can, of course, vary. It is also to be understood that the terminology used herein is for the purpose of describing particular embodiments only, and is not intended to limit the scope of the present invention.

Use of the singular forms "a," "an," and "the" include plural references unless the context clearly dictates otherwise. Thus, for example, reference to "a cell" includes a plurality of cells, reference to "a semiconductor nanocrystal" includes a plurality of such semiconductor nanocrystals, reference to "an encoded cell" includes a plurality of encoded cells, and the like.

Unless defined otherwise or the context clearly dictates otherwise, all technical and scientific terms used herein have the same meaning as commonly understood by one of ordinary skill in the art to which this invention belongs. Although any methods and materials similar or equivalent to those described herein can be used in the practice or testing of the invention, the preferred methods and materials are now described.

All publications mentioned herein are for the purpose of disclosing and describing the particular materials and methodologies for which the reference was cited. The publications discussed herein are provided solely for their disclosure prior to the filing date of the present application. Nothing herein is to be construed as an admission that the invention is not entitled to antedate such disclosure by virtue of prior invention.

In describing the present invention, the following terms will be employed, and are intended to be defined as indicated below.

The term "nanoparticle" refers to a particle, generally a semiconductive or metallic particle, having a diameter in the range of about 1 nm to about 1000 nm, preferably in the range of about 2 nm to about 50 nm, more preferably in the range of about 2 nm to about 20 nm (for example about 2, 3, 4, 5, 6, 7, 8, 9,10,11,12, 13, 14, 15, 16, 17, 18, 19, or 20 nm).

The terms "semiconductor nanoparticle" and "semiconductive nanoparticle" refer to a nanoparticle as defined above that is composed of an inorganic semiconductive material, an alloy or other mixture of inorganic semiconductive materials, an organic semiconductive material, or an inorganic or organic semiconductive core contained within one or more semiconductive overcoat layers.

The term "metallic nanoparticle" (SCNC) refers to a nanoparticle as defined above that is composed of a metallic material, an alloy or other mixture of metallic materials, or a metallic core contained within one or more metallic overcoat layers.

The terms "semiconductor nanocrystal," "quantum dot" and "Qdot^{™} nanocrystal" are used interchangeably herein to refer to semiconductor nanoparticles composed of an inorganic crystalline material that is luminescent (i.e., they are capable of emitting electromagnetic radiation upon excitation), and include an inner core of one or more first semiconductor materials that is optionally contained within an overcoating or "shell" of a second semiconductor material. A semiconductor nanocrystal core surrounded by a semiconductor shell is referred to as a "core/shell" semiconductor nanocrystal. The surrounding shell material will preferably have a bandgap energy that is larger than the bandgap energy of the core material and may be chosen to have an atomic spacing close to that of the core substrate. Suitable semiconductor materials for the core and/or shell include, but not limited to, the following: materials comprised of a first element selected from Groups 2 and 12 of the Periodic Table of the Elements and a second element selected from Group 16 (e.g., ZnS, ZnSe, ZnTe, CDs, CdSe, CdTe, HgS, HgSe, HgTe, MgS, MgSe, MgTe, CaS, CaSe, CaTe, SrS, SrSe, SrTe, BaS, BaSe, BaTe, and the like); materials comprised of a first element selected from Group 13 of the Periodic Table of the Elements and a second element selected from Group 15 (GaN, GaP, GaAs, GaSb, InN, InP, InAs, InSb, and the like); materials comprised of a Group14 element (Ge, Si, and the like); materials such as PbS, PbSe and the like; and alloys and mixtures thereof. As used herein, all reference to the Periodic Table of the Elements and groups thereof is to the new -IUPAC system for numbering element groups, as set forth in the Handbook of Chemistry and Physics, 81st Edition (CRC Press, 2000).

An SCNC is optionally surrounded by a "coat" of an organic capping agent. The organic capping agent may be any number of materials, but has an affinity for the SCNC surface. In general, the capping agent can be an isolated organic molecule, a polymer (or a monomer for a polymerization reaction), an inorganic complex, or an extended crystalline structure. The coat can be used to convey solubility, e.g., the ability to disperse a coated SCNC homogeneously into a chosen solvent, functionality, binding properties, or the like. In addition, the coat can be used to tailor the optical properties of the SCNC.

Thus, the terms "semiconductor nanocrystal," "SCNC," "quantum dot" and "Qdot^{™} nanocrystal" as used herein include a coated SCNC core, as well as a core/shell SCNC.

"Monodisperse particles" include a population of particles wherein at least about 60% of the particles in the population, more preferably about 75 to about 90, or any integer therebetween, percent of the particles in the population fall within a specified particle size range. A population of monodisperse particles deviates less than 10% rms (root-mean-square) in diameter, and preferably deviates less than 5% rms.

The phrase "one or more sizes of SCNCs" is used synonymously with the phrase "one or more particle size distributions of SCNCs." One of ordinary skill in the art will realize that particular sizes of SCNCs are actually obtained as particle size distributions.

By "luminescence" is meant the process of emitting electromagnetic radiation (light) from an object. Luminescence results when a system undergoes a transition from an excited state to a lower energy state with a corresponding release of energy in the form of a photon. These energy states can be electronic, vibrational, rotational, or any combination thereof. The transition responsible for luminescence can be stimulated through the release of energy stored in the system chemically or added to the system from an external source. The external source of energy can be of a variety of types including chemical, thermal, electrical, magnetic, electromagnetic, and physical, or any other type of energy source capable of causing a system to be excited into a state higher in energy than the ground state. For example, a system can be excited by absorbing a photon of light, by being placed in an electrical field, or through a chemical oxidation-reduction reaction. The energy of the photons emitted during luminescence can be in a range from low-energy microwave radiation to high-energy x-ray radiation. Typically, luminescence refers to photons in the range from UV to IR radiation.

"Preferential binding" refers to the increased propensity of one member of a binding pair to bind to a second member as compared to other components in the sample.

The terms "polynucleotide," "oligonucleotide," "nucleic acid" and "nucleic acid molecule" are used interchangeably herein to refer to a polymeric form of nucleotides of any length, and may comprise ribonucleotides, deoxyribonucleotides, analogs thereof, or mixtures thereof. This term refers only to the primary structure of the molecule. Thus, the term includes triple-, double- and single-stranded deoxyribonucleic acid ("DNA"), as well as triple-, double- and single-stranded ribonucleic acid ("RNA"). It also includes modified, for example by alkylation, and/or by capping, and unmodified forms of the polynucleotide. More particularly, the terms "polynucleotide," "oligonucleotide," "nucleic acid" and "nucleic acid molecule" include polydeoxyribonucleotides (containing 2-deoxy-D-ribose), polyribonucleotides (containing D-ribose), including tRNA, rRNA, hRNA, and mRNA, whether spliced or unspliced, any other type of polynucleotide which is an N- or C-glycoside of a purine or pyrimidine base, and other polymers containing nonnucleotidic backbones, for example, polyamide (e.g., peptide nucleic acids (PNAs)) and polymorpholino (commercially available from the Anti-Virals, Inc., Corvallis, Oregon, as Neugene) polymers, and other synthetic sequence-specific nucleic acid polymers providing that the polymers contain nucleobases in a configuration which allows for base pairing and base stacking, such as is found in DNA and RNA. There is no intended distinction in length between the terms "polynucleotide," "oligonucleotide," nucleic acid" and "nucleic acid molecule," and these terms are used interchangeably herein. These terms refer only to the primary structure of the molecule. Thus, these terms include, for example, 3'-deoxy-2',5'-DNA, oligodeoxyribonucleotide N3'-P5' phosphoramidates, oligodeoxyribonucleotide N3'-P5' thiophosphoramidates, 2'-O-alkylsubstituted RNA, double- and single-stranded DNA, as well as double- and single-stranded RNA, and hybrids thereof including for example hybrids between DNA and RNA or between PNAs and DNA or RNA, and also include known types of modifications, for example, labels, alkylation, "caps," substitution of one or more of the nucleotides with an analog, internucleotide modifications such as, for example, those with uncharged linkages (e.g., methyl phosphonates, phosphotriesters, phosphoramidates, thiophosphoramidates, carbamates, etc.), with negatively charged linkages (e.g., phosphorothioates, phosphorodithioates, etc.), and with positively charged linkages (e.g., aminoalkylphosphoramidates, aminoalkylphosphotriesters), those containing pendant moieties, such as, for example, proteins (including enzymes (e.g. nucleases), toxins, antibodies, signal peptides, poly-L-lysine, etc.), those with intercalators (e.g., acridine, psoralen, etc.), those containing chelates (of, e.g., metals, radioactive metals, boron, oxidative metals, etc.), those containing alkylators, those with modified linkages (e.g., alpha anomeric nucleic acids, etc.), as well as unmodified forms of the polynucleotide or oligonucleotide.

It will be appreciated that, as used herein, the terms "nucleoside" and "nucleotide" will include those moieties which contain not only the known purine and pyrimidine bases, but also other heterocyclic bases which have been modified. Such modifications include methylated purines or pyrimidines, acylated purines or pyrimidines, or other heterocycles. Modified nucleosides or nucleotides can also include modifications on the sugar moiety, e.g., wherein one or more of the hydroxyl groups are replaced with halogen, aliphatic groups, or are functionalized as ethers, amines, or the like. The term "nucleotidic unit" is intended to encompass nucleosides and nucleotides.

Furthermore, modifications to nucleotidic units include rearranging, appending, substituting for or otherwise altering functional groups on the purine or pyrimidine base that form hydrogen bonds to a respective complementary pyrimidine or purine. The resultant modified nucleotidic unit optionally may form a base pair with other such modified nucleotidic units but not with A, T, C, G or U. A basic sites may be incorporated which do not prevent the function of the polynucleotide. Some or all of the residues in the polynucleotide can optionally be modified in one or more ways.

Standard A-T and G-C base pairs form under conditions which allow the formation of hydrogen bonds between the N3-H and C4-oxy of thymidine and the NI and C6-NH₂, respectively, of adenosine and between the C2-oxy, N3 and C4-NH₂, of cytidine and the C2-NH₂, N'-H and C6-oxy, respectively, of guanosine. Thus, for example, guanosine (2-amino-6-oxy-9-β-D-ribofuranosyl-purine) may be modified to form isoguanosine (2-oxy-6-amino-9-β-D-ribofuranosyl-purine). Such modification results in a nucleoside base which will no longer effectively form a standard base pair with cytosine. However, modification of cytosine (1-β-D-ribofuranosyl-2-oxy-4-amino-pyrimidine) to form isocytosine (1-β-D-ribofuranosyl-2-amino-4-oxy-pyrimidine) results in a modified nucleotide, which will not effectively base pair with guanosine but will form a base pair with isoguanosine. Isocytosine is available from Sigma Chemical Co. (St. Louis, MO); isocytidine may be prepared by the method described by Switzer et al. (1993) Biochemistry 32:10489-10496 and references cited therein; 2'-deoxy-5-methyl-isocytidine may be prepared by the method of Tor et al. (1993) J. Am. Chem. Soc. 115:4461-4467 and references cited therein; and isoguanine nucleotides may be prepared using the method described by Switzer et al. (1993), *supra,* and Mantsch et al. (1993) Biochem. 14:5593-5601, or by the method described in U.S. Patent No. 5,780,610 to Collins et al. Other nonnatural base pairs may be synthesized by the method described in Piccirilli et al. (1990) Nature 343:33-37 for the synthesis of 2,6-diaminopyrimidine and its complement (1-methylpyrazolo-[4,3]pyrimidine-5,7-(4H,6H)-dione. Other such modified nucleotidic units which form unique base pairs are known, such as those described in Leach et al. (1992) J. Am. Chem. Soc. 114:3675-3683 and Switzer et al., *supra.*

"Nucleic acid probe" and "probes" are used interchangeably and refer to a structure comprising a polynucleotide, as defined above, that contains a nucleic acid sequence that can bind to a corresponding target. The polynucleotide regions of probes may be composed of DNA, and/or RNA, and/or synthetic nucleotide analogs.

"Complementary" or "substantially complementary" refers to the ability to hybridize or base pair between nucleotides or nucleic acids, such as, for instance, between the two strands of a double stranded DNA molecule or between a polynucleotide primer and a primer binding site on a single stranded nucleic acid to be sequenced or amplified. Complementary nucleotides are, generally, A and T (or A and U), or C and G. Two single-stranded RNA or DNA molecules are said to be substantially complementary when the nucleotides of one strand, optimally aligned and compared and with appropriate nucleotide insertions or deletions, pair with at least about 80% of the nucleotides of the other stand, usually at least about 90% to 95%, and more preferably from about 98 to 100%.

Alternatively, substantial complementarity exists when an RNA or DNA strand will hybridize under selective hybridization conditions to its complement. Typically, selective hybridization will occur when there is at least about 65% complementary over a stretch of at least 14 to 25 nucleotides, preferably at least about 75%, more preferably at least about 90% complementary. *See,* Kanehisa (1984) Nucleic Acids Res. 12:203.

"Preferential hybridization" as a form of preferential binding refers to the increased propensity of one polynucleotide to bind to a complementary target polynucleotide in a sample as compared to noncomplementary polynucleotides in the sample or as compared to the propensity of the one polynucleotide to form an internal secondary structure such as a hairpin or stem-loop structure under at least one set of hybridization conditions.

Stringent hybridization conditions will typically include salt concentrations of less than about 1M, more usually less than about 500 mM and preferably less than about 200 mM. Hybridization temperatures can be as low as 5° C, but are typically greater than 22° C, more typically greater than about 30° C, and preferably in excess of about 37° C. Longer fragments may require higher hybridization temperatures for specific hybridization. Other factors may affect the stringency of hybridization, including base composition and length of the complementary strands, presence of organic solvents and extent of base mismatching, and the combination of parameters used is more important than the absolute measure of any one alone. Other hybridization conditions which may be controlled include buffer type and concentration, solution pH, presence and concentration of blocking reagents to decrease background binding such as repeat sequences or blocking protein solutions, detergent type(s) and concentrations, molecules such as polymers which increase the relative concentration of the polynucleotides, metal ion(s) and their concentration(s), chelator(s) and their concentrations, and other conditions known in the art. Less stringent, and/or more physiological, hybridization conditions are used where a labeled polynucleotide amplification product cycles on and off a substrate linked to a complementary probe polynucleotide during a real-time assay which is monitored during PCR amplification such as a molecular beacon assay. Such less stringent hybridization conditions can also comprise solution conditions effective for other aspects of the method, for example reverse transcription or PCR

The terms "aptamer" (or "nucleic acid antibody") is used herein to refer to a single- or double-stranded polynucleotide that recognizes and binds to a desired target molecule by virtue of its shape. *See, e.g.,* PCT Publication Nos. WO 92/14843, WO 91/19813, and WO 92/05285.

"Polypeptide" and "protein" are used interchangeably herein and include a molecular chain of amino acids linked through peptide bonds. The terms do not refer to a specific length of the product. Thus, "peptides," "oligopeptides," and "proteins" are included within the definition of polypeptide. The terms include polypeptides contain co-and/or post-translational modifications of the polypeptide, for example, glycosylations, acetylations, phosphorylations, and sulphations. In addition, protein fragments, analogs (including amino acids not encoded by the genetic code, e.g., homocysteine, ornithine, D-amino acids, and creatine), natural or artificial mutants or variants or combinations thereof, fusion proteins, derivatized residues (e.g., alkylation of amine groups, acetylations or esterifications of carboxyl groups) and the like are included within the meaning of polypeptide.

The terms "substrate" and "support" are used interchangeably and refer to a material having a rigid or semi-rigid surface.

As used herein, the term "binding pair" refers to first and second molecules that bind specifically to each other with greater affinity than to other components in the sample. The binding between the members of the binding pair is typically noncovalent. Exemplary binding pairs include immunological binding pairs (*e.g*., any haptenic or antigenic compound in combination with a corresponding antibody or binding portion or fragment thereof, for example digoxigenin and anti-digoxigenin, fluorescein and anti-fluorescein, dinitrophenol and anti-dinitrophenol, bromodeoxyuridine and anti-bromodeoxyuridine, mouse immunoglobulin and goat anti-mouse immunoglobulin) and nonimmunological binding pairs (*e.g*., biotin-avidin, biotin-streptavidin, hormone [*e.g*., thyroxine and cortisol]-hormone binding protein, receptor-receptor agonist or antagonist (*e.g*., acetylcholine receptor-acetylcholine or an analog thereof) IgG-protein A, lectin-carbohydrate, enzyme-enzyme cofactor, enzyme-enzyme-inhibitor, and complementary polynucleotide pairs capable of forming nucleic acid duplexes) and the like. One or both member of the binding pair can be conjugated to additional molecules.

Terms such as "connected," "attached," "linked," and "conjugated" are used interchangeably herein and encompass direct as well as indirect connection, attachment, linkage or conjugation unless the context clearly dictates otherwise.

Where a range of values is recited, it is to be understood that each intervening integer value, and each fraction thereof, between the recited upper and lower limits of that range is also specifically disclosed, along with each subrange between such values. The upper and lower limits of any range can independently be included in or excluded from the range, and each range where either, neither or both limits are included is also encompassed within the invention. Where a value being discussed has inherent limits, for example where a component can be present at a concentration of from 0 to 100%, or where the pH of an aqueous solution can range from 1 to 14, those inherent limits are specifically disclosed. Where a value is explicitly recited, it is to be understood that values which are about the same quantity or amount as the recited value are also within the scope of the invention.

Where a combination is disclosed, each subcombination of the elements of that combination is also specifically disclosed and is within the scope of the invention. Conversely, where different elements or groups of elements are disclosed, combinations thereof are also disclosed. Where any element of an invention is disclosed as having a plurality of alternatives, examples of that invention in which each alternative is excluded singly or in any combination with the other alternatives are also hereby disclosed; more than one element of an invention can have such exclusions, and all combinations of elements having such exclusions are hereby disclosed.

The terms "specific-binding molecule" and "affinity molecule" are used interchangeably herein and refer to a molecule that will selectively bind, through chemical or physical means to a detectable substance present in a sample. By "selectively bind" is meant that the molecule binds preferentially to the target of interest or binds with greater affinity to the target than to other molecules. For example, an antibody will selectively bind to the antigen against which it was raised; A DNA molecule will bind to a substantially complementary sequence and not to unrelated sequences. The affinity molecule can comprise any molecule, or portion of any molecule, that is capable of being linked to a semiconductor nanocrystal and that, when so linked, is capable of recognizing specifically a detectable substance. Such affinity molecules include, by way of example, such classes of substances as antibodies, as defined below, monomeric or polymeric nucleic acids, aptamers, proteins, polysaccharides, sugars, and the like. *See, e.g.,* Haugland, "Handbook of Fluorescent Probes and Research Chemicals" (Sixth Edition), and any of the molecules capable of forming a binding pair as described above.

An "SCNC conjugate" is an SCNC linked to a first member of a binding pair, as defined above. For example, an SCNC is "linked" or "conjugated" to, or chemically "associated" with, a member when the SCNC is coupled to, or physically associated with the member. Thus, these terms intend that the SCNC may either be directly linked to the member or may be linked via a linker moiety, such as via a chemical linker. The terms indicate items that are physically linked by, for example, covalent chemical bonds, physical forces such van der Waals or hydrophobic interactions, encapsulation, embedding, or the like. For example, nanocrystals can be associated with biotin which can bind to the proteins avidin and streptavidin.

When used in relation to a composition comprising a cell and an SCNC or other detectable moiety, the term "associated" is intended to include cells in which the SCNC is contained in the nucleus, in the cytoplasm, in an organelle contained within the cell, embedded either in whole or in part in the cytoplasmic membrane, the nuclear membrane or any other membrane within the cell, is bound to a molecule within the cell or in the cell membrane, or otherwise fixed to the cell in a manner resistant to the environment or changes in the environment, such as experimental manipulations, exposure to candidate pharmacological agents, or the like.

The term "antibody" as used herein includes antibodies obtained from both polyclonal and monoclonal preparations, as well as: hybrid (chimeric) antibody molecules (see, for example, Winter et al. (1991) Nature 349:293-299; and U.S. Patent No. 4,816,567); F(ab')2 and F(ab) fragments; Fv molecules (noncovalent heterodimers, see, for example, Inbar et al. (1972) Proc Natl Acad Sci USA 69:2659-2662; and Ehrlich et al. (1980) Biochem 19:4091-4096); single-chain Fv molecules (sFv) *(see, e.g.,* Huston et al. (1988) Proc Natl Acad Sci USA 85:5879-5883); dimeric and trimeric antibody fragment constructs; minibodies (*see, e.g.,* Pack et al. (1992) Biochem 31:1579-15 84; Cumber et al. (1992) J Immunology 149B:120-126); humanized antibody molecules *(see, e.g.,* Riechmann et al. (1988) Nature 332:323-327; Verhoeyan et al. (1988) Science 239:1534-1536; and U.K. Patent Publication No. GB 2,276,169, published 21 September 1994); and, any functional fragments obtained from such molecules, wherein such fragments retain specific-binding properties of the parent antibody molecule.

As used herein, the term "monoclonal antibody" refers to an antibody composition having a homogeneous antibody population. The term is not limited regarding the species or source of the antibody, nor is it intended to be limited by the manner in which it is made. Thus, the term encompasses antibodies obtained from murine hybridomas, as well as human monoclonal antibodies obtained using human hybridomas or from murine hybridomas made from mice expression human immunoglobulin chain genes or portions thereof. *See, e.g.,* Cote et al. (1985) Monoclonal Antibodies and Cancer Therapy, Alan R Liss, p. 77.

"Multiplexing" herein refers to an assay or other analytical method in which multiple cell types can be assayed simultaneously by using more than spectral code to encode each cell type, each different code having at least one different fluorescence characteristic (for example excitation wavelength, emission wavelength, emission intensity, FWHM (full width at half maximum peak height), or fluorescence lifetime).

For example, two different preparations of SCNCs may have the same composition but different particle sizes, and thus differ in excitation and/or emission wavelength. Or, two different preparations may have the same particle size or particle size distribution but different composition, and thus also differ in excitation and/or emission wavelength. Different preparations having different compositions of SCNCs can have different fluorescent lifetimes, and thus their emission spectra can be distinguished even when they have the same emission wavelength and intensity, for example by sampling the emission from the encoded substance at different times after excitation. Differences in FWHM can be achieved for example by using SCNCs of different composition, or of the same composition but which are synthesized differently, or by mixing different SCNC "preparations" having overlapping emission peaks together to form a new preparation.

An SCNC having a known emission wavelength and/or intensity may be included with the SCNCs used for the encoding to provide an internal standard for calibrating the wavelength and/or intensity of the other SCNC(s) used in the conjugate. In addition, other nanoparticles, e.g., metallic or magnetic nanoparticles, or other fluorescent species, examples of which are tabulated *infra,* can be used for the encoding.

The phenotypic assays of the invention can be performed in multiplex formats. Multiplex methods are provided employing 2, 3, 4, 5, 10,15, 20, 25, 50, 100, 200, 500, 1000 or more different encoded cell types which can be used simultaneously to assay for a phenotype.

Where different ligands are included in a multiplex assay, the different ligands can be encoded so that they can be distinguished. Any encoding scheme can be used, conveniently, the encoding scheme can employ one or more different fluorescent species, which can be nanoparticles, e.g., fluorescent semiconductor nanocrystals and other metallic or magnetic nanoparticles, or other fluorescent species. For the sake of simplicity, the following discussion will refer to semiconductor nanocrystals as the encoding species. However, it is to be understood that this convention is not intended to be limiting in any way and that other encoding species, e.g., other nanoparticles, such as metallic or magnetic nanoparticles, and other fluorescent species, as well as combinations of encoding species such as SCNCs, other nanoparticles and other fluorescent species, can be used to encode cells according to the disclosure that follows.

In addition, organic fluorescent species can be used to encode cells in combination with nanoparticles. Suitable fluorescent species include, but are not limited to, fluorescein, 5-carboxyfluorescein (FAM), rhodamine, 5-(2'-aminoethyl) aminonapthalene-1-sulfonic acid (EDANS), anthranilamide, coumarin, terbium chelate derivatives, Reactive Red 4, BODIPY dyes and cyanine dyes. In a preferred aspect, the organic fluorescent donors include Alexa 488, fluorescein, fluorescein iso-thiocyanate (FITC); Cy3, Cy5, PE, Texas Red, Cascade Blue, Bodipy, TMR and tetramethyl rhodamine isothiocyanate (TRITC).

Other fluorescent species are set forth below in Table 1. Those of skill in the art will know of other suitable fluorescence species suitable for use in the present invention.

**TABLE 1**

| **Fluorochrome** | **Excitation Wavelength** | **Emission Wavelength** |
|---|---|---|
| Acid Fuchsin | 540 | 630 |
| Acridine Orange (Bound to DNA) | 502 | 526 |
| Acridine Red | 455-600 | 560-680 |
| Acridine Yellow | 470 | 550 |
| Acriflavin | 436 | 520 |
| AFA (Acriflavin Feulgen SITSA) | 355-425 | 460 |
| Alizarin Complexon | 530-560 | 580 |
| Alizarin Red | 530-560 | 580 |
| Allophycocyanin | 650 | 661 |
| ACMA | 430 | 474 |
| AMCA-S, AMC | 345 | 445 |
| Aminoactinomycin D | 555 | 655 |
| 7-Aminoactinomycin D-AAD | 546 | 647 |
| Aminocoumarin | 350 | 445 |
| Anthroyl Stearate | 361-381 | 446 |
| Astrazon Brilliant Red 4G | 500 | 585 |
| Astrazon Orange R | 470 | 540 |
| Astrazon Red 6B | 520 | 595 |
| Asfrazan Yellow 7 GLL | 450 | 480 |
| Atabrine | 436 | 490 |
| Auramine | 460 | 550 |
| Aurophosphine | 450-490 | 515 |
| Aurophosphine G | 450 | 580 |
| BAO 9-(Bisamino-phenyloxadiazole) | 365 | 395 |
| BCECF | 505 | 530 |
| Berberine Sulphate | 430 | 550 |
| Bisbenzamide | 360 | 600-610 |
| BOBO-1, BO-PRO-1 | 462 | 481 |
| Blancophor FFG Solution | 390 | 470 |
| Blancophor SV | 370 | 435 |
| Bodipy F1 | 503 | 512 |
| Bodipy TMR | 542 | 574 |
| Bodipy TR | 589 | 617 |
| BOPRO 1 | 462 | 481 |
| Brilliant Sulpho-flavin FF | 430 | 520 |
| Calcein | 494 | 517 |
| Calcien Blue | 370 | 435 |
| Calcium Green | 505 | 532 |
| Calcium Orange | 549 | 576 |
| Calcofluor RW Solution | 370 | 440 |
| Calcofluor White | 440 | 500-520 |
| Calcofluor White | | |
| ABT Solution | 380 | 475 |
| Calcofluor White | | |
| Standard Solution | 365 | 435 |
| 5-(and 6-)carboxy SNARF-1 indicator | 548(low pH) | |
| 576(high pH) | 587(low pH) | |
| 635(high pH) 6-Carboxyrhodamine 6G | 525 | 555 |
| Cascade Blue | 400 | 425 |
| Catecholamine | 410 | 470 |
| Chinacrine | 450-490 | 515 |
| CL-NERF | 504(low pH) | |
| 514(highpH) | 587(low pH) | |
| 540(high pH) Coriphosphine O | 460 | 575 |
| Coumarin-Phalloidin | 387 | 470 |
| CY3.18 | 554 | 568 |
| CY5.18 | 649 | 666 |
| CY7 | 710 | 805 |
| DANS (1-DimethylAmino- Naphthaline-5-Sulphonic Acid) | 340 | 525 |
| DANSA (DiaminoNaphthyl- Sulphonic Acid) | 340-380 | 430 |
| Dansyl NH-CH, in water | 340 | 578 |
| DAPI | 350 | 470 |
| DiA | 456 | 590 |
| Diamino Phenyl Oxydiazole (DAO) | 280 | 460 |
| Di-8-ANEPPS | 488 | 605 |
| Dimethylamino-5-Sulphonic Acid | 310-370 | 520 |
| DiI [DiIC₁₈(3)] | 549 | 565 |
| DiO [DiOC₁₈(3)] | 484 | 501 |
| Diphenyl Brilliant Flavine 7GFF | 430 | 520 |
| DM-NERF | 497(low pH) | |
| 510(high pH) | 527(low pH) | |
| 536(high pH) Dopamine | 340 | 490-520 |
| ELF-97 alcohol | 345 | 530 |
| Eosin | 525 | 545 |
| Erythrosin ITC | 530 | 558 |
| Ethidium Bromide | 510 | 595 |
| Euchrysin | 430 | 540 |
| FIF (Formaldehyde Induced Fluorescence) | 405 | 435 |
| Flazo Orange | 375-530. | 612 |
| Fluorescein | 494 | 518 |
| Fluorescein Iso-thiocyanate (FITC) | 490 | 525 |
| Fluo 3 | 485 | 503 |
| FM1-43 | 479 | 598 |
| Fura-2 | 335 (high [Ca²⁺]) | 363 (low [Ca²⁺¹]) |
| | | 512 (low [Ca²¹⁺]) |
| Fura Red | 505 (high [Ca²⁺]) | 472 (low [Ca²⁺]) |
| | 436 (high [Ca²⁺]) | 657 (low [Ca²⁺]) |
| | 637 (high [Ca²⁺]) | |
| Genacryl Brilliant Red B | 520 | 590 |
| Genacryl Brilliant Yellow 10GF | 430 | 485 |
| Genacryl Pink 3G | 470 | 583 |
| Genacryl Yellow 5GF | 430 | 475 |
| Gloxalic Acid | 405 | 460 |
| Granular Blue | 355 | 425 |
| Haematoporphyrin | 530-560 | 580 |
| Hoechst 33258, 33342 (Bound to DNA) | 352 | 461 |
| 3-Hydroxypyrene-5,-8,10-TriSulfonic Acid | 403 | 513 |
| 7-Hydroxy-4-methylcoumarin | 360 | 455 |
| 5-Hydroxy-Tryptamine (5-HT) | 380-415 | 520-530 |
| Indo-1 | 350 | 405-482 |
| Intrawhite Cf Liquid | 360 | 430 |
| Leucophor PAF | 370 | 430 |
| Leucophor SF | 380 | 465 |
| Leucophor WS | 395 | 465 |
| Lissamine Rhodamine B200 (RD200) | 575 | 595 |
| Lucifer Yellow CH | 425 | 528 |
| Lucifer Yellow VS | 430 | 535 |
| LysoSensorBlueDND-192,DND-167 | 374 | 425 |
| LysoSensor Green DND-153, DND-189 | 442 | 505 |
| LysoSensor Yellow/Blue | 384(low pH) | |
| 329(high pH) | 540(low pH) | |
| 440(high pH) LysoTracker Green | 504 | 51.1 |
| LysoTracker Yellow | 534 | 551 |
| LysoTracker Red | 577 | 592 |
| Magdala Red | 524 | 600 |
| Magnesium Green | 506 | 531 |
| Magnesium Orange | 550 | 575 |
| Maxilon Brilliant Flavin 10 GFF | 450 | 495 |
| Maxilon Brilliant Flavin 8 GFF | 460 | 495 |
| Mitotracker Green FM | 490 | 516 |
| Mitotracker Orange CMTMRos | 551 | 576 |
| MPS (Methyl Green Pyronine Stilbene) | 364 | 395 |
| Mithramycin | 450 | 570 |
| NBD | 465 | 535 |
| NBD Amine | 450 | 530 |
| Nile Red | 515-530 | 525-605 |
| Nitrobenzoxadidole | 460-470 | 510-650 |
| Noradrenaline | 340 | 490-520 |
| Nuclear Fast Red | 289-530 | 580 |
| Nuclear Yellow | 365 | 495 |
| Nylosan Brilliant Flavin E8G | 460 | 510 |
| Oregon Green 488 fluorophore | 496 | 524 |
| Oregon Green 500 fluorophore | 503 | 522 |
| Oregon Green 514 fluorophore | 511 | 530 |
| Pararosaniline (Feulgen) | 570 | 625 |
| Phorwite AR Solution | 360 | 430 |
| Phorwite BKL | 370 | 430 |
| Phorwite Rev | 380 | 430 |
| Phorwite RPA | 375 | 430 |
| Phosphine 3R | 465 | 565 |
| Phosphine R | 480-565 | 578 |
| Pontochrome Blue Black | 535-553 | 605 |
| POPO-1, PO-PRO-1 | 434 | 456 |
| Primuline | 410 | 550 |
| Procion Yellow | 470 | 600 |
| Propidium Iodide | 536 | 617 |
| Pyronine | 410 | 540 |
| Pyronine B | 540-590 | 560-650 |
| Pyrozal Brilliant Flavin 7GF | 365 | 495 |
| Quinacrine Mustard | 423 | 503 |
| R-phycoerythrin | 565 | 575 |
| Rhodamine 110 | 496 | 520 |
| Rhodamine 123 | 511 | 534 |
| Rhodamine 5 GLD | 470 | 565 |
| Rhodamine 6G | 526 | 555 |
| Rhodamine B | 540 | 625. |
| Rhodamine B 200 | 523-557 | 595 |
| Rhodamine B Extra | 550 | 605 |
| Rhodamine BB | 540 | 580 |
| Rhodamine BG | 540 | 572 |
| Rhodamine Green fluorophore | 502 | 527 |
| Rhodamine Red | 570 | 590 |
| Rhodamine WT | 530 | 555 |
| Rhodol Green fluorophore | 499 | 525 |
| Rose Bengal | 540 | 550-600 |
| Serotonin | 365 | 520-540 |
| Sevron Brilliant Red 2B | 520 | 595 |
| Sevron Brilliant Red 4G | 500 | 583 |
| Sevron Brilliant Red B | 530 | 590 |
| Sevron Orange | 440 | 530 |
| Sevron Yellow L | 430 | 490 |
| SITS (Primuline) | 395-425 | 450 |
| SITS (Stilbene Isothiosulphonic Acid) | 365 | 460 |
| Sodium Green | 507 | 535 |
| Stilbene | 335 | 440 |
| Snarf 1 | 563 | 639 |
| Sulpho Rhodamine B Can C | 520 | 595 |
| Sulpho Rhodamine G Extra | 470 | 570 |
| SYTOX Green nucleic acid stain | 504 | 523 |
| SYTO Green fluorescent nucleic acid stains | 494±6 | 515±7 |
| SYTO Green fluorescent nucleic acid stains | 515±7 | 543±13 |
| SYTO 17 red fluorescent nucleic acid stain | 621 | 634 |
| Tetracycline | 390 | 560 |
| TRITC (Tetramethyl Rhodamine Isothiocyanate) | 557 | 576 |
| Texas Red | 596 | 615 |
| Thiazine Red R | 510 | 580 |
| Thioflavin S | 430 | 550 |
| Thioflavin TCN | 350 | 460 |
| Thioflavin 5 | 430 | 550 |
| Thiolyte | 370-385 | 477-484 |
| Thiozol Orange | 453 | 480 |
| Tinopol CBS | 390 | 430 |
| TOTO 1, TO-RRO-1 | 514 | 533 |
| TOTO 3, TO-PRO-3 | 642 | 661 |
| True Blue | 365 | 420-430 |
| Ultratile | 656 | 678 |
| Uranine B | 420 | 520 |
| Uvitex SFC | 365 | 435 |
| X-Rhodamine | 580 | 605 |
| Xylene Orange | 546 | 580 |
| XRITC | 582 | 601 |
| YOYO-1, YOYO-PRO-1 | 491 | 509 |
| YOYO-3, YOYO-PRO-3 | 612 | 613 |

Different populations of spectrally encoded cells can be created, each population comprising one or more different semiconductor nanocrystals. Different populations of the cells, and thus different assays, can be blended together, and the assay can be performed in the presence of the blended populations. The individual cells are scanned for their spectral properties, which allows the spectral code to be decoded and thus identifies the cell. Because of the large number of different semiconductor nanocrystals and combinations thereof which can be distinguished, large numbers of different encoded cells can be simultaneously interrogated.

"Optional" or "optionally" means that the subsequently described event or circumstance may or may not occur, and that the description includes instances where the event or circumstance occurs and instances in which it does not. For example, the phrase "optionally surrounded by a 'coat' of an organic capping agent" with reference to an SCNC includes SCNCs having such a coat, and SCNCs lacking such a coat.

### PRODUCTION OF SCNCS

SCNCs can be made from any material and by any technique that produces SCNCs having emission characteristics useful in the methods, articles and compositions taught herein. The SCNCs have absorption and emission spectra that depend on their size, size distribution and composition. Suitable methods of production are disclosed in U.S. Pats. Nos. 6,207,229, 6,048,616; 5,990,479; 5,690,807; 5,505,928; 5,262,357; PCT Publication No. WO 99/26299 (published May 27, 1999; inventors Bawendi et al*.);* Murray et al. (1993) J. Am. Chem. Soc. 115:8706-8715; Guzelian et al. (1996) J. Phys. Chem. 100:7212-7219; Peng et al. (2001) J. Am. Chem. Soc. 123:183-184; Hines et al. (1996) J. Phys. Chem. 100:468; Dabbousi et al. (1997) J. Phys. Chem. B 101:9463; Peng et al. (1997) J. Am. Chem. Soc. 119:7019; Peng et al. (1998) J. Am. Chem. Soc. 120:5343; and Qu et al. (2001) Nano Lett. 1:333-337.

Examples of materials from which SCNCs can be formed include group ll- VI, III-V and group IV semiconductors such as ZnS, ZnSe, ZnTe, CdS, CdSe, CdTe, MgS, MgSe, MgTe, CaS, CaSe, CaTe, SrS, SrSe, SrTe, BaS, BaSe, BaTe, GaN, GaP, GaAs, GaSb, InP, InAs, InSb, AlS, AlP, AlSb, Pb, Ge, Si, and other materials such as PbS, PbSe, and mixtures of two or more semiconducting materials, and alloys of any semiconducting material(s).

The composition, size and size distribution of the semiconductor nanocrystals affect their absorption and emission spectra. Exemplary SCNCs that emit energy in the visible range include CdS, CdSe, CdTe, ZnSe, ZnTe, GaP, and GaAs. Exemplary SCNCs that emit energy in the near IR range include InP, InAs, InSb, PbS, and PbSe. Exemplary SCNCs that emit energy in the blue to near-ultraviolet include ZnS and GaN. The size of SCNCs in a given population can be determined by the synthetic scheme used and/or through use of separation schemes, including for example size-selective precipitation and/or centrifugation. The separation schemes can be employed at an intermediate step in the synthetic scheme or after synthesis has been completed. For a given composition, larger SCNCs absorb and emit light at longer wavelengths than smaller SCNCs. SCNCs absorb strongly in the visible and UV and can be excited efficiently at wavelengths shorter than their emission peak. This characteristic allows the use in a mixed population of SCNCs of a single excitation source to excite all the SCNCs if the source has a shorter wavelength than the shortest SCNC emission wavelength within the mixture; it also confers the ability to selectively excite subpopulation(s) of SCNCs within the mixture by judicious choice of excitation wavelength.

The surface of the SCNC is preferably modified to enhance emission efficiency by adding an overcoating layer to form a "shell" around the "core" SCNC, because defects in the surface of the core SCNC can trap electrons or holes and degrade its electrical and optical properties. Addition of an insulating shell layer removes nonradiative relaxation pathways from the excited core, resulting in higher luminescence efficiency. Suitable materials for the shell include semiconductor materials having a higher bandgap energy than the core and preferably also having good conductance and valence band offset. Thus, the conductance band of the shell is desirably of a higher energy and the valence band is desirably of a lower energy than those of the core. For SCNC cores that emit energy in the visible (*e.g*., CdS, CdSe, CdTe, ZnSe, ZnTe, GaP, GaAs) or near IR (*e.g.,* InP, InAs, InSb, PbS, PbSe), a material that has a bandgap energy in the ultraviolet may be used for the shell, for example ZnS, GaN, and magnesium chalcogenides, *e.g*., MgS, MgSe, and MgTe. For an SCNC core that emits in the near IR, materials having a bandgap energy in the visible, such as CdS or CdSe, or the ultraviolet may be used. Preparation of core-shell SCNCs is described in, *e.g*., Dabbousi et al. (1997) J. Phys. Chem. B 101:9463; Kuno et al. (1997) J. Phys. Chem. 106:9869; Hines et al. (1996) J. Phys. Chem. 100:468; PCT Publ. No. WO 99/26299; and U.S. Pat. No. 6,207,229 to Bawendi et al. issued March 27, 2001. The SCNCs can be made further luminescent through overcoating procedures as described in Danek et al. (1996) Chem. Mat. 8(1): 173-180, and Peng et al. (1997) J. Am. Chem. Soc. 119:7019-7029.

In a preferred embodiment, the nanocrystals are used in a core/shell configuration wherein a first semiconductor nanocrystal forms a core ranging in diameter, for example, from about 20 Å. to about 100 Å, with a shell of another semiconductor nanocrystal material grown over the core nanocrystal to a thickness of, for example, 1-10 monolayers in thickness. In a preferred embodiment, a1-10 monolayer thick shell of CdS is epitaxially grown over a core of CdSe.

Most SCNCs are typically prepared in coordinating solvent, such as TOPO and trioctyl phosphine (TOP), resulting in the formation of a passivating organic layer on the surface of SCNCs with and without a shell. Such passivated SCNCs can be readily solubilized in organic solvents, for example toluene, chloroform and hexane. Molecules in the passivating layer can be displaced or modified to provide an outermost coating that adapts the SCNCs for use in other solvent systems, for example aqueous systems.

Alternatively, an outermost layer of an inorganic material such as silica can be added around the shell to improve the aqueous dispersibility of the SCNCs, and the surface of the silica can optionally be derivatized (Bruchez *et al.* (1998), *supra).*

A displacement reaction may also be employed to modify the SCNC to improve the solubility in a particular organic solvent. For example, if it is desired to associate the SCNCs with a particular solvent or liquid, such as pyridine, the surface can be specifically modified with pyridine or pyridine-like moieties which are soluble or miscible with pyridine to ensure solvation. Water-dispersible SCNCs can be prepared as described in U.S. Patent No. 6,251,303 to Bawendi et al*.* and PCT Publ. No. WO 00/17655, published March 30,2000.

The surface layer of the SCNCs may be modified by displacement to render the SCNC reactive for a particular coupling reaction. For example, displacement of trioctylphosphine oxide (TOPO) moieties with a group containing a carboxylic acid moiety enables the reaction of the modified SCNCs with amine containing moieties to provide an amide linkage. For a detailed description of these linking reactions, *see, e.g.,* U.S. Patent No. 5,990,479 to Weiss et al*.;* Bruchez *et al.* (1998), *supra,* Chan *et al.* (1998), *supra*, Bruchez "Luminescent SCNCs: Intermittent Behavior and use as Fluorescent Biological Probes" (1998) Doctoral dissertation, University of California, Berkeley, and Mikulec "SCNC Colloids: Manganese Doped Cadmium Selenide, (Core)Shell Composites for Biological Labeling, and Highly Fluorescent Cadmium Telluride" (1999) Doctoral dissertation, Massachusetts Institute of Technology. The SCNC may be conjugated to other moieties directly or indirectly through a linker.

Examples of suitable spacers or linkers are polyethylene glycols, dicarboxylic acids, polyamines and alkylenes. The spacers or linkers are optionally substituted with functional groups, for example hydrophilic groups such as amines, carboxylic acids and alcohols or lower alkoxy group such as methoxy and ethoxy groups. Additionally, the spacers will have an active site on or near a distal end. The active sites are optionally protected initially by protecting groups. Among a wide variety of protecting groups which are useful are FMOC, BOC, t-butyl esters, t-butyl ethers, and the like. Various exemplary protecting groups are described in, for example, Atherton et al., Solid Phase Peptide Synthesis, IRL Press (1989).

### THE CELL

The cell(s) used in the methods described herein can be of any origin, including from prokaryotes, eukaryotes, or archeons. The cell(s) may be living or dead. If obtained from a multicellular organism, the cell may be of any cell type. The cell(s) may be a cultured cell line or a primary isolate, the cell(s) may be mammalian, amphibian, reptilian, plant, yeast, bacterium, spirochetes, or protozoan. The cell(s) may be, for example, human, murine, rat, hamster, chicken, quail, goat or dog. The cell may be a normal cell, a mutated cell, a genetically manipulated cell, a tumor cell, etc.

Exemplary cell types from multicellular organisms include acidophils, acinar cells, pinealocytes, adipocytes, ameloblasts, astrocytes, basal (stem) cells, basophils, hepatocytes, neurons, bulging surface cells, C cells, cardiac muscle cells, centroacinar cells, chief cells, chondrocytes, Clara cells, columnar epithelial cells, corpus luteal cells, decidual cells, dendrites, endrocrine cells, endothelial cells, enteroendocrine cells, eosinophils, erythrocytes, extraglomerular mesangial cells, fetal fibroblasts, fetal red blood cells, fibroblasts, follicular cells, ganglion cells, giant Betz cells, goblet cells, hair cells, inner hair cells, type I hair cells, hepatocytes, endothelial cells, Leydig cells, lipocytes, liver parenchymal cells, lymphocytes, lysozyme-secreting cells, macrophages, mast cells, megakaryocytes, melanocytes, mesangial cells, monocytes, myoepithelial cells, myoid cells, neck mucous cells, nerve cells, neutrophils, oligodendrocytes, oocytes, osteoblasts, osteochondroclasts, osteoclasts, osteocytes, pillar cells, sulcal cells, parathyroid cells, parietal cells, pepsinogen-secreting cells, pericytes, pinealocytes, pituicytes, plasma cells, platelets, podocytes, spermatocytes, Purkinje cells, pyramidal cells, red blood cells, reticulocytes, Schwann cells, Sertoli cells, columnar cells, skeletal muscle cells, smooth muscle cells, somatostatin cells, enteroendocrine cells, spermatids, spermatogonias, spermatozoas, stellate cells, supporting Deiter cells, support Hansen cells, surface cells, surface epithelial cells, surface mucous cells, sweat gland cells, T lymphocytes, theca lutein cells, thymocytes, thymus epithelial cell, thyroid cells, transitional epithelial cells, type I pneumonocytes, and type II pneumonocytes.

Exemplary types of tumor cells include adenomas, carcinomas, adenocarcinomas, fibroadenomas, ameloblastomas, astrocytomas, mesotheliomas, cholangiocarcinomas, cholangiofibromas, cholangiomas, chondromas, chondrosarcomas, chordomas, choriocarcinomas, craniopharyngiomas, cystadenocarcinomas, cystadenomas, dysgerminomas, ependymomas, epitheliomas, erythroid leukemias, fibroadenomas, fibromas, fibrosarcomas, gangliogliomas, ganglioneuromas, ganglioneuroblastomas, gliomas, granulocytic leukemias, hemangiomas, hemangiopericytomas, hemangiosarcomas, hibemomas, histiocytomas, keratoacanthomas, leiomyomas, leiomyosarcomas, lipomas, liposarcomas, luteomas, lymphangiomas, lymphangiosarcomas, lymphomas, medulloblastomas, melanomas, meningiomas, mesotheliomas, myelolipomas, nephroblastomas, neuroblastomas, neuromyoblastomas, odontomas, oligodendrogliomas, osteochondromas, osteomas, osteosarcomas, papillomas, paragangliomas, pheochromocytomas, pinealomas, pituicytomas, retinoblastomas, rhabdomyosarcomas, sarcomas, schwannomas, seminomas, teratomas, thecomas and thymomas.

Exemplary bacteria which may be encoded include *Staphylococcus aureus, Legionella pneumophila, Escherichia coli, M tuberculosis, S. typhimurium, Vibrio cholera, Clostridium perfringens, Clostridium tetani, Clostridium botulinum, Clostridium baratii, Clostridium difficile, M. leprae, Helicobacter pylori, Hemophilus influenza type b, Corynebacterium diphtheriae,Corynebacterium minutissimum, Bordetella pertussis, Streptococcus pneumoniae, Neisseria gonorrhoeae, Neisseria meningitides, Shigella dysenteriae, Pseudomonas aeruginosa, Bacteroides fragilis, Prevotella melaninogenica, Fusobacterium, Erysipelothrix rhusiopathiae, Listeria monocytogenes, Bacillus anthracis, Hemophilus ducreyi, Francisella tularensis, Yersinia pestis, Bartonella henselae, Klebsiella, Enterobacter, Serratia, Proteus, and Shigella.*

Exemplary spirochetes which may be encoded include *Treponema pallidum, T. pertenue, T. carateum, Borrelia recurrentis, B. vincentii, B. burgdorferi, and Leptospira icterohaemorrhagiae.*

Exemplary fungi which may be encoded include *Actinomyces bovis, Aspergillus fumigatus, Blastomyces dermatitidis, Candida albicans, Coccidioides immitis, Cryptococcus enoformans, Histoplasma capsulatum, Sporotrichum schenckii, Actinomyces israelii, Actinomyces bovis, Aspergillus fumigatus, Blastomyces dermatitidis, Candida albicans, Coccidioides immitis, Cryptococcus neoformans, Histoplasma capsulatum, Nocardia asteroides, Pneumocystis carinii, Sporothrix schenckii, Pichia pastoris. Saccharomyces cerevisiae, and Schizosaccharomyces pombe.*

Exemplary protozoa and parasites which may be encoded include Plasmodium falciparum, Entamoeba histolytica, trypansomes, Leishmania, Toxpolasma gondii, Giardia lamblia, Chlamydia trachomatis.

### SPECTRALLY ENCODED CELLS

Semiconductor nanocrystals and combinations thereof with other fluorescent species can be used to spectrally encode cells either by allowing SCNCs producing a single color or mixtures of colors to associate specifically or non-specifically to the surface of the cells or to be incorporated into the cells. Populations of cells thus encoded can then be mixed with other populations of cells with different mixtures of colors encoding them. The mixed samples of encoded cells can then be decoded.

There are several methods whereby SCNCs can be used to spectrally encode cells. The SCNCs can be coated with a substance, *e.g*., a carboxyl or amine group-containing ligand that allows the SCNCs to be linked to the proteinaceous lipid bilayer of cells or to the surface of prokaryotic cells. This is done by mixing cells (*e*.*g*., from 1 cell to ∼10¹¹ cells) for an appropriate period of time (*e.g.*, about 1 minute to about 24 hours) with an appropriate concentration of SCNCs (*e.g*., 1 pM to 1M). The excess SCNCs can be separated by filtering out SCNCs or centrifuging the cells at a speed slow enough to sediment the cells but not the SCNCs.

Alternatively, SCNCs can be conjugated with a specific molecule, *e.g*., a cell surface marker-specific antibody, that has a known affinity for a molecule on the surface of the cell and by this means the SCNCs could encode the cells by incubating cells and SCNCs. The binding partner on the surface of the cell can be an endogenous protein or a protein which is not normally endogenous to the cell, but which the cell is induced to express.

Cells can also be encoded by introducing SCNCs to the interior of the cell either by coating the SCNC with a molecule recognized by a molecule on the surface of the cell and allowing an active uptake procedure to occur (*e.g*., receptor mediated endocytosis) or by forcing the SCNCs into the cell (by transient permeabilization or via lipid vesicles or by high speed injection).

The encoding process can be performed on either living or dead cells.

The encoded cells can be subjected to an assay which introduces a specific label to interact with the cells; for example, the label can be an SCNC or another fluorescent or non-fluorescent label The specific interaction can be via receptor-ligand interactions, an adhesion molecule and its binding partner, a drug and the cellular protein to which it binds, or any other specific interaction between an entity on the cell and an introduced, labeled analyte. The labeled or unlabeled encoded cells can then be interrogated using a detection system or systems which can decode the cells and identify which cells are labeled, for example flow cytometry or another detection system described herein.

The initial mixed samples of encoded cells can then be grown in the presence or absence of a selective force (*e.g*., heat, ultraviolet light, osmotic stress, shear stress, selective media, a cytostatic or cytotoxic agent, and the like). After a certain growth period (for example, from 1 minute to 1 week depending on the cell type and type of assay being performed) the number of cells bearing the diluted code can be determined.

Through the use of the techniques described herein, a number of assays may be performed simultaneously in a single tube for a number of different analytes. This may be accomplished using a number of differently encoded cells in the same tube. The cells may then be categorized and detected by exposure to a 488 nm laser. The relative emission intensities of the different fluorescence channels are used to detect and classify which assay (which cell) is being measured.

The use of SCNCs greatly reduces the difficulty encountered with coding schemes using dye molecules because it allows simple and efficient classification and detection simultaneously with a single light source. The usually narrow dye molecule excitation spectra demand multiple excitation sources in order to successfully classify the dyes and their relative abundances.

The desired fluorescence characteristics of the cells may be obtained by mixing SCNCs of different sizes and/or compositions in a fixed amount and/or ratio to obtain the desired spectrum, which can be determined prior to association with the cells. Subsequent treatment of the cells (through for example covalent attachment, or passive absorption or adsorption) with the staining solution results in a material having the designed fluorescence characteristics.

A number of cell encoding or staining solutions can be prepared, each having a distinct distribution of sizes and compositions, to achieve the desired fluorescence characteristics. These solutions may be mixed in fixed proportions to arrive at a spectrum having the predetermined ratios and intensities of emission from the distinct SCNCs suspended in that solution. Upon exposure of this solution to a light source, the emission spectrum can be measured by techniques that are well established in the art. If the spectrum is not the desired spectrum, then more of the SCNC solution needed to achieve the desired spectrum can be added and the solution "titrated" to have the correct emission spectrum. These solutions may be colloidal solutions of SCNCs dispersed in a solvent, or they may be pre-polymeric colloidal solutions, which can be polymerized to form a matrix with SCNCs contained within.

The composition of the staining solution can be adjusted to have the desired fluorescence characteristics, preferably under the exact excitation source that will be used for the decoding. A multichannel auto-pipetter connected to a feedback circuit can be used to prepare an SCNC solution having the desired spectral characteristics, as described above. If the several channels of the titrater/pipetter are charged with several unique solutions of SCNCs, each having a unique excitation and emission spectrum, then these can be combined stepwise through addition of stock solutions.

Once the staining solution has been prepared, it can be used to incorporate a unique spectral code into a given cell or a cell population. The staining procedure can also be carried out in sequential steps.

### ATTACHING SCNCs TO CELLS

The SCNCs can be attached to the cells by covalent attachment as well as by entrapment, or can be coupled to one member of a binding pair the other member of which is attached to the cells. For instance, SCNCs are prepared by a number of techniques that result in reactive groups on the surface of the SCNC. *See, e.g.,* Bruchez et al. (1998) Science 281:2013-2016, Chan et al. (1998) Science 281:2016-2018, Colvin et al. (1992) J. Am. Chem. Soc. 114:5221-5230, Katari et al. (1994) J. Phys. Chem. 98:4109-4117, Steigerwald et al. (1987) J. Am. Chem. Soc 110:3046. The reactive groups present on the surface of the SCNCs can be coupled to reactive groups present on the cell. For example, SCNCs which have carboxylate groups present on their surface can be coupled to cells with amine groups using a carbodiimide activation step.

Any cross-linking method that links a SCNC to a cell and does not adversely affect the properties of the SCNC or the cell can bye used. In a cross-linking approach, the relative amounts of the different SCNCs can be used to control the relative intensities, while the absolute intensities can be controlled by adjusting the reaction time to control the number of reacted sites in total. After the cells are crosslinked to the SCNCs, the cells are optionally rinsed to wash away unreacted SCNCs.

A sufficient amount of fluorophore must be used to encode the cells so that the intensity of the emission from the fluorophores can be detected by the detection system used and the different intensity levels must be distinguishable, were intensity is used in the coding scheme but the fluorescence emission from the SCNCs or other fluorophores used to encode the cells must not be so intense to as to saturate the detector used in the decoding schema.

Where intact cellular structures are desired, the methods used to encode the cells cause minimal disruption of the viability of the cell and of the integrity of membranes. Alternatively, the cells can be fixed and treated with routine histochemical or cytochemical procedures. A fixative that does not affect the encoding should be used.

Semiconductor nanocrystals of varying core sizes (1-15 nm (10-150 angstroms)), composition and/or size, distribution can be conjugated to a specific-binding molecule which bind specifically to an molecule on a cell membrane or within a cell. Any specific "anti-molecule" can be used, for example, an antibody, an immunoreactive fragment of an antibody, and the like. Preferably, the anti-molecule is an antibody. The semiconductor nanocrystal conjugates are used to associate the SCNC with the cell or, once within the cell, to identify intracellular components, organelles, molecule or the like.

More specifically, the specific-binding molecule may be derived from polyclonal or monoclonal antibody preparations, may be a human antibody, or may be a hybrid or chimeric antibody, such as a humanized antibody, an altered antibody, F(ab')₂ fragments, F(ab) fragments, Fv fragments, a single-domain antibody, a dimeric or trimeric antibody fragment construct, a minibody, or functional fragments thereof which bind to the analyte of interest. Antibodies are produced using techniques well known to those of skill in the art and disclosed in, for example, U.S. Patent Nos. 4,011,308; 4,722,890; 4,016,043; 3,876,504; 3,770,380; and 4,372,745,

For example, polyclonal antibodies are generated by immunizing a suitable animal, such as a mouse, rat, rabbit, sheep or goat, with an antigen of interest In order to enhance immunogenicity, the antigen can be linked to a carrier prior to immunization. Such carriers are well known to those of ordinary skill in the art.

Immunization is generally performed by mixing or emulsifying the antigen in saline, preferably in an adjuvant such as Freund's complete adjuvant, and injecting the mixture or emulsion parenterally (generally subcutaneously or intramuscularly). The animal is generally boosted 2-6 weeks later with one or more injections of the antigen in saline, preferably using Freund's incomplete adjuvant Antibodies may also be generated by *in vitro* immunization, using methods known in the art. Polyclonal antiserum is then obtained from the immunized animal.

Monoclonal antibodies are generally prepared using the method of Kohler and Milstein (1975) Nature 256:495-497, or a modification thereof. Typically, a mouse or rat is immunized as described above. However, rather than bleeding the animal to extract serum, the spleen (and optionally several large lymph nodes) is removed and dissociated into single cells. If desired, the spleen cells may be screened (after removal of nonspecifically adherent cells) by applying a cell suspension to a plate or well coated with the antigen. B-cells, expressing membrane-bound immunoglobulin specific for the antigen, will bind to the plate, and are not rinsed away with the rest of the suspension. Resulting B-cells, or all dissociated spleen cells, are then induced to fuse with myeloma cells to form hybridomas, and are cultured in a selective medium (e:g. , hypoxanthine, aminopterin, thymidine medium, "HAT"). The resulting hybridomas are plated by limiting dilution, and are assayed for the production of antibodies which bind specifically to the immunizing antigen (and which do not bind to unrelated antigens). The selected monoclonal antibody-secreting hybridomas are then cultured either *in vitro* (e.g., in tissue culture bottles or hollow fiber reactors), or *in vivo* (e.g., as ascites in mice).

Human monoclonal antibodies are obtained by using human rather than murine hybridomas. See, e.g., Cote, et al. Monclonal Antibodies and Cancer Therapy, Alan R. Liss, 1985, p. 77

Monoclonal antibodies or portions thereof may be identified by first screening a B-cell cDNA library for DNA molecules that encode antibodies that specifically bind to p185, according to the method generally set forth by Huse et al. (1989) Science 246:1275-1281. The DNA molecule may then be cloned and amplified to obtain sequences that encode the antibody (or binding domain) of the desired specificity.

As explained above, antibody fragments which retain the ability to recognize the molecule of interest, will also find use in the subject invention. A number of antibody fragments are known in the art which comprise antigen-binding sites capable of exhibiting immunological binding properties of an intact antibody molecule. For example, functional antibody fragments can be produced by cleaving a constant region, not responsible for antigen binding, from the antibody molecule, using e.g., pepsin, to produce F(ab')₂ fragments. These fragments will contain two antigen binding sites, but lack a portion of the constant region from each of the heavy chains. Similarly, if desired, Fab fragments, comprising a single antigen binding site, can be produced, e.g., by digestion of polyclonal or monoclonal antibodies with papain. Functional fragments, including only the variable regions of the heavy and light chains, can also be produced, using standard techniques such as recombinant production or preferential proteolytic cleavage of immunoglobulin molecules. These fragments are known as Fᵥ. See, e.g., Inbar et al. (1972) Proc. Nat. Acad. Sci. USA 69:2659-2662; Hochman et al. (1976) Biochem 15:2706-2710; and Ehrlich et al. (1980) Biochem 19:4091-4096.

A single-chain Fv ("sFv" or "scFv'') polypeptide is a covalently linked V_{H}-V_{L} heterodimer which is expressed from a gene fusion including V_{H}- and V_{L}-encoding genes linked by a peptide-encoding linker. Huston et al. (1988) Proc. Nat. Acad Sci. USA 85:5879-5883. A number of methods have been described to discern and develop chemical structures (linkers) for converting the naturally aggregated, but chemically separated, light and heavy polypeptide chains from an antibody V region into an sFv molecule which will fold into a three dimensional structure substantially similar to the structure of an antigen-binding site. See, e.g., U.S. Patent Nos. 5,091,513, 5,132,405 and 4,946,778. The sFv molecules may be produced using methods described in the art. See, e.g., Huston et al. (1988) Proc. Nat. Acad. Sci. USA 85:5879-5883; U.S. Patent Nos. 5,091,513, 5,132,405 and 4,946,778. Design criteria include determining the appropriate length to span the distance between the C-terminus of one chain and the N-terminus of the other, wherein the linker is generally formed from small hydrophilic amino acid residues that do not tend to coil or form secondary structures. Such methods have been described in the art. See, e.g., U.S. Patent Nos. 5,091,513, 5,132,405 and 4,946,778. Suitable linkers generally comprise polypeptide chains of alternating sets of glycine and serine residues, and may include glutamic acid and lysine residues inserted to enhance solubility.

"Mini-antibodies" or "minibodies" will also find use with the present invention. Minibodies are sFv polypeptide chains which include oligomerization domains at their C-termini, separated from the sFv by a hinge region. Pack et al. (1992) Biochem 31:1579-1584. The oligomerization domain comprises self-associating á-helices, e.g., leucine zippers, that can be further stabilized by additional disulfide bonds. The oligomerization domain is designed to be compatible with vectorial folding across a membrane, a process thought to facilitate *in vivo* folding of the polypeptide into a functional binding protein. Generally, minibodies are produced using recombinant methods well known in the art. See, e.g., Pack et al. (1992) Biochem 31:1579-1584; Cumber et al. (1992) J Immunology 149B:120-126.

### INTRODUCTION OF THE SCNCs INTO THE CELL

In general, transfer methods into cells can be divided into three categories: physical (*e.g*., electroporation, direct transfer, and particle bombardment), chemical (e.g., proteinoids, microemulsions, and liposomes), and biological (*e.g*., virus-derived vectors, receptor-mediated uptake, phagocytosis). Derivatizing a ligand for a cellular receptor which is endocytosed with an agent acts as a means to ferry that agent into the cell.

The procedure for attaching an agent such as an SCNC to a ligand varies according to the chemical structure of the ligand. Generally, the ligand contains a variety of functional groups which are available for reaction with a suitable functional group on a biologically active molecule to bind the agent thereto. Alternatively, the ligand and/or agent may be derivatized to expose or attach additional reactive functional groups. The derivatization may involve attachment of any of a number of linker molecules such as those available from Pierce Chemical Company, Rockford III.

A linker can be used to join, covalently or noncovalently, the ligand and agent. Suitable linkers are well known to those of skill in the art and include, but are not limited to, straight or branched-chain carbon linkers, heterocyclic carbon linkers, or peptide linkers. *See, e.g.,* Birch and Lennox, Monoclonal Antibodies: Principles and Applications, Chapter 4, Wiley-Liss, New York, N.Y. (1995); U.S. Pat. Nos. 5,218,112 and 5,090,914; Hermanson (1996) Bioconjugate Techniques, Academic Press, San Diego, Calif.

A bifunctional linker having one functional group reactive with a group on a particular agent, and another group reactive with a ligand, may be used to form the desired conjugate. Alternatively, derivatization may involve chemical treatment of the ligand and/or agent; *e.g*., glycol cleavage of a sugar moiety with periodate to generate free aldehyde groups. The free aldehyde groups may then be reacted with free amine or hydrazine groups on an agent to bind the agent thereto. *See,* U.S. Pat. No. 4,671,958. Procedures for generation of free sulfhydryl groups on antibodies or antibody fragments are also known. See, U.S. Pat. No. 4,659,839. Many procedures and linker molecules for attachment of proteins to other molecules are known. *See, e.g.,* European Patent Application No. 188,256; U.S. Pats. Nos., 4,671,958, 4,659,839, 4,414,148, 4,699,784; 4,680,338; 4,569,789; and 4,589,071; and Borlinghaus et al. (1987) Cancer Res. 47:4071-4075.

Conjugates comprising cleavable linkages may be used. Cleaving of the linkage to release the agent from the ligand and/or linker may be prompted by enzymatic activity or conditions to which the conjugate is subjected. The cis-aconitic acid spacer can be used to release the agent from the ligand in endosomes. Disulfide linkages are also cleavable in the reducing environment of the endosomes.

A number of different cleavable linkers are known to those of skill in the art. *See* U.S. Pats. Nos. 4,618,492; 4,542,225, and 4,625,014. The mechanisms for release of an agent from these linker groups include, for example, irradiation of a photolabile bond and acid-catalyzed hydrolysis. U.S. Pat. No. 5,141,648 discloses conjugates comprising linkers of specified chemical structure, wherein the linkage is cleaved *in vivo* thereby releasing the attached compound. The linker is susceptible to cleavage at a mildly acidic pH, and is believed to be cleaved during transport into the cytoplasm of a target cell, thereby releasing the agent inside a target cell. U.S. Pat. No. 4,671,958 includes a description of conjugates comprising linkers which are cleaved by proteolytic enzymes of the complement system.

Alternatively, methods may be used to transport SCNCs out of the endosome. A number of suitable methods are known in the art. SCNC bound to a ligand which binds specifically to the polymeric immunoglobulin receptor can be used for efficient introduction into cells. Ferkol et al. (1993) J. Clin. Invest. 92:2394-2400; and Ferkol et al. (1995) J. Clin. Invest. 95:493-502. As an example, SCNC may be linked to ricin A, which is capable of penetrating the endosomal membrane into the cytosol. Beaumell et al. (1993) J. Biol. Chem. 268:23661-23669.

Nonlimiting examples of artificial means for transporting SCNCs across cell membranes include action of chemical agents such as detergents, enzymes or adenosine triphosphate; receptor- or transport protein-mediated uptake; liposomes or alginate hydrogels; phagocytosis; pore-forming proteins; microinjection; electroporation; hypoosmotic shock; or minimal physical disruption such as scrape loading, patch clamp methods, or bombardment with solid particles coated with or in the presence of the SCNCs of the invention.

These techniques include transfection, infection, biolistic impact, electroporation, microinjection, scraping, or any other method which introduces the gene of interest into the host cell *(see,* U.S. Pat. No. 4,743,548, U.S. Pat. No. 4,795,855, U.S. Pat. No. 5,068,193, U.S. Pat. No. 5,188,958, U.S. Pat. No. 5,463,174, U.S. Pat. No. 5,565,346 and U.S. Pat. No. 5,565,347).

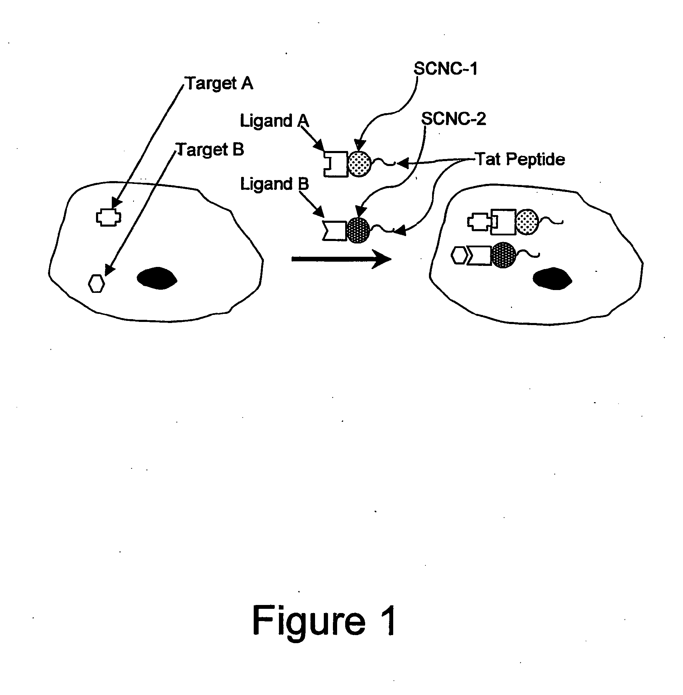

One method for introducing SCNCs into cells employs the use of peptides that encourage entry of SCNCs into the cell, e.g., the HIV-Tat peptide that facilitates viral passage into cells; the Tat peptide has been used to introduce magnetic nanoparticles into mammalian cells. SCNCs can be coated with Tat peptide sequences alone or along with other peptides, oligonucleotide or other affinity molecule to facilitate SCNC uptake by the cells and delivered to their appropriate binding partner or cellular compartment. Attachment can be achieved via any standard bioconjugation process well known in the art. SCNCs of any size and composition can be coated with both a peptide that recognizes a specific binding motif on an intracellular protein and also with a peptide that corresponds to the HIV-Tat sequence. Incubation of such modified SCNCs with a mammalian cell allows the SCNCs to enter the cell, probably via adsorptive endocytosis. Once inside the cell, the modified SCNCs can interact with and bind to the protein of oligonucleotide that contains the region recognized by the other peptide or oligonucleotide, respectively, on the surface of the SCNC. This will provide information as to the localization, trafficking and abundance of that protein. If a second color of SCNC that carries an affinity molecule for a second intracellular protein or oligonucleotide is introduced via a similar method then the relative positions of the two molecules can be determined (see Fig. 1).

Another method for introducing SCNCs into cells involves the use of micelles and liposomes. Micelles can be formed in aqueous solution by the use of micelle forming agents such as emulsifying agents, cholic acid and derivatives thereof, phosphatides, detergents, cationic lipids, and the like. Emulsifying agents include, for example, those marketed under the tradenames Cremophore EL, the Tweens, and the pluronics. Cholic acid and its derivatives include the trihydroxycholic acids, such as glyocholic acid, taurocholic acids, and their salts. Phosphatides for use in the invention include especially those that contain at least one saturated fatty acid residue that is branched, such as glycerol where two of the hydroxyl groups are esterified with residues from saturated fatty acids of C₁₀₋₂₀ where at least one of the carbon atoms has an alkyl group. Examples of such phosphatides includes, for example, 1,2-di(8-methylheptadecanoyl)-sn-glycero-3-phosphocholine, 1,2-di(10-methylstearoyl)-sn-glycero-3-phosphocholine, 1,2-(10-methylnonadecanoyl)-sn-glycero-3-phosphocholine, and the like. As will be evident to one of skill in the art, other compounds may also be added to the micelle forming agents, such as a lipoid component, bile acid salts, dihexanoyl lecithin, and the like.

SCNCs can be introduced into cells using transfection by micelle-based or liposome-based methods. Conjugated or unconjugated SCNCs in solution (about 1 fin to about 10 mM) are mixed with a micelle forming agent or any other species that can be used to form effective micelles or liposomes, at various concentrations to form SCNC trapped in the micelles. The solution containing the SCNCs trapped in the micelles can then be added to mammalian or other eukaryotic or prokaryotic cells, wherein the lipid and SCNC compositions and concentrations are varied, the micelle forming agent:SCNC ratio is varied, the cell density is varied and the time of exposure to the SCNC trapped in the micelles or liposomes is varied (e.g., from 1 minute to 48 hours) to determine the optimum transfection conditions. The efficacy of introduction of such SCNCs into cells can be assessed by standard epi-fluoreseent microscopy or by any other detection system utilizing the broadband excitation and flexible emission spectra of SCNCs.

Useful liposomes include cationic phospholipids, neutral phospholipids, lipids and mixtures thereof. Additional components may be included, such as targeting peptides or proteins, fusion peptides (*e.g*., from Sendai virus, influenza virus, hemagluttinating virus of Japan (HVJ)), envelope proteins of viruses, polycationic substances such as poly-L-lysine or DEAE-dextran, molecules which bind to the surface of airway epithelial cells including antibodies, adhesion molecules and growth factors, and the like.

The SCNC can be formulated as an SCNC-liposome complex formulation. Such complexes comprise a mixture of lipids which bind to the SCNC or a ligand attached to the SCNC, providing a hydrophobic coat which allows the agent to be delivered into cells. Liposomes that can be used include DOPE (dioleoyl phosphatidyl ethanol amine) and CUDMEDA (N-(5-cholestrum-3-ol 3-urethanyl)- N',N'-dimethylethylene diamine). Cationic liposomes which may be used in the present invention include 3-[N-(N',N'-dimethyl-aminoethane)-carbamoyl]-cholesterol (DC-Chol), N,N,N-trimethyl-2,3-bis((1-oxo-9-octadecenyl)oxy)-(Z,Z)-1-propanaminium methyl sulfate (DOTAP), lipopolyamines such as lipospermine (DOGS), (+/-)-N-(2-hydroxyethyl)-N,N-dimethyl-2,3-bis(dodecyloxy)-1-propanaminium bromide (DLRIE), DOTMA, DOSPA, DMRIE, GL-67, GL-89, Lipofectin, and Lipofectamine (Thiery et al. (1997) Gene Ther. 4:226-237; Felgner et al. (1995) Annals N.Y. Acad Set. 772:126-139; Eastman et al. (1997) Hum. Gene Ther: 8:765-773). Also encompassed are the cationic phospholipids.

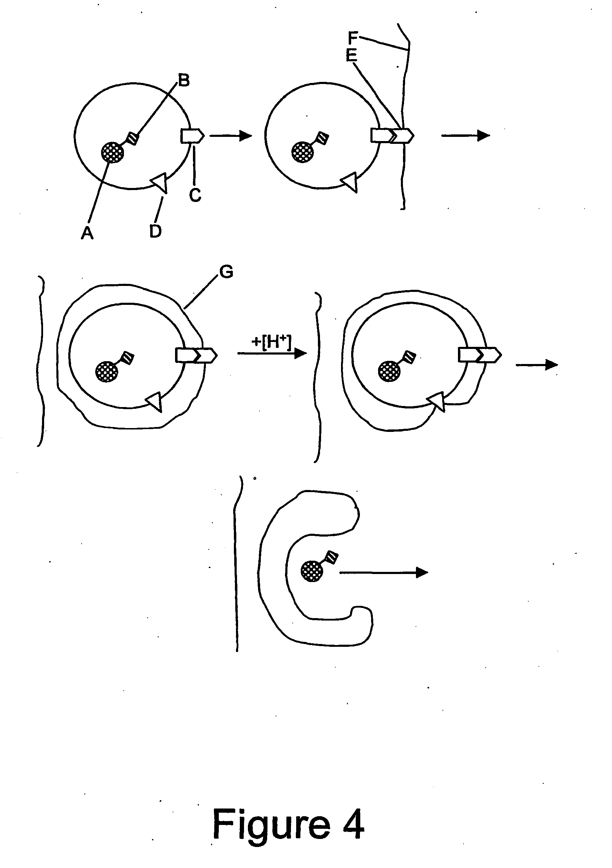

The process of receptor-mediated endocytosis results in the contents of an endosome fusing with liposomes and their subsequent degradation. Certain viruses (e.g., Semliki forest virus) avoid being transported to liposomes by being released from the endosome prior to endosoma-lysosome fusion. These viruses behave in this manner because proteins in the viral coat (e.g., hemagglutinin) are induced to cause fusion and release of viral particles in the acidic environment of the endosomes. Thus, SCNCs and proteins to trigger receptor-mediated endocytosis can be enclosed in liposomes, thereby permitting acid-induced fusogenic proteins to be introduced into cells (Fig. 4).

SCNC conjugates with a proteins or peptides of interest (e.g., the signal transduction domains of a receptor) can be entrapped within lipsomes using standard techniques. The lipsomes bilayers have proteins incorporated therein or attracted to their surface using methods already described. The proteins associated with liposome membrane can include a ligand train to induce receptor-mediated endocytosis (e.g.; transferrin) and proteins that induce fusion to the endosome under acidic conditions e.g., hemagglutinin, or some portion of such a proteins that is sufficient to generate its activity. The ligand used for receptor-mediated endocytosis can also act as a specific cell-targeting agent when introducing the lipsomes to mixed cell cultures or in whole organisms or in mixed blood cell or tissue cell populations. The SCNC-liposome can then be added to a population of cells and will be taken up and deposited into the cytoplasm of the target cells. Liposomes could be loaded with multiple SCNC conjugates and the cells subsequently treated in the appropriate manner and the localization of each type of SCNC analyzed microscopically.

SCNCs can also be incorporated into cells using an artificial viral envelope, either alone or in combination with other materials. Artificial membranes can be prepared, for example, by double detergent dialysis as described in U.S. Patent No. 5,252,348 and published EP patent application 0 555 333 B1. These viral envelopes have a cholesterol:phospholipid ratio of about 0.8 to about 1.2, preferably 1.0, similar to natural viral envelopes. The particles also have a homogenous size structure similar to that of natural viral particles and a physically stable unilamellar membrane structure.

In another method, SCNCs conjugated to specific biomolecules or unconjugated SCNCs can be incorporated into cells by forming pores in the cells. The pores can be formed by, for example, electroporation, osmotic shock, or by the use of a porogen. Electroporation is a common method for introducing foreign material, such as DNA, into cells (see Hui, 1995, Methods in Molecular Biology, Chapter 2, 48:29-40). The electroporation method of the invention consists of delivering high voltage pulses to cells thereby making pores in the cell membrane to facilitate the transport of SCNCs into cells. The electroporation process consists of two major steps: reversible breakdown of the cell membranes, and recovery of permeablized cells. Thus, the electrical and incubation parameters are optimized to facilitate the transfer of SCNCs across the membrane. In general, cells in suspension (from 1 to 10¹⁰ cells) can be placed in an electroporation cuvette with an appropriately sized SCNC (10Å to 150 A) at various concentrations (approximately 1 fmol to approximately 10 mM). The cuvette is then connected to an appropriate power supply and the cells/SCNCs are subjected to a high voltage pulse of defined magnitude and length. The voltage, capacitance and resistance can be varied appropriately depending on the cells or efficiency of the protocol. For example the voltage can be varied between about 1 V to about 100 kV, preferably 1 to 5 kV), the - capacitance can be varied between about 0.1 µf to about 100 f, preferably between about 1 µf to about 50 µf, and the resistance can be varied from about 0.1 Ω to about infinity. Cells should then be allowed to recover in the appropriate medium and detection of successfully transfected cells assessed using the appropriate detection systems for the SCNC.

Alternatively, porogen can be digitonin, saporin, or a member of the complement cascade. Cells may be permeabilized with digitonin as described in Hagstrom et al. (1997) J. Cell. Sci. 110:2323-31, and in Sterne-Marr et al. (1992) Meth. Enzymol. 219:97-111, to allow the SCNC to be incorporated into the cell.

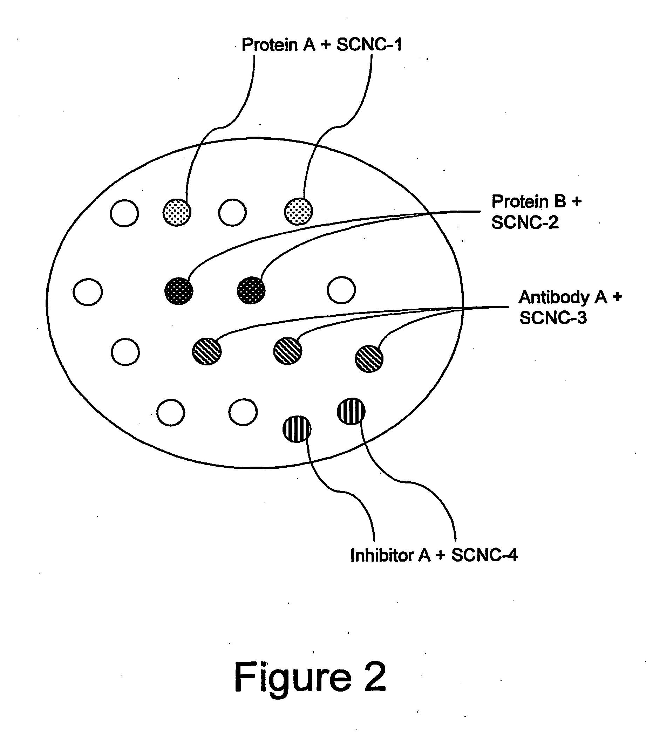

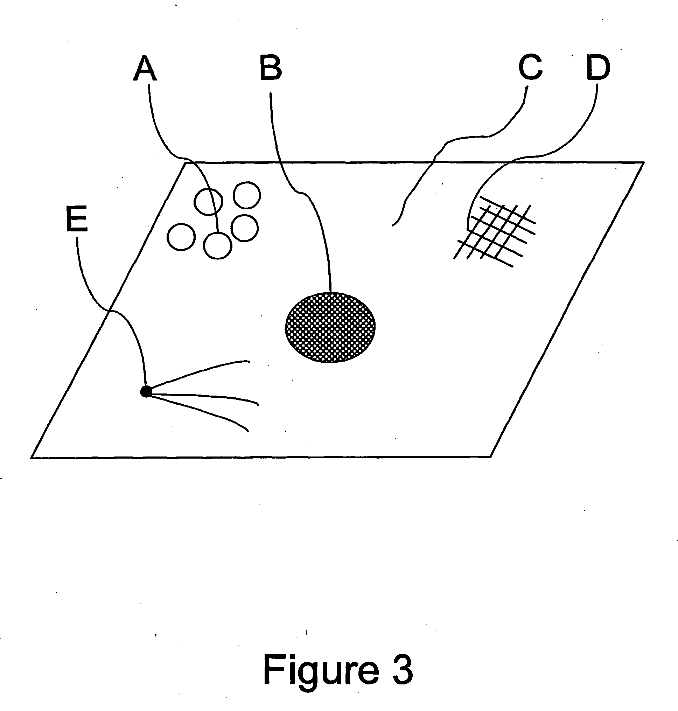

There are many other ways in which SCNC can be introduced into cells, e.g., microinjection, passive pinocytosis or uptake via coating with viral fusogenic proteins. SCNCs can be used as flexible markers for identifying microinjected cells. To this end, SCNCs are microinjected either alone (as control) or together with other molecules of interest to allow color-coded identification of a particular microinjected cell. The method of microinjection uses a syringe needle, usually a heat-drawn sharp-ended glass tube, to puncture the cell membrane to deliver a solution containing SCNCs. SCNCs are suspended in an appropriate microinjected buffer at the required concentration (1 fm to 10 mM). The needle is aimed and actuated by using a micro-manipulator and viewed under a microscope. Once the cell is punctured, a controlled quantity of SCNCs is injected by applying a controlled pressure to the syringe plunger. After microinjection, the cells are left to recover. The size and composition of the SCNC (10 Å to 150 Å) determines the emission wavelength. This type of marker can be used, for example, to differentially mark cells injected with a particular molecule within a population of cells injected with multiple molecule (Fig. 2). Because all SCNCs can be excited at a common wavelength of light, or a wavelength may be selected to excite all species that have been used to encode the population of cells, all injected cells can be visualized concurrently and the effects of the co-injected molecule observed. In addition, the use of SCNCs as markers allows a flexible third, fourth, fifth, and greater, color to be used in multicolor immunofluorescent staining experiments (Fig. 3).

### THE CODING SCHEME

The cells are encoded to allow rapid analysis of cell, identity, as well as allowing multiplexing. The coding scheme preferably employs one or more different SCNCs, although a variety of additional agents, including chromophores; fluorophores and dyes, and combinations thereof can be used in combination with SCNCs. For organic dyes, different dyes that have distinguishable fluorescence characteristics can be used. Different SCNC populations having the same peak emission wavelength but different peak widths can be used to create different codes if sufficient spectral data can be gathered to allow the populations to be distinguished. Such different populations can also be mixed to create intermediate linewidths and hence more unique codes. In addition, the coding scheme can be based on differences in excitation wavelength, emission wavelength, emission intensity, FWHM (full width at half maximum peak height), fluorescence lifetime, or combinations thereof.

The number of SCNCs used to encode a single cell locale can be selected based on the particular application. Single SCNCs can be detected; however, a plurality of SCNCs from a given population is preferably incorporated in a single cell to provide a stronger, more continuous emission signal from each cell and thus allow shorter analysis time.

Different SCNC populations can be prepared with peak wavelengths separated by approximately 1 nm, and the peak wavelength of an individual SCNC can be readily determined with 1 nm accuracy. In the case of a single-peak spectral code, each wavelength is a different code. For example, CdSe SCNCs have a range of emission wavelengths of approximately 490-640 nm and thus can be used to generate about 150 single-peak codes at 1 man resolution.

A spectral coding system that uses only highly separated spectral peaks having minimal spectral overlap and does not require stringent intensity regulation within the peaks allows for approximately 100,000 to 10,000,000 or more unique codes in different schemes.

A binary coding scheme combining a first SCNC population having an emission wavelength within a 490-565 nm channel and a second SCNC population within a 575-650 nm channel produces 3000 valid codes using 1-nm resolved SCNC populations if a minimum peak separation of 75 nm is used. The system can be expands to include many peaks, the only requirement being that the minimum separation between peak wavelengths in valid codes is sufficient to allow their resolution by the detection methods used in that application.

A binary code using a spectral bandwidth of 300 nm, a coding-peak resolution, i.e., the minimum step size for a peak within a single channel, of 4 nm, a minimum interpeak spacing of 50 nm, and a maximum of 6 peaks in each code results in approximately 200,000 different codes. This assumes a purely binary code, in which the peak within each channel is either "on" or "off" By adding a second "on" intensity, i.e., wherein intensity is 0, 1 or 2, the number of potential codes increases to approximately 5 million. If the coding-peak resolution is reduced to 1 nm, the number of codes increases to approximately 1 x 10¹⁰.

Valid codes within a given coding scheme can be identified using an algorithm. Potential codes are represented as a binary code, with the number of digits in the code corresponding to the total number of different SCNC populations having different peak wavelengths used for the coding scheme. For example, a 16-bit code could represent 16 different SCNC populations having peak emission wavelengths from 500 nm through 575 nm, at 5 nm spacing. A binary code 1000 0000 0000 0001 in this scheme represents the presence of the 500 nm and 575 nm peaks. Each of these 16-bit numbers can be evaluated for validity, depending on the spacing that is required between adjacent peaks; for example, 0010 0100 0000 0000 is a valid code if peaks spaced by 15 mn or greater can be resolved, but is not valid if the minimum spacing between adjacent peaks must be 20 nm. Using a 16-bit code with 500 to 575 nm range and 5 nm spacing between peaks, the different number of possible valid codes for different minimum spectral spacing between adjacent peaks is shown in Table 2.

| Table 2. The number of unique codes with a binary 16-bit system. | | | | | | |
|---|---|---|---|---|---|---|
| Spectral Separation | 5 nm | 10 nm | 15 nm | 20 nm | 25 nm | 30 nm |
| Number of unique codes | 65535 | 2583 | 594 | 249 | 139 | 91 |

If different distinguishable intensities are used, then the number of valid codes dramatically increases. For example, using the 16-bit code above, with 15 nm minimum spacing between adjacent peaks in a code, 7,372 different valid codes are possible if two intensities, i.e., a ternary system, are used for each peak, and 38,154 different valid codes are possible for a quaternary system, *i.e.,* wherein three "on" intensities can be distinguished.

Codes utilizing intensities require either precise matching of excitation sources or incorporation of an internal intensity standard into the cells due to the variation in extinction coefficient exhibited by individual SCNCs when excited by different wavelengths.

It is preferred that the light source used for the encoding procedure be as similar as possible (preferably of the same wavelength and intensity) to the light source that will be used for decoding. The light source may be related in a quantitative manner, so that the emission spectrum of the final material may be deduced from the spectrum of the staining solution.

Codes can optionally be created by using substantially non-overlapping colors of SCNCs, and then combining the SCNCs in unique ratios, or according to absolute levels. Alternative codes might be created by relying on overlapping signal deconvolution.

The code creation methods optionally use a computer program to combine or mix together, *in silico* (that is, using computer modeling), emission signals from SCNCs. These individual marker signal spectra can be real spectra from SCNCs that have already been manufactured, or simulated spectra for SCNC batches that can be manufactured. Candidate code spectra are then compared against one another, with acceptable codes added to the library in order to create an optimal set of codes that are sufficiently different from each other to allow robust code assignment given constraints such as code-number requirements and instrument resolution. A further method uses stored patterns of known code spectra against which to evaluate an unknown spectrum, in order to assign a code to the unknown spectrum, or to declare it as "no match." To do this, several steps are performed, some optional: (1) creation of a code; (2) creation of a template for the code; (3) comparison of a sample spectrum against all possible templates; and (4) assignment of "match" or "no match" to the sample based upon its degree of similarity to one of the templates and/or dissimilarity to the remainder.

Coded objects can be created by attaching one or more SCNC batches to an object or to many objects simultaneously. One criterion for creating useful codes is that, when a code is analyzed, it can be uniquely identified within the statistical confines of the experiment or actual code reading equipment. Generally, all codes to be used in a given application should be spectrally resolvable, i.e., sufficiently spectrally dissimilar within manufacturing tolerances and/or reading error, such that the rate of incorrect decoding is very low. The acceptable error rate depends on the application. Codes may be created randomly or systematically. Using the random approach, mixtures of SCNCs are created and then used as codes. Using the systematic approach, SCNC batches are chosen, and mixed together in the appropriate ratios to generate the codes. In both approaches, the composite emission spectrum of each new code is compared to the emission spectrum of all other codes that will be used in the application. This can be done prior to the actual physical creation of the code, by using predicted spectra, or can be done by reading the spectrum of the new code prior to, or after, attaching the code to the object(s). If the code is non-overlapping, i.e., will not be misclassified when noise, aging, reader differences, or other factors are taken into account, then the code is valid to be used. The emission spectrum of the new code is stored digitally so that putative new codes, and unknown codes during code reading, can be compared against it. Preferably, reading accuracy will be incorporated into the comparison of prior codes with new codes, the reading accuracy generally being determined based on known properties of one or more of the excitation energy source, the sensor, and the data manipulation performed by the processor.

When many items are being coded with the same code, e.g., when attaching SCNCs to cells, microspheres or beads in a batch mode, it is useful to analyze more than one of those items and store an average, or representative, spectrum for the code. Once this has been done, the actual spectrum for each sample item can be compared with the average spectrum to ensure that they are correctly identified. They may also be compared against the spectra of other codes to ensure that they are not mis-identified. Furthermore, statistical information regarding, for example, reproducibility and confidence levels can be gleaned at this stage.

The stored emission spectrum may herein be called the code's "template" and can have been generated experimentally by analyzing coded object(s) or SCNC mixtures, or can be generated *in silico* by adding together emission spectra from the SCNCs that make up the code, along with any required correction factors.

Template emission spectra may be generated by using the instrument (or a similar instrument, or a computer model of the instrument) that will be used for reading the code, optionally correcting for any instrument-to-instrument variation. For example, for SCNC-encoded cell assays it is desirable to analyze wells that contain a single or a few different known coded cells that have been processed through assay conditions. The template emission spectra may be generated for each encoded cell reader instrument so that during analysis, the templates for a given reader or assay are used.

Many different systematic methods for creating codes can be envisioned. For example, two colors of SCNCs may be used and the ratio of color 1:color-2 varied to create different codes. Using additional colors, the different ratios can be varied to create codes that are more complex.

SCNC batches that have the same color, i.e., the same peak wavelength, but have different peak widths, can be used to create two different codes if sufficient spectral data is gathered to allow these to be defined as being, significantly different. These batches can also be mixed to create intermediate linewidths and hence more unique codes.

A computer-based method that uses all physically available SCNC spectra, or that uses electronically generated spectra of all manufacturable SCNC batches, can be used. In this case, the computer is programmed to combine systematically or randomly different amounts of these SCNC spectra, *in silico,* along with any correction factors desired due to energy or electron transfer, emission intensity variations, or wavelength changes that may occur. The electronically created spectra are compared against current codes and any that are sufficiently distinguishable are candidates for manufacturing into real physical codes. This type of approach can also be used to create code sets, i.e., manufacturable emission spectra that are chosen to be maximally different from one another according to predetermined comparison criteria such as the residual value from a least squares fitting, or other methods known in the art.

Data on the overall emission spectrum of a code can be gathered by exciting the SCNCs with an appropriate source, e.g., laser, lamp, light-emitting diode, or the like, and reading the emitted light with a device that provides spectral information for the object, e.g., grating spectrometer, prism spectrometer, imaging spectrometer, or the like, or use of interference (bandpass) filters. Using a two-dimensional area imager such as a CCD camera, many objects may be imaged simultaneously. Spectral information can be generated by collecting more than one image via different bandpass, longpass, or shortpass filters (interference filters, colored glass filters, or electronically tunable filters are appropriate). More than one imager may be used to gather data simultaneously through dedicated filters, or the filter may be changed in front of a single imager. Once this data has been gathered, it can be processed to generate spectral information about objects in the image, or for each pixel, or group of pixels in the image, via straightforward image processing techniques.

The emission spectrum from the sample object is compared against all the known templates. This can be done using many techniques known in the art such as least squares fitting, Fourier analysis, Kolmogorov-Smirnov Test, Pearson Rank Correlation test, or the like (see Numerical Recipes in C, Press et al., Cambridge University Press,1996). In each case, a measure of the goodness of fit of the unknown to each template is generated, (e.g., a residual value for a least squares approach, or other fit measure dependent on the fitting algorithm used such as one of the "robust" or absolute magnitude methods described by Press et al., supra). If this goodness of fit falls within the pre-determined range for only one of the codes then this is the identity of the unknown code, otherwise the unknown is classified as "no match," or as matching too many templates.

It might be desirable to make the matching process insensitive to absolute intensity variations. This can be done by including a linear or non-linear intensity normalization factor during the matching process, which is varied to generate the lowest residual value or other match parameter for each comparison. The normalization factor can be allowed to vary without limits or can be constrained to be within a given range to limit the amount of correction for intensity variations.

The spectral data can also be normalized spectrally, i.e., shifting the data spectrally in a linear or nonlinear manner, to correct for variations in the wavelength that may occur due to the instrument or due to temperature changes, degradation, or other effects that cause the SCNCs to emit at different wavelengths. Again, the spectral shift factor may be constrained to be within a given range.

When the emission spectrum also contains signal from a reporter or reference SCNC, e.g., in the case of encoded cell assays, this may be quantitated at the same time, and may also be normalized according to the factors described above. Any spectral overlap from the code into the assay signal may also be corrected for in this way.

Spectral data will often be collected from more windows and/or allowed discrete wavelengths than there are colors of SCNCs present. This allows SCNCs of only slightly differing wavelengths to be used to create the codes. Additional spectral data also makes the classification process more robust than simple one-color, one-data point approaches. An advantage of the pattern matching approach for analysis is that, independent of the method of code creation, any sufficiently different spectra can be used as unique codes. Since unique fingerprints can be obtained for each code based on individual raw spectra, concrete statistical estimate can be used in determinations such as goodness of fit, confidence intervals, and determination of uniqueness. In addition, this method allows for empirical determination of codes following chemical processing as blanks, removing much of the ambiguity associated with pre-formatted idealized code sets.

### SPECTRALLY ENCODED MICROSPHERES.

Microspheres for use in the invention disclosed herein can be spectrally encoded through incorporation of SCNCs *See, e.g.,* U.S. Patent No. 6,207,392 to Weiss et al., issued March 27, 2001, International Pat. Publ. No. WO 00/17103 (inventors Bawendi et al.), published March 30, 2000, and Han et al. (2001) Nature Biotech. 19:632-635.

Preferably, microspheres or beads used to encode cells are approximately less than about 1 micrometer, preferably 0.01 to about 0.5 micrometer, more preferably 0.01 to about 0.1 micrometer, and can be manipulated using normal solution techniques when suspended in a solution. Each individual cell can be encoded with a single microsphere having a unique code. Alternatively, each individual cell can be encoded with more than one microsphere as needed to provide a uniquely encoded cell. The beads can be prepared to contain a population of SCNCs having a single peak emission wavelength or the beads can be prepared to contain more than a single population of SCNCs, each population having a peak emission wavelength, or other fluorescence characteristic (for example excitation wavelength, emission wavelength, emission intensity, FWHM (full width at half maximum peak height), or fluorescence lifetime) that is distinguishable from that of the other populations, such that each bead has a unique spectral signature.

Polymeric microspheres or beads can be prepared from a variety of different polymers, including but not limited to polystyrene, cross-linked polystyrene, polyacrylic, polylactic acid, polyglycolic acid, poly(lactide coglycolide), polyanhydrides, poly(methyl methacrylate), poly(ethylene-co-vinyl acetate), polysiloxanes, polymeric silica, latexes, dextran polymers and epoxies. The materials have a variety of different properties with regard to swelling and porosity, which are well understood in the art. The terms "bead," "sphere," "microbead" and "microsphere" are used interchangeably herein.

The desired fluorescence characteristics of the microspheres may be obtained by mixing SCNCs of different sizes and/or compositions in a fixed amount and ratio to obtain the desired spectrum, which can be determined prior to association with the microspheres. Subsequent treatment of the microspheres (through, for example, covalent attachment, co-polymerization, or passive absorption or adsorption) with the staining solution results in a material having the designed fluorescence characteristics.

A number of SCNC solutions can be prepared, each having a distinct distribution of sizes and compositions, to achieve the desired fluorescence characteristics. These solutions may be mixed in fixed proportions to arrive at a spectrum having the predetermined ratios and intensities of emission from the distinct SCNCs suspended in that solution. Upon exposure of this solution to a light source, the emission spectrum can be measured by techniques that are well established in the art. If the spectrum is not the desired spectrum, then more of the SCNC solution needed to achieve the desired spectrum can be added and the solution "titrated" to have the correct emission spectrum. These solutions may be colloidal solutions of SCNCs dispersed in a solvent, or they may be pre-polymeric colloidal solutions, which can be polymerized to form a matrix with SCNCs contained within.

The composition of the staining solution can be adjusted to have the desired fluorescence characteristics, preferably under the exact excitation source that will be used for the decoding. A multichannel auto-pipettor connected to a feedback circuit can be used to prepare an SCNC solution having the desired spectral characteristics, as described above. If the several channels of the titrator/pipettor are charged with several unique solutions of SCNCs, each having a unique excitation and emission spectrum, then these can be combined stepwise through addition of stock solutions.

Once the staining solution has been prepared, it can be used to incorporate a unique spectral code into a given bead population. If the method of incorporation of the SCNCs into the beads is absorption or adsorption, then the solvent that is used for the staining solution should be one that is suitable for swelling the microspheres, and can be selected based on the microsphere composition. Typical solvents for swelling microspheres include those in which the microsphere material is more soluble, for example dichloromethane, chloroform, dimethylformamide, tetrahydrofuran and the like. These can be mixed with a solvent in which the microsphere material is less soluble, for example methanol or ethanol, to control the degree and rate of incorporation of the staining solution into the material.

The microspheres swell when added to the staining solution and incorporate a plurality of SCNCs in the relative proportions that are present in the staining solution. After removal of the staining solution from the material, a nonswelling solvent is added, the material shrinks, or unswells, thereby trapping the SCNCs in the material. Alternatively, SCNCs can be trapped by evaporation of the swelling solvent from the material. After rinsing with a nonswelling solvent in which the SCNCs can be suspended, the SCNCs are trapped in the material, and can be retained by further use of a nonswelling solvent. Typical nonswelling solvents include hexane and toluene. The thus-encoded beads can be separated and exposed to a variety of solvents without a change in the emission spectrum under the light source. When the material used is a polymer bead, the material can be separated from the rinsing solvent by any suitable technique, for example, centrifugation, evaporation, fluidized bed drying, etc., or combined procedures, and can be redispersed into aqueous solvents and buffers through the use of detergents in the suspending buffer.

The staining procedure can also be carried out in sequential steps. A first staining solution can be used to stain the beads with one population of SCNCs. The beads can then be separated from the first staining solution and added to a second staining solution to stain the beads with a second population of SCNCs. These steps can be repeated until the desired spectral properties are obtained from the material when excited by a light source.

The SCNCs can be attached to the beads by covalent attachment as well as by entrapment in swelled beads, or can be coupled to one member of a binding pair the other member of which is attached to the beads. For instance, SCNCs are prepared by a number of techniques that result in reactive groups on the surface of the SCNC. *See, e.g.,* Bruchez et al. (1998) Science 281:2013-2016, Chan et al. (1998) Science 281:2016-2018, Colvin et al. (1992) J. Am. Chem. Soc. 114:5221-5230, Katari et al. (1994) J. Phys. Chem. 98:4109-4117, Steigerwald et al. (1987) J. Am. Chem. Soc. 110:3046. The reactive groups present on the surface of the SCNCs can be coupled to reactive groups present on a surface of the material. For example, SCNCs which have carboxylate groups present on their surface can be coupled to beads with amine groups using a carbodiimide activation step.

Any cross-linking method that links a SCNC to a bead and does not adversely affect the properties of the SCNC or the bead can be used. In a cross-linking approach, the relative amounts of the different SCNCs can be used to control the relative intensities, while the absolute intensities can be controlled by adjusting the reaction time to control the number of reacted sites in total. After the beads are crosslinked to the SCNCs, the beads are optionally rinsed to wash away unreacted SCNCs.

A sufficient amount of fluorophore must be used to encode the beads so that the intensity of the emission from the fluorophores can be detected by the detection system used and the different intensity levels must be distinguishable, where intensity is used in the coding scheme but the fluorescence emission from the SCNCs or other fluorophores used to encode the beads must not be so intense to as to saturate the detector used in the decoding scheme.

The beads can be encoded to allow rapid analysis thereof, and thus of the cell encoded therewith, identity, as well as allowing multiplexing. The coding scheme preferably employs one or more different SCNCs, although a variety of additional agents, including chromophores, fluorophores and dyes, and combinations thereof can be used alternatively or in combination with SCNCs. For organic dyes, different dyes that have distinguishable fluorescence characteristics can be used. Different SCNC populations having the same peak emission wavelength but different peak widths can be used to create different codes if sufficient spectral data can be gathered to allow the populations to be distinguished. Such different populations can also be mixed to create intermediate linewidths and hence more unique codes.

The number of SCNCs used to encode a single bead or substrate locale can be selected based on the particular application. Single SCNCs can be detected; however, a plurality of SCNCs from a given population is preferably incorporated in a single bead to provide a stronger, more continuous emission signal from each bead and thus allow shorter analysis time.

The beads can be encoded using the coding scheme described *supra.*

### THE EXCITATION SOURCE

By exposing the encoded cells prepared and described as above to light of an excitation source, the SCNCs disposed in or on the cell will be excited to emit light. This excitation source is of an energy capable of exciting at least one population of SCNCs used in the experiment to emit light and preferably chosen to be of higher energy than the shortest emission wavelength of the SCNCs used. Further, the excitation source can be chosen such that it excites a minimum number of SCNCs in the sample to produce detectable light. Preferably the excitation source will excite a sufficient number of different populations of SCNCs to allow unique identification of all the encoded materials used in the experiment. For example, using two different populations of cells having different ratios of red to blue SCNCs, it would not be sufficient to only excite the red emitting SCNCs, e.g., by using green or yellow light, of the sample in order to decode the cells. It would be necessary to use a light source comprising at least one wavelength that is capable of exciting the blue emitting and the red emitting SCNCs simultaneously, e.g., violet or ultraviolet. There may be one or more light sources used to excite the different populations of SCNCs simultaneously, or sequentially, but a given light source will only excite subpopulations of SCNCs that emit at lower energy than the light source, due to the absorbance spectra of the SCNCs.

In addition, if a lamp source is used, degradation of the lamp can result in changes in the excitation source, thereby compromising the codes.

### DETECTION OF EMISSION

An example of an imaging system for automated detection for use with the present methods comprises an excitation source, a monochromator (or any device capable of spectrally resolving the image, or a set of narrow band filters) and a detector array. The excitation source can comprise blue or UV wavelengths shorter than the emission wavelength(s) to be detected. This may be: a broadband UV light source, such as a deuterium lamp with a filter in front; the output of a white light source such as a xenon lamp or a deuterium lamp after passing through a monochromator to extract out the desired wavelengths; or any of a number of continuous wave (cw) gas lasers, including but not limited to any of the Argon Ion laser lines (457, 488, 514, etc. nm) or a HeCd laser; solid state diode lasers in the blue such as GaN and GaAs (doubled) based lasers or the doubled or tripled output of YAG or YLF based lasers; or any of the pulsed lasers with output in the blue.

The emitted light can be detected with a device that provides spectral information for the substrate, e.g., grating spectrometer, prism spectrometer, imaging spectrometer, or the like, or use of interference (bandpass) filters. Using a two-dimensional area imager such as a CCD camera, many objects may be imaged simultaneously. Spectral information can be generated by collecting more than one image via different bandpass, longpass, or shortpass filters (interference filters, or electronically tunable filters are appropriate). More than one imager may be used to gather data simultaneously through dedicated filters, or the filter may be changed in front of a single imager. Imaging based systems, like the Biometric Imaging system, scan a surface to find fluorescent signals.

A scanning system can be used in which the sample to be analyzed is scanned with respect to a microscope objective. The luminescence is put through a single monochromator or a grating or prism to spectrally resolve the colors. The detector is a diode array that then records the colors that are emitted at a particular spatial position. The software then recreates the scanned image.

In the embodiment where cell or the population of cells is encoded with light-scattering SERS or SERRS particle, the Raman signal is detected using an epifluorescence laser confocal microscope comprising a visible or infra red excitation laser, a dichroic beam-splitter, a microscope objective, an excitation cutoff filter, spectrometer and high efficiency detector such as a CCD camera. Alternatively, the detection system can use line illumination and collection by adding a cylindrical lens to the excitation pathway and using a 2D detector. Alternatively, the detection system can use area illumination and detection, and generate spectral data by using a tunable bandpass filter or a number of fixed bandpass filters placed in the detection pathway.

### DECODING MULTIPLE FLUORESCENCE EMISSIONS

When imaging samples labeled with multiple fluorophores, it is desirable to resolve spectrally the fluorescence from each discrete region within the sample. Such samples can arise, for example, from multiple types of SCNCs (and/or other fluorophores) being used to encode cells, from a single type of SCNC being used to encode cells but bound to a molecule labeled with a different fluorophore, or from multiple cells labeled with different types of fluorophores which overlap. Decoding the spectral code of an encoded substrate can take place prior to, simultaneously with, or subsequent to obtaining information from a functional assay performed on the cells.

Many techniques have been developed to solve this problem, including Fourier transform spectral imaging (Malik et al. (1996) J. Microsc. 182:133; Brenan et al. (1994) Appl. Opt. 33:7520) and Hadamard transform spectral imaging (Treado et al. (1989) Anal. Chem. 61:732A; Treado et al. (1990) Appl. Spectrosc. 44:1-4; Treado et al. (1990) Appl. Spectrosc. 44:1270; Hammaker et al. (1995) J. Mol. Struct. 348:135; Mei et al. (1996) J. Anal. Chem. 354:250; Flateley et al. (1993) Appl. Spectrosc. 47:1464), imaging through variable interference (Youvan (1994) Nature 369:79; Goldman et al. (1992) Biotechnology 10:1557), acousto-optical (Mortensen et al. (1996) IEEE Trans. Inst. Meas. 45:394; Turner et al. (1996) Appl. Spectrosc. 50:277) or liquid crystal filters (Morris et al. (1994) Appl. Spectrosc. 48:857) or simply scanning a slit or point across the sample surface (Colarusso et al. (1998) Appl. Spectrosc. 52:106A), all of which are capable of generating spectral and spatial information across a two-dimensional region of a sample.

One-dimensional spectral imaging can easily be achieved by projecting a fluorescent image onto the entrance slit of a linear spectrometer. In this configuration, spatial information is retained along the y-axis, while spectral information is dispersed along the x-axis (Empedocles et al. (1996) Phys. Rev. Lett. 77(18):3873). The entrance slit restricts the spatial position of the light entering the spectrometer, defining the calibration for each spectrum. The width of the entrance slit, in part, defines the spectral resolution of the system.

Two-dimensional images can be obtained by eliminating the entrance, slit and allowing the discrete images from individual points to define the spatial position of the light entering the spectrometer. In this case, the spectral resolution of the system is defined, in part, by the size of the discrete images. Since the spatial position of the light from each point varies across the x-axis, however, the calibration for each spectrum will be different, resulting in an error in the absolute energy values. Splitting the original image and passing one half through a dispersive grating to create a separate image and spectra can eliminate this calibration error. With appropriate alignment, a correlation can be made between the spatial position and the absolute spectral energy.

To avoid ambiguity between images that fall along the same horizontal line, a second beam-splitter can be added, with a second dispersive element oriented at 90 degrees to the original. By dispersing the image along two orthogonal directions, it is possible to unambiguously distinguish the spectra from each discrete point within the image. The spectral dispersion can be performed using gratings, for example holographic transmission gratings or standard reflection gratings. For example, using holographic transmission gratings, the original image is split into 2 (or 3) images at ratios that provide more light to the spectrally dispersed images, which have several sources of light loss, than the direct image. This method can be used to spectrally image a sample containing discrete point signals, for example in high throughput screening of discrete spectral images such as single cells or ensembles of cells immobilized on a substrate, and for highly parallel reading of spectrally encoded cells. The images are then projected onto a detector and the signals are recombined to produce an image that contains information about the amount of light within each band-pass.

Alternatively, techniques for calibrating point spectra within a two-dimensional image are unnecessary if an internal wavelength reference (the "reference channel") is included within the spectrally encoded cell. The reference channel is preferably either the longest or shortest wavelength emitting fluorophore in the code. The known emission wavelength of the reference channel allows determination of the emission wavelengths of the fluorophores in the dispersed spectral code image. In addition to wavelength calibration, the reference channel can serve as an intensity calibration where coding schemes with multiple intensities at single emission wavelengths are used. Additionally, a fixed intensity of the reference channel can also be used as an internal calibration standard for the quantity of label bound to the surface of each bead.

In one system for reading spectrally encoded cells, a confocal excitation source is scanned across the surface of a sample. When the source passes over an encoded cell, the fluorescence spectrum is acquired. By raster-scanning the point-excitation source over the sample, all of the cells within a sample can be read sequentially.

### ENCODED CELLS IMMOBILIZED ON CHIPS

Qcell^{™} encoding technology may be used to study membrane receptor proteins. Membrane receptor proteins constitute an important target class for drug development, yet are difficult to purify and immobilize on protein chips. Native expression of membrane proteins in SCNC-encoded cells greatly facilitates the correct folding and identification of these proteins for use in a variety of proteomics and diagnostics applications. Encoded cells are randomly deposited on the chip surface, and there is no need to spatially arrange each receptor for encoding. This assay platform is compatible with a variety of detection technologies that measure binding of fluorescent-tagged ligands to proteins on a chip surface.

Encoded cells may be utilized in conjunction with a substrate, and may be grown on, attached to, or placed upon the substrate. The substrate can comprise a wide range of material, either biological, nonbiological, organic, inorganic, or a combination of any of these. For example, the substrate may be a polymerized Langmuir Blodgett film, functionalized glass, Si, Ge, GaAs, GaP, SiO₂, SiN₄, modified silicon, or any one of a wide variety of gels or polymers such as (poly)tetrafluoroethylene, (poly)vinylidenedifluoride, polystyrene, cross-linked polystyrene, polyacrylic, polylactic acid, polyglycolic acid, poly(lactide coglycolide), polyanhydrides, poly(methyl methacrylate), poly(ethylene-co-vinyl acetate), polysiloxanes, polymeric silica, latexes, dextran polymers, epoxies, polycarbonate, or combinations thereof.

Substrates can be planar crystalline substrates such as silica-based substrates (e.g. glass, quartz, or the like), or crystalline substrates used in, e.g., the semiconductor and microprocessor industries, such as silicon, gallium arsenide and the like.

Silica aerogels can also be used as substrates, and can be prepared by methods known in the art. Aerogel substrates may be used as freestanding substrates or as a surface coating for another substrate material.

The substrate can take any form and typically is a plate, slide, bead, pellet, disk, particle, strand, precipitate, membrane, optionally porous gel, sheets, tube, sphere, container, capillary, pad, slice, film, chip, multiwell plate or dish, optical fiber, and the like. The substrate may contain raised or depressed regions on which an encoded cell is located. The surface of the substrate can be etched using well known techniques to provide for desired surface features, for example trenches, v-grooves, mesa structures, or the like.

Surfaces on the substrate can be composed of the same material as the substrate or can be made from a different material, and can be coupled to the substrate by chemical or physical means. Such coupled surfaces may be composed of any of a wide variety of materials, for example, polymers, plastics, resins, polysaccharides, silica or silica-based materials, carbon, metals, inorganic glasses, membranes, or any of the above-listed substrate materials. In one embodiment, the surface will be optically transparent and will have surface Si-OH functionalities, such as those found on silica surfaces.

The substrate and/or its optional surface are chosen to provide appropriate optical characteristics for the synthetic and/or detection methods used. The substrate and/or surface can be transparent to allow the exposure of the substrate by light applied from multiple directions. The substrate and/or surface may be provided with reflective "mirror" structures to increase the recovery of light emitted by the semiconductor nanocrystal or other label. The substrate and/or its surface may also be coated to decrease the amount of spurious incident light

The substrate and/or its surface is generally resistant to, or is treated to resist, the conditions to which it is to be exposed in use, and can be optionally treated to remove any resistant material after exposure to such conditions.

### SCNCs AS LABELS TO STUDY INTRACELLULAR PROTEIN/PROTEIN INTERACTIONS.

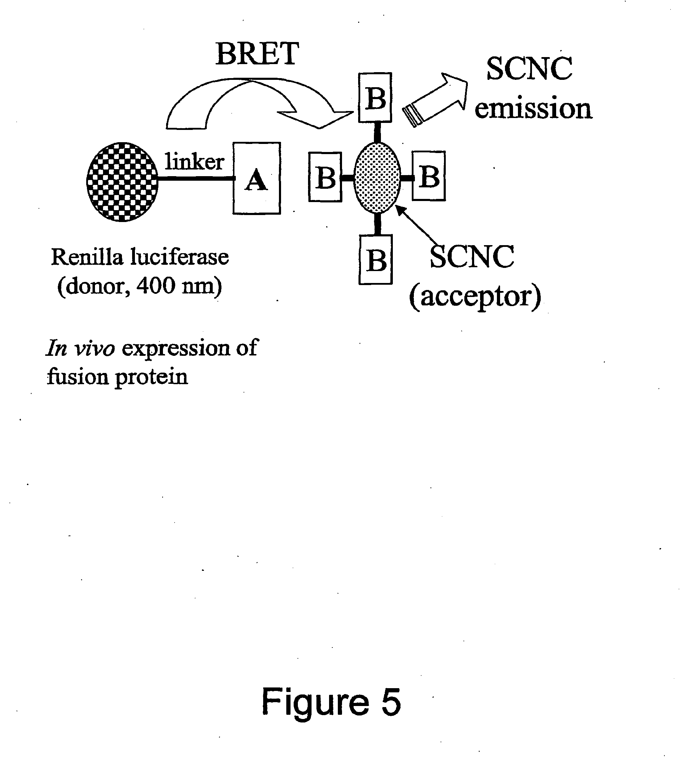

To measure intracellular protein/protein interactions, a cell line expressing, for example, a gene fusion of renilla luciferase and a protein of interest (protein A) can be used. An SCNC, conjugated to a potential interacting protein (protein B), is delivered into cells using the Chariot reagent, a peptide reagent based on the HIV-tat sequence (see Example 1). The binding of protein A and protein B is measured by bioluminescence resonance energy transfer (BRET). See Fig. 5. The light emitted by renilla luciferase is transferred to the SCNC only if protein A is bound to protein B, and the distance between luciferase and the SCNC is less than 100 angstroms. In the invention, several different SCNC/protein conjugates are delivered into cells and their interactions with protein A are studied in real time by measuring the SCNC emission properties. This approach can be used to study the assembly of complex structures such as transcription factor complexes or the splicesome inside living cells.

### ENCODING DNA TRANSFECTIONS TO SCREEN, IN A COMBINATORIAL FASHION, THE FUNCTIONS OF GENES IDENTIFIED IN GENE EXPRESSION MICROARRAY EXPERIMENTS.

As explained above, there are a number of distinct methods for delivering SCNCs into cells. One of these methods relies on a cationic lipid that is similar to commercial reagents for transfecting DNA molecules into cells which can be used to co-deliver DNA and SCNC codes into cells. The encoding of DNA transfections greatly facilitates the functional analysis of genes identified in microarray experiments. For example, a microarray experiment can identify hundreds of genes that are specifically turned on in response to a compound that induces cell apoptosis. To identify single genes or gene combinations responsible for the apoptotic phenotype, for example, many separate DNA transfections and assays are required using conventional methods. Multiplexing the assays with encoded DNA greatly facilitates these assays because the genotype is linked to phenotype via an easily read optical SCNC code. The encoded transfectants are mixed and added to wells, and the effects of a specific compound or incubation condition can be screened simultaneously against the phenotypes of many gene combinations within a single well.

### ENCODED CELLS FOR MULTIPLEX SCREENING OF DIFFERENT DRUG TARGETS EXPRESSED IN A COMMON HOST CELL LINE

Encoded cell technology can be used to screen multiple targets simultaneously in the same assay well. Each target is expressed in a common host cell line, and the identity of the receptor is encoded in the semiconductor nanocrystal code. Examples of high-value drug targets and corresponding cell-based assays include the following:
G protein coupled receptors: competition binding assays, reporter gene assays, calcium assays;
Ion channels: competition binding assays, reporter gene assays, calcium assays, membrane potential assays;
Nuclear receptors: reporter gene assays, calcium assays; and
Cytokine receptors: competition binding assays, reporter gene assays, calcium assays.

### ENCODED CELLS FOR COMPARING COMPLEX PHENOTYPES AMONG DIFFERENT CELL TYPES

Encoded cell technology is not limited to target-specific cell based assays. A complex phenotype, such as apoptosis or cell migration, can be compared between different cell types in the same assay well because each cell type is encoded with a unique SCONE code. Multiplexing complex phenotypic assays in the same assay well may be valuable for kinetic assays, or for measuring the effects of a single compound on cell-type specific responses. Examples of such phenotypic assays include the following: apoptosis, cell migration, cytoplasm to nucleus translocation, retrograde transport, neurite outgrowth, and receptor internalization.

### SCNCS AS LABELS FOR IMAGING INTRACELLULAR ORGANELLES OR STUDYING PROTEIN TRAFFICKING IN LIVE CELLS

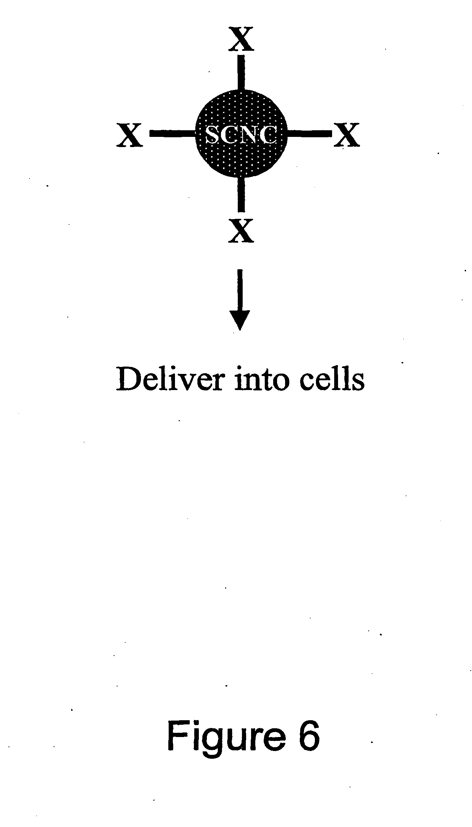

Delivery of SCNCs conjugated to specific peptides, proteins or antibodies into cells may provide a new and powerful method for live cell imaging (Fig. 6, in which X is, for example, a peptide ligand, protein, localization sequence or antibody). A peptide sequence may act as an affinity handle for binding the SCNC to a specific intracellular target, or it can target the SCNC to a specific intracellular organelle. Mulitiplex analysis of several proteins in a live cell is invaluable in screening and target validation applications.

### ENCODING FIXED CELLS FOR HISTOCHEMICAL APPLICATIONS

Encoded cell technology can also be used to multiplex any histochemical staining assay. For example, kits are commercially available for measuring the cytoplasm to nucleus translocation of several transcription factors (Cellomics). The kit is comprised of an Alexa-488-conjugated antibody that recognizes a specific transcription factor, and buffers to fix and mount the cells. Cells are incubated and fixed at various times after adding a compound, and the cytoplasm to nucleus translocation of the transcription factor is measured by fluorescence microscopy. Different encoded cell types can be used to study the translocation of a single protein among different cell types. Thus, the effect of a single compound can be screened for its ability to block or activate the translocation of a protein among different cell types.

Examples of Cellomics kits that can be encoded with SCNCs include those for NFεB, STAT1, STAT2, STAT3, STAT5, c-Jun, ATF-2, p38 MARK, JNK/SAPK, ERK MAPK.

Other cell-based assay kits that can be used with encoded cells include cell viability, neurite outgrowth, apoptosis, mitotic index, cell motility, and receptor internalization.

### MULTIPLEX SCREENING OF CELL VIABILITY

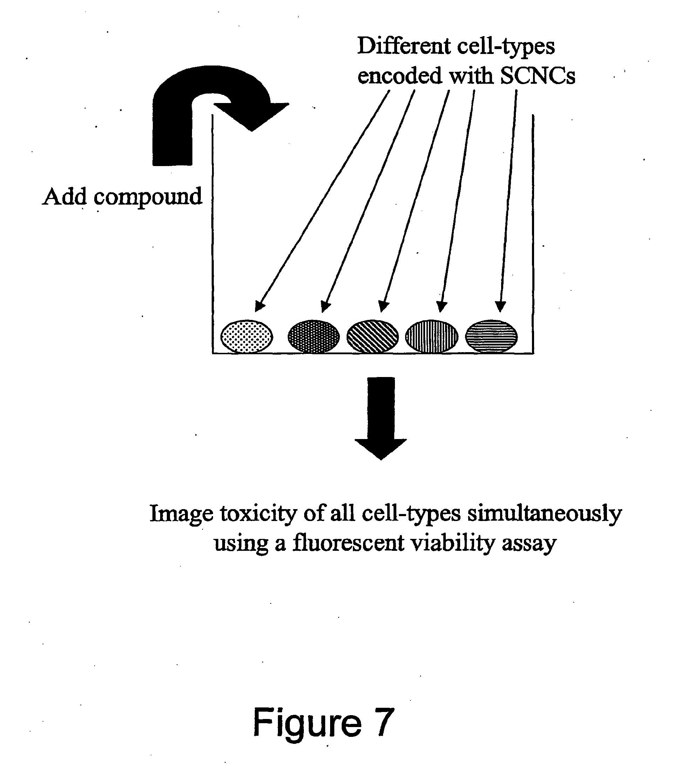

A single compound can be screened for toxicity against multiple cell types in a single well (Fig. 7).

### SELECTIVITY PROFILING AS A TOOL FOR PREDICTING COMPOUND TOXICITY.

Encoded cells can be used to measure the selectivity of compounds against many drug targets and cell types. This information can be used to predict toxicity, because many compounds are toxic due to non-selective interactions.

### SELECTIVITY PROFILING AS A TOOL TO INCREASE THE EFFICIENCY OF LEAD OPTIMIZATION

Selectivity profiling can also aid lead optimization. A thorough understanding of target selectivity at an early stage in the drug discovery pipeline can lead to better choices for lead optimization.

### COMBINING TARGET DISTRIBUTION AND COMPOUND SELECTIVITY TO PREDICT BIODISTRIBUTION OF COMPOUNDS

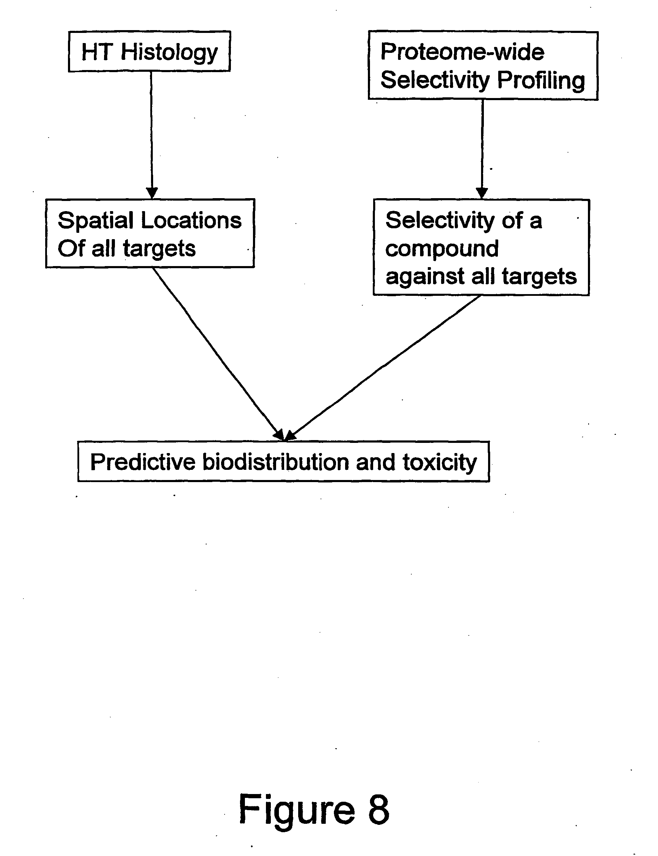

Combining a compound's proteome-wide selectivity with the proteome-wide tissue distribution of targets enables predictive *in silico* biodistribution models (Fig. 8).

### TRANSPORTER ASSAYS

Transporter proteins are a high-value target class because of their role in drug uptake. For example, selective serotinin reuptake inhibitors (SSRIs) interact with the serotonin transporter protein. SCNCs can be applied to transporter assays in several ways. First, SCNCs can be conjugated to transporter ligands and used in competition uptake assays to screen for compounds that block uptake of the SCNC conjugate. Another use is to encode cell lines expressing different transporters and to compare the uptake efficiency of a fluorescent-labeled ligand among different transporter types.

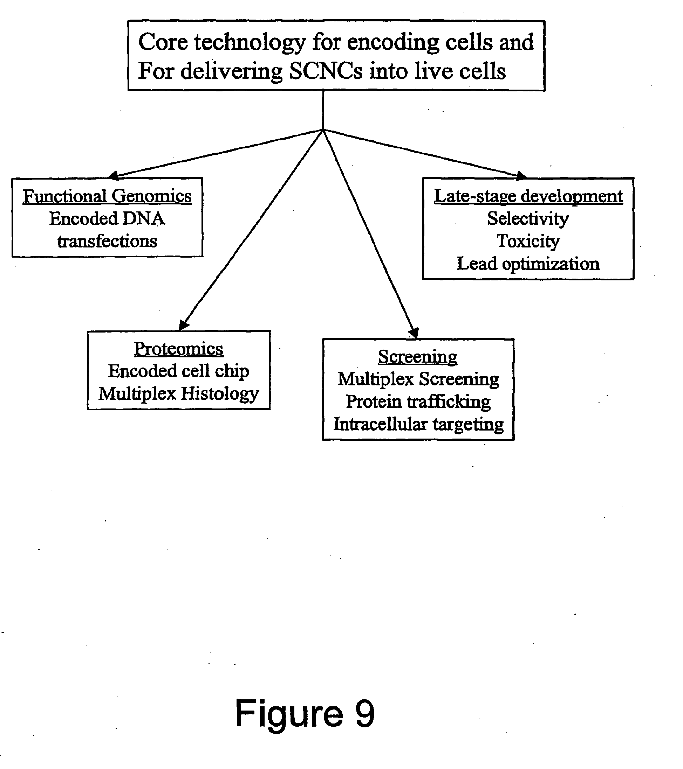

Thus, the applications of encoded cells are extremely wide-ranging (Fig. 9).

### GPCR PATHWAY ASSAYS

The present invention provides a method of screening test compounds and test conditions for the ability to modulate (activate or inhibit, enhance or depress) a GPCR pathway, and provides methods of assessing GPCR pathway function, such as the function of an orphan GPCR, in a cell in general. In the present methods, SCNCs are coupled with a candidate ligand or a library of candidate ligands, as detailed above, and translocation of the ligand by the GPCR pathway is followed by detecting the spatial location of SCNCs, or the change in spatial location of SCNCs, in extracellular fluid (natural or artificial, e.g., a growth or assay medium), in a cell, the cell cytosol, a cell membrane, or an intracellular compartment or membrane, e.g., an intracellular vesicle, the cell nucleus or nuclear membrane, mitochondria or mitochondria membrane, golgi apparatus, other organelle, or other intracellular compartment or membrane. The relative extent of translocation or change of spatial location of SCNCs under varied test conditions may be compared, or a test condition may be compared, to a control condition or to a predetermined standard. Depending on the assay design, the determination of translocation of the ligand is an indicator of modulation, e.g., agonist stimulation, of GPCR activity or of the presence of a GPCR in a cell, in a cell membrane or the like.

Translocation of the ligand is evidenced by an increase in the intensity of the detectable signal located within the cell cytosol, cell membrane, or an intracellular compartment and/or a decrease in the intensity of the detectable signal located within the cytosol, membrane, or intracellular compartment, wherein the change occurs after exposure to the test compounds. Translocation may thus be detected by comparing changes in the detectable signal in the same cell over time (i.e., pre- and post-exposure to the test compound or to one or more members of the library of test compounds). Alternatively, a test cell may be compared to a pre-established standard. If a known modulator, e.g., an agonist or antagonist ligand, is available, the present methods can be used to screen a chemical compound library for and study candidate GPCR agonists and antagonists.

The methods of the present invention provide easily detectable results. For example, translocation of a ligand, such as a GPCR ligand or beta-arrestin, coupled to an SCNC, in response to GPCR activation or inhibition, results in a relative change in the spatial location of the detectable signal within the cell cytosol, membrane or intracellular compartment. In addition, the concomitant decrease in detectable signal from the original location of the signal in the cell cytosol, membrane or intracellular compartment can be used to measure translocation of the ligand. In certain cells, the activation of the GPCRs will result in essential clearing of detectable signal from the original location of the signal, and an concomitant increase in the detectable signal within the cell cytosol, membrane or intracellular compartment. In the present methods, it is preferred that the assay design results in an increase in the detectable signal within the cell cytosol, membrane or intracellular compartment after GPCR activation. Preferably, the signal will increase at least two-fold, more preferably at least three-fold, still more preferably at least five-fold, and most preferably at least ten fold.

The present invention thus provides a convenient method of identifying modulators for an orphan GPCR. Orphan GPCRs are novel receptors and are typically identified by the sequence comparison-based methods, but whose cognate ligands are not known. It is estimated that from 400 to as many as 5000 orphan GPCRs may be coded for in the human genome, representing a vast potential for developing new drugs.

The present invention provides a convenient and efficient method for identifying a natural or synthetic ligand that initiates orphan GPCR activation, and for identifying ligands that inhibit such activation, thereby characterizing the pharmacology of the orphan GPCR. The method of the invention can be used to detect the orphan GPCRs GPCR10, OX1R and OX2R, and GPCR 24 using the ligands prolactin-releasing peptide, orexin-A/orexin-B, and melanin concentrating hormone, respectively. Thus, the functions of orphan GPCRs can be identified as controlling feeding behavior.

### PREPARATION OF CELLS THAT EXPRESS GPCRS

Methods for preparing cells that express GPCRs have been described. See, e.g., U.S. patents 6,051,386, 6,069,296, 6,111,076 and 6,280.934.

Generally, complementary DNA encoding GPCRs can be obtained and can be expressed in an appropriate cell host using techniques well known in the art. Typically, once a full length GPCR cDNA has been obtained, it can be expressed in a mammalian cell line, yeast cell, amphibian cell or insect cell for functional analysis. Preferably, the cell line is a mammalian cell line that has been characterized for GPCR expression and that optionally contains a wide repertoire of G-proteins to allow functional coupling to downstream effectors. Examples of such cell lines include Chinese Hamster Ovary (CHO) or Human Embryonic Kidney 293 (HEK293) lines. Cells in which the cDNA is expressed can be encoded using the methods disclosed herein, thus allowing the multiplex screening of ligands. The expressed receptor can then be screened in a variety of functional assays to identify an activating ligand as disclosed above and in U.S. patents 6,051,386, 6,069,296, 6,111,076 and 6,280,934. Preferably, the functional assay methods use SCNCs, although other functional responses can be monitored can also be used. Other functional responses include changes in intracellular calcium or cAMP levels, and metabolic activation, which can be measured using the Cytosensor microphysiometer. In another embodiment, the receptor is co-expressed with promiscuous G-proteins thereby aggregating signal transduction through a common pathway involving phospholipase C and calcium mobilization. Changes in calcium mobilization may be detected using SCNCs, as discussed above, or via standard fluorescence-based techniques using a high throughput imaging system such as FLIPR® (Fluorescent Imaging Plate Reader). Examples of high throughput microscopes include Discovery 1 from Universal Imaging Corporation, CellPix from Axon Instruments, LeadSeeker from Amersham/Pharmacia, and Explorer from Acumen. The ability to screen in a high-throughput manner permits the screening of orphan receptors against a wide range of candidate ligands, such as those contained in a library. The library of candidate ligands may contain known or suspected GPCR ligands, as well as molecules for which the receptor is unknown. In addition, the methods of the invention permit screening against biological extracts of tissues, fluids, and cell supernatants, thereby identifying novel ligands for GPCRs. Additionally, the methods of the invention can be used to screen against peptide libraries or compound libraries. Once an activating ligand is obtained, high-throughput screens of the invention can be used to search for modulators of the receptor, such as agonists and antagonists. The invention thus allows for the identification of various agonists and antagonists of the known and orphan GPCRs that can be used to evaluate the physiological role of the receptor and its potential as a therapeutic target for drug discovery.

### KITS

Kits comprising reagents useful for performing the methods of the invention may be made. The components of the kit are retained by a housing. Instructions for using the kit to perform a method of the invention are provided with the housing, and may be located inside the housing or outside the housing, and may be printed on the interior or exterior of any surface forming the housing which renders the instructions legible. For example, a kit comprises an SCNC population, and a reagent useful for encoding a cell using the SCNC population. Exemplary reagents useful for encoding a cell are described above. These reagents may be used alone or in combination. Additionally, the kit may be designed for multiplex applications and contain a plurality of SCNC populations useful for simultaneously encoding a plurality of Different cell populations.

### EXAMPLES

The following examples are set forth so as to provide those of ordinary skill in the art with a complete description of how to perform the present invention, and are not intended to limit the scope of what is regarded as the invention. Efforts have been made to ensure accuracy with respect to numbers used (e.g., amounts, temperature, etc.) but some experimental error and deviation should be accounted for. Unless otherwise indicated, parts are parts by weight, temperature is degree centigrade and pressure is at or near atmospheric, and all materials are commercially available. The practice of the present invention will employ, unless otherwise indicated, conventional techniques of synthetic organic chemistry, biochemistry, molecular biology, and the like, which are within the skill of the art Such techniques are explained fully in the literature. See, e.g., Sambrook, Fritsch & Maniatis, Molecular Cloning: A Laboratory Manual, Second Edition (1989); Oligonucleotide Synthesis (MJ. Gait, ed,1984); Nucleic Acid Hybridization (B.D. Haines & SJ. Higgins, eds., 1984); Methods in Enzymology (Academic Press, Inc.); Kirk-Othmer's Encyclopedia of Chemical Technology*;* and House's Modem Synthetic Reactions.

### Preparation of Polymer-coated SCNCs

A. Synthesis of Hydrophobically Modified Hydrophilic Polymers: A modified polyacrylic acid was prepared by diluting 100 g [0.48 mol COONa] of poly(acrylic acid, sodium salt) (obtained from Aldrich, molecular weight 1200) was diluted two-fold in water and acidified in a 1.0 L round bottom flask with 150 ml (1.9 mol) of concentrated HCl. The acidified polymer solution was concentrated to dryness on a rotary evaporator (100 mbar, 80°C). The dry polymer was evacuated for 12 hours at <10 mbar to ensure water removal. A stirbar and 47.0 g (0.24 mol) of 1-[3-(dimethyl-amino)-propyl]-ethylcarbodiimide hydrochloride (EDC-Aldrich 98%) were added to the flask, then the flask was sealed and purged with N₂, and fit with a balloon. 500 ml of anhydrous N-N,dimethylformamide (Aldrich) was transferred under positive pressure through a cannula to this mixture; and the flask was swirled gently to dissolve the solids. 32 ml (0.19 mol) of octylamine was transferred dropwise under positive pressure through a cannula from a sealed oven-dried graduated cylinder into the stirring polymer/EDC solution, and the stirring continued for 12 hours. This solution was concentrated to <100 ml on a rotary evaporator (30 mbar, 80°C), and the polymer was precipitated by addition of 200 ml di-H₂O to the cooled concentrate, which produced a gummy white material. This material was separated from the supernatant and triturated with 100 ml di-H₂O three more times. The product was dissolved into 400 ml ethyl acetate (Aldrich) with gentle heating, and basified with 200 ml di-H₂O and 100 g N-N-N-N-tetramethylammonium hydroxide pentahydrate (0.55 mo) for 12 hours. The aqueous layer was removed and precipitated to a gummy white product with 400 ml of 1.27 M HCl. The product was decanted and triturated with 100 ml of di-H₂O twice more, after which the aqueous washings were back-extracted into 6x100 ml portions of ethyl acetate. These ethyl acetate solutions were added to the product flask, and concentrated to dryness (100 mbar, 60°C). The crude polymer was dissolved in 300 ml of methanol and purified in two aliquots over LH-20 (Amersham-Pharmacia-5.5 cm x 60 cm column) at a 3 ml/minute flow rate. Fractions were tested by NMR for purity, and the pure fractions were pooled, while the impure fractions were re-purified on the LH-20 column. After pooling all of the pure fractions, the polymer solution was concentrated by rotary evaporation to dryness, and evacuated for 12 hours at <10 mbar. The product was a white powder (25.5 g, 45 % of theoretical yield), which showed broad NMR peaks in CD₃OD [δ = 3.1 b (9.4), 2.3 b (9.7), 1.9 1.7 1.5 1.3 b (63.3) 0.9 bt (11.3)], and clear IR signal for both carboxylic acid (1712 cm⁻¹) and amide groups (1626 cm⁻¹, 1544 cm⁻¹).
B. Preparation of Surface Modified Nanocrystals: Twenty milliliters of 3-5 µM (3-5 nmoles) of TOPO/TOP coated CdSe/ZnS nanocrystals (*see*, Murray et al. (1993) J. Am. Chem. Soc. 115:8706) were precipitated with 20 milliliters of methanol. The flocculate was centrifuged at 3000 x g for 3 minutes to form a pellet of the nanocrystals. The supernatant was thereafter removed and 20 milliliters of methanol was again added to the particles. The particles were vortexed to loosely disperse the flocculate throughout the methanol. The flocculate was centrifuged an additional time to form a pellet of the nanocrystals. This precipitation/centrifugation step was repeated an additional time to remove any excess reactants remaining from the nanocrystal synthesis. Twenty milliliters of chloroform were added to the nanocrystal pellet to yield a freely dispersed sol. 300 milligrams of hydrophobically modified poly(acrylic acid) was dissolved in 20 ml of chloroform. Tetrabutylammonium hydroxide (1.0 M in methanol) was added to the polymer solution to raise the solution to pH 10 (pH was measured by spotting a small aliquot of the chloroform solution on pH paper, evaporating the solvent and thereafter wetting the pH paper with distilled water). Thereafter the polymer solution was added to 20 ml of chloroform in a 250 ml round bottom flask equipped with a stir bar. The solution was stirred for 1 minute to ensure complete admixture of the polymer solution. With continued stirring the washed nanocrystal dispersion described above was added dropwise to the polymer solution. The dispersion was then stirred for two minutes to ensure complete mixing of the components and thereafter the chloroform was removed *in vacuo* with low heat to yield a thin film of the particle-polymer complex on the wall of the flask. Twenty milliliters of distilled water were added to the flask and swirled along the walls of the flask to aid in dispersing the particles in the aqueous medium. The dispersion was then allowed to stir overnight at room temperature. At this point the nanocrystals are freely dispersed in the aqueous medium, possess pendant chemical functionalities and may therefore be linked to affinity molecules of interest using methods well known in the art for biolabeling experiments. In addition, the fact that the nanocrystals now have a highly charged surface means they can be readily utilized in polyelectrolyte layering experiments for the formation of thin films and composite materials.
C. Crosslinking of Polymer Stabilized Nanocrystals with a Diamino Crosslinker: Ten milliliters of nanocrystals at 3.5 µM, stabilized as described *supra*, were purified by tangential flow filtration using a 100 K polyethersulfone membrane against one liter of distilled water and one liter of 50 mM Morpholinoethanesulfonic acid buffer, pH 5.9. The nanocrystals were concentrated to 10 milliliters and the pH of the aqueous dispersion was decreased to pH 6.5 with 50 µl additions of 0.1M HCl. 67 milligrams (315 µmoles) EDC were added to the stirring nanocrystal dispersion. The reaction was allowed to proceed for 10 minutes before 1 milliliter of 0.5M borate buffer (pH 8.5) containing 3.94 µmoles of the crosslinking reagent lysine (a diamino carboxylic acid) were added to the reaction mixture. The reaction mixture was stirred for 2 hours at room temperature and then transferred to a 50,000 molecular weight cut-off polyethersulfone dialysis bag. Dialysis was performed for 24 hours against 2 changes of 4 liters of water.

### EXAMPLE 1

### Peptide-mediated uptake of SCNCs

Chariot (Active Motif, Carlsbad, CA) is a peptide reagent based on the HIV-tat sequence (Schwarze et al. (1999) Science 285:1569-1572), and has been used to deliver a variety of macromolecules into cells. Chariot forms a non-covalent complex with a molecule of interest (protein, peptide, antibody, or SCNC), and acts as a carrier to deliver molecules into cells.
To deliver SCNCs into cells using Chariot, tissue culture cells were seeded into six-well tissue culture plates (surface area of 962 mm² per well) at a cell density of 3 x 10⁵ cells per well and incubated overnight at 37°C in a 5% CO₂ atmosphere. The transfection efficiency was dependent on the percent confluency of cells; the optimal percent confluency for Chinese Hamster Ovary (CHO) cells was about 50-70%.

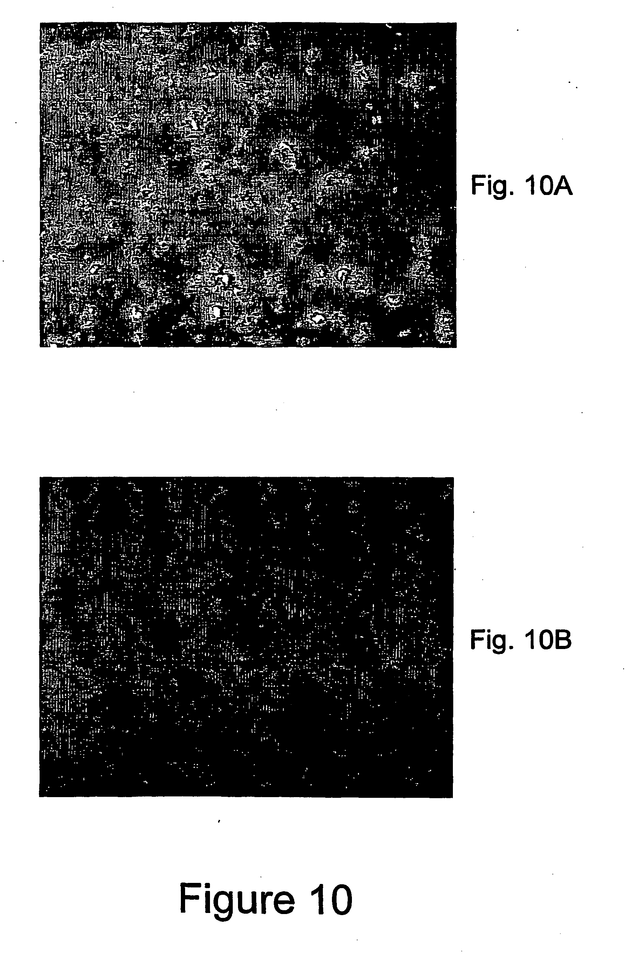

A transfection mixture was prepared by first diluting 616 nm emitting SCNCs into PBS in a final volume of 100 µl. The diluted SCNCs were combined with a mixture containing 94 µl sterile water and 6 µl Chariot reagent, and the 200 µl transfection mix was incubated at room temperature for 30 min. To transfect, cells were rinsed with PBS, and the 200 µl transfection mix was added directly to the cell monolayer, followed by 400 µl of serum-free growth medium. The final SCNC concentration ranged from 10 to 120 nM, depending on the cell type and SCNC material. The cells were incubated at 37 °C, 5% CO₂ for 1 hour, and 1 ml of serum containing growth medium was added to each well. The cells were allowed to incubate for an additional 2 hours. To visualize internalized SCNCs, the cells were analyzed by fluorescence microscopy (Fig. 10) or flow cytometry using the appropriate filter sets.

### EXAMPLE 2

### Nonspecific Uptake of SCNCs

SCNCs can be internalized by cells in the absence of a specific carrier molecule. Noncrosslinked polymer-coated SCNCs prepared as described above are sufficiently hydrophobic that they bind to cells and are taken up by nonspecific endocytotic pathways. Cells encoded with SCNCs were prepared as described in Example 1, except the Chariot reagent was omitted from the transfection mix. An example of nonspecific uptake of SCNCs is shown in Figure

### EXAMPLE 3

### Cationic lipid-mediated and micelle-mediated uptake of SCNCs

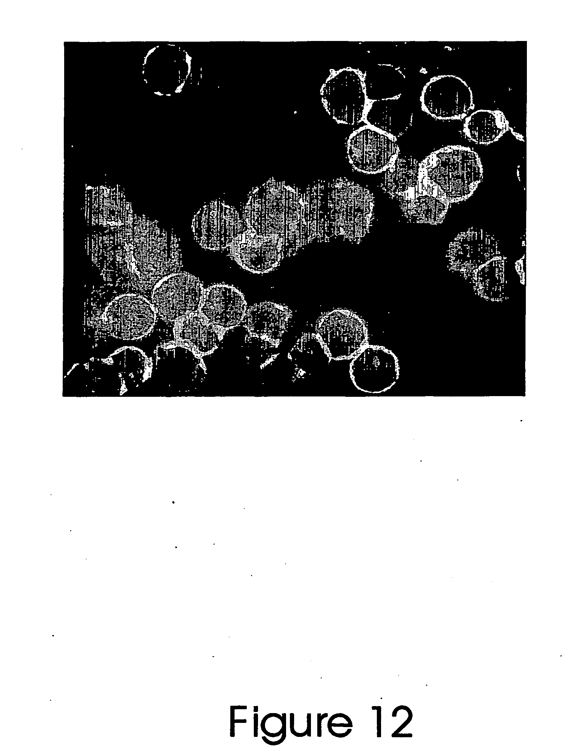

BioPORTER (BioPORTER, Gene Therapy Systems, San Diego, CA) is a cationic lipid that is similar to other lipid-based reagents for DNA transfections. It forms ionic interactions with negatively charged groups of a molecule (protein, peptide, antibody, or SCNC), and delivers the molecule into cells via fusion with the cell membrane. Cells were seeded at the same density as described in Example 1. A transfection mix, comprised of carboxylated SCNCs and PBS in a final volume of 100 µl, was added to a tube containing 10 µl of dried BioPORTER reagent. The solution was mixed gently by pipetting, incubated at room temperature for 5 minutes, and diluted by adding 900 µl of serum free medium. Cells were washed with PBS, and the diluted SCNC solution (1 ml) was added to the cell monolayer. The final SCNC concentration was 2-60 nM, depending on the cell line and SCNC material being tested. The cells were incubated at 37°C, 5% CO₂ for 3 hr, and internalized SCNCs were visualized by fluorescence microscopy (Fig. 12). Alternatively, 3 ml of serum-containing medium was added to each well and the cells were incubated overnight for analysis the next day.

### Micelle-mediated uptake of SCNCs

SCNCs were stabilized by entrapping phosphine/phosphine oxide ligands onto the surface of SCNCs with specific polymers through hydrophobic interaction. The most common ligands used in the synthesis of SCNCs are TOP and TOPO. TOP and TOPO bind to the surface Cd or Se through P-metal bond and their hydrophobic octyl chains are pointing toward solvent making the surface of SCNCs hydrophobic. Partially grafted poly(acrylic acid) (PAA), in which octylamines were attached to about 40% carboxyl groups of PAA through amide bond formation, were adsorbed onto the hydrophobic surface of SCNCs through hydrophobic interaction, leading water-soluble SCNCs. The remaining carboxyl groups can be used to conjugate to biological molecules or to be crosslinked with each other in order to make stable SCNCs.
In another method, the hydrophilic shell of micelles was chemically crosslinked, where the surface of the micelles is made up with carboxyl groups, which can then be used to form bioconjugates for biological applications. The amphiphilic block coploymer entraps or encapsulates SCNCs rendering the SCNCs water-soluble. The polymers can be diblock, triblock or multiblock copolymer, which contains at least one block of a hydrophobic segment and at least one block of hydrophilic segment. The surface of the micelles or functional groups in hydrophilic block of the block copolymer can be carboxyl, aldehyde, alcohol, amine or any reactive groups. The micelles encoded with one or more SCNCs were further stabilized through crosslinking of the hydrophilic shell. For micelle-mediated uptake of SCNC, cells and aqueous solutions of SCNCs trapped in micelles were prepared as described above. To transfect, a mixture comprised of SCNCs/micelles and serum free medium was prepared at a final volume of 500 us and incubated for 5 min at room temperature. Cells were washed with PBS, and the transfection mixture was added to the cell monolayer such that the final SCNC/micelle concentration was approximately 20 nM. Cells were incubated at 37°C, 5% CO₂ for 1 hr, and 1 ml of serum containing growth medium was added to each well. The cells were incubated for an additional 2 hr and analyzed. To analyze cells the following day, 2 ml of serum containing medium was added and the cells were incubated overnight. The incorporation of SCNCs into the cells was detected by fluorescence microscopy using a 535 nm emission filter or a 625 nm emission filter.

### EXAMPLE 4

### Co-delivery of DNA and SCNCs

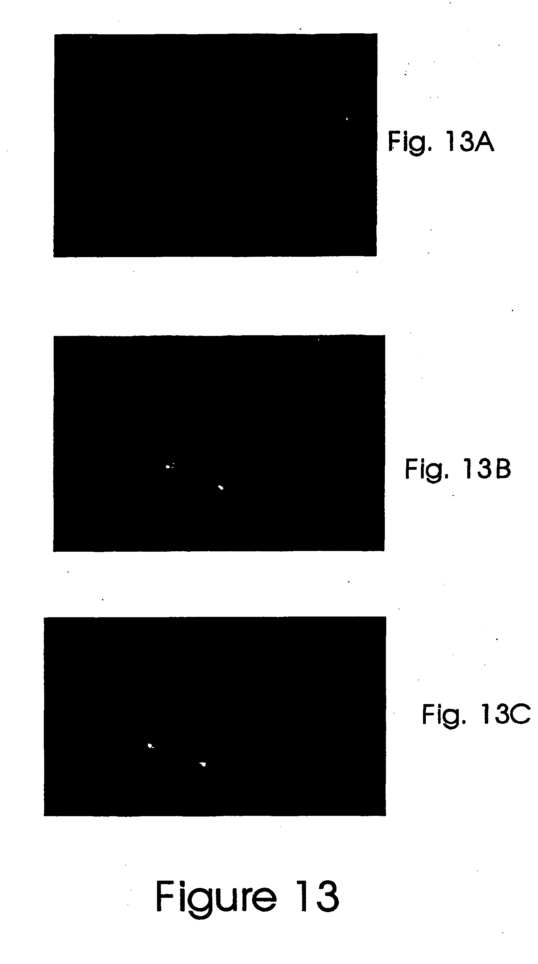

It is possible to co-deliver SCNCs and DNA using cationic lipids for encoded DNA transfection applications. A transfection solution comprised of 2 nM red (emitting at 630 nm) SCNC polymer cross-linked prepared as described above, and 3 µg of DNA carrying an EGFP (enhanced green fluorescent protein)/rac kinase fusion sequence was prepared and added to the BioPORTER reagent as described in Example 3. Cells were cultured 2 days for EFGP expression and analyzed by fluorescence microscopy and flow cytometry. The microscopy results show that it was possible to find cells that expressed EGFP and contained red SCNCs (Fig. 13). Control experiments using the EGFP fusion DNA or red SCNCs alone suggest that the DNA transfection efficiency decreased in the presence of SCNCs. This may be caused by SCNC competing with DNA for the BioPORTER reagent.

### EXAMPLE 5

### Decoding of SCNC-labeled Cells

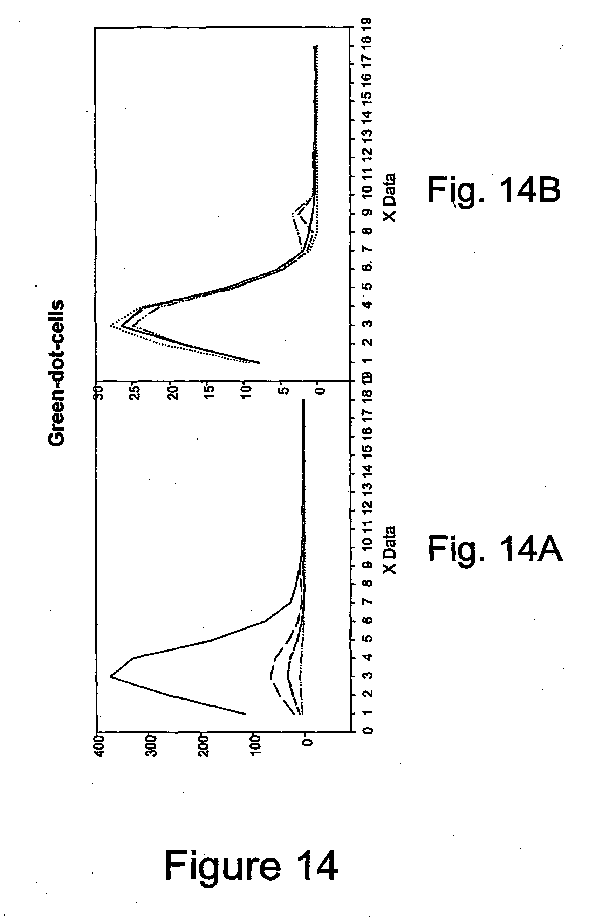

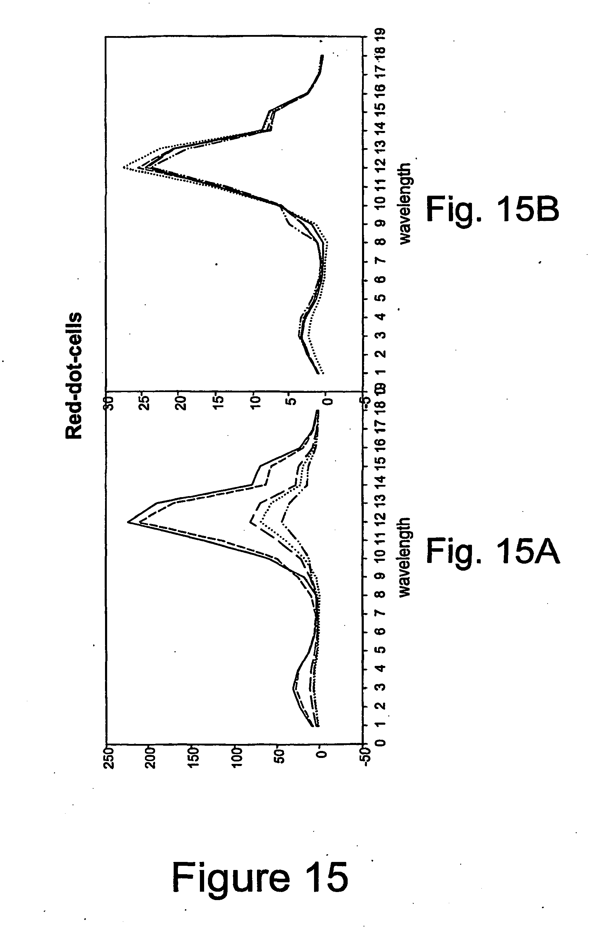

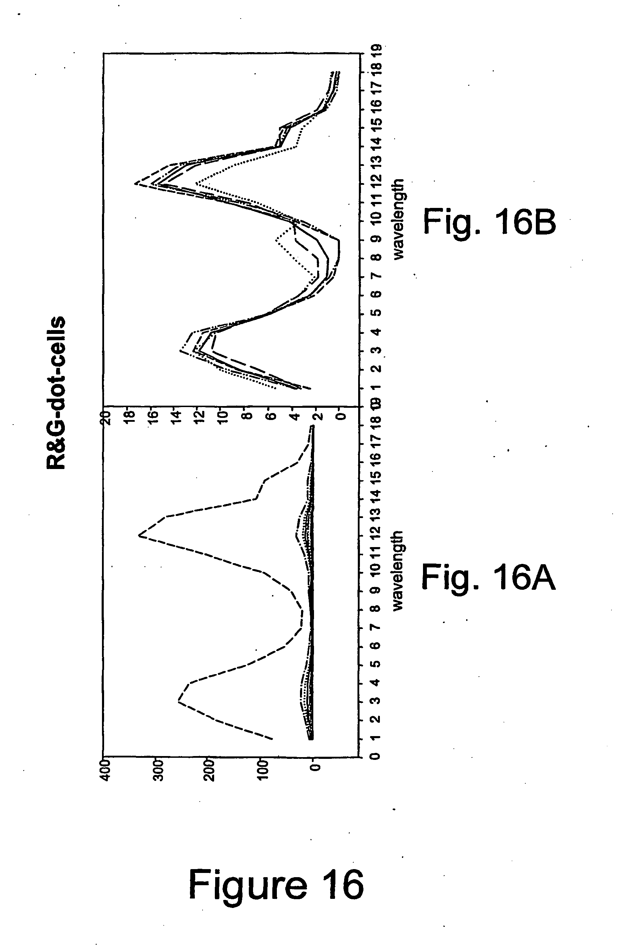

SCNC codes can be detected inside cells using the green (530 nm) or red (630 nm) crosslinked polymer-coated SCNCs prepared as described above were delivered into CHO cells as single colors or mixtures of two colors using the Chariot reagent. Individual cells were identified and analyzed over the range of 510 nm to 680 nm using an 18-filter set. The results show that for individual cells, absolute SCNC fluorescence intensity can vary more than 10-fold, but that normalized spectral patterns are very similar for green or red SCNCs (Figs. 14 and 15). Mixing green and red SCNCs prior to adding them to the cell monolayer results in cells that also have very similar spectral patterns after fluorescence normalization (Fig. 16). Thus, these results suggest that pattern recognition can be used as an encoding strategy for cells.

### EXAMPLE 6

### Encoding Multiple Cell Lines with SCNCs

A first cell line expressing a G-protein coupled receptor, e.g., a serotonin receptor, is taken into suspension and fixed in an appropriate fixative (e.g., 3% paraformaldehyde). A specific mixture of SCNCs having known fluorescence characteristics is used to encode this population of cells. A second cell line expressing a different G-protein coupled receptor, e.g., a beta adrenergic receptor, is encoded with a second spectral code in a similar manner. The first and second spectral codes have distinguishable fluorescence characteristics.

The separately encoded cells are then mixed together in the well of a microtiter plate and this mixed population is interrogated with a labeled ligand (labeled with either a fluorophore or a SCNC detectable different from the code) which may or may not bind to the G-protein coupled receptors on the cell lines. After an incubation period the encoded cells are allowed to settle to the bottom of the well and each encoded population of cells is measured to determine if label is associated with it using a scanning spectrometer based detection system.

### EXAMPLE 7

### Incorporation of SCNC into Yeast Mutant Cells

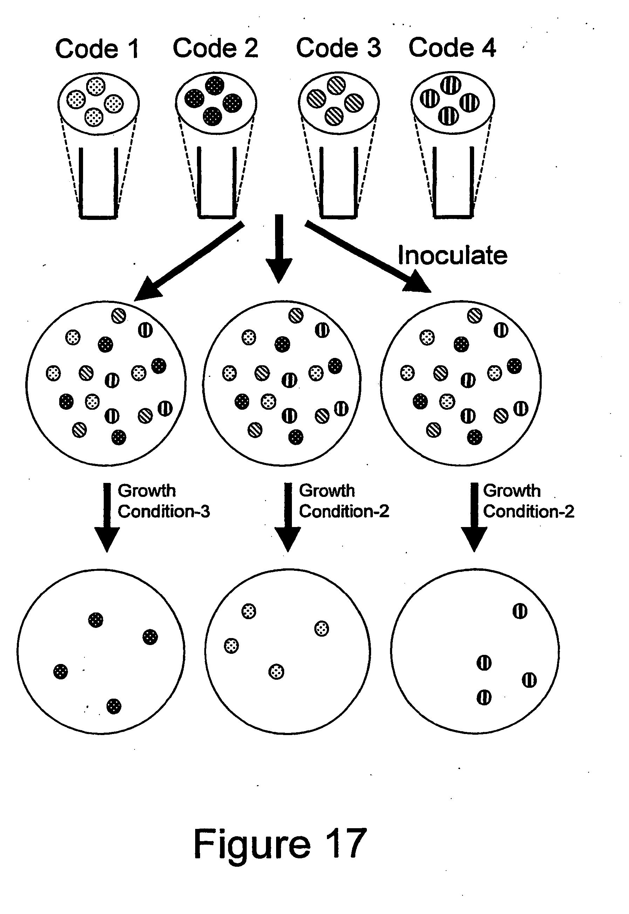

Populations of specific and distinct yeast mutants are permeabilized to introduce a specific color set of SCNCs. Each of the populations of yeast mutants are prepared with an SCNC code that is distinguishable from the other of the populations of yeast mutants (*see* Fig. 17). The encoded mutants are then used to inoculate a common plate containing a suitable growth medium. Several plates containing such mixed inocula of yeast mutants can be prepared.

Sets of inoculated plates are incubated under a chosen condition having altered temperature, light source, humidity or nutrient availability as compared to standard growth conditions. After an appropriate growth period (1 hour to 1 week) colonies which have formed can be spectrally decoded to identify the original mutant from which it derived.

### EXAMPLE 8

### Immunostaining of SCNC-encoded Cells: Herceptin Antibody Immunostaining of SKBR3 Cells

SKBR3 cells were seeded into an 8-well chamber slide at a density of 80,000 cells per well and encoded with green (530 nm) SCNCs using the cationic lipid BioPorter as described in Example 3. Encoded or unencoded cells were incubated overnight at 37° C, 5% CO₂, Cells were washed three times with PBS, and fixed in the presence of 3.7% formaldehyde for 10 minutes. The cells were washed 3 times with PBS, and incubated in the presence of PBS/1% bovine serum albumin (BSA) at room temperature for 30 minutes to minimize non-specific binding.

The cells in each well were incubated with 5 µg/ml herceptin antibody in PBS/1% BSA in a total volume of 150 µl for 30 min at room temperature. The cells were washed five times with PBS, and incubated with a 1:500 dilution of biotinylated goat anti-human IgG (Vector Laboratories, 1.5 mg/ml) for 30 minutes. The cells were washed again with PBS and incubated with a 1:400 dilution of streptavidin-conjugated Cy3 (Amersham, 1 mg/ml) for 30 minutes. The cells were washed again with PBS and the slide was mounted using 50% glycerol in PBS. The cells were imaged using a Nikon fluorescence microscope equipped with a Cy3 and green SCNC filter set. Control experiments indicate that binding of herceptin is unaffected by the SCNC code. The results indicate that the blinding of herceptin is unaffected by the SCNC encoding process or by the presence of intracellular SCNC.

### EXAMPLE 9

### Immunostaining of SCNC-encoded Cells: Encoded Anti-tubulin Immunostaining of CHO Cells

Chinese hamster ovary (CHO) cells were seeded into an 8-well chamber slide at a density of 15,000 cells per chamber. The cells were encoded with green (530 nm) SCNCs dots using Chariot reagent as described in Example 1. The cells were incubated overnight in complete medium (DMEM-F12, 10% fetal bovine serum (FBS), 2 mM L-glutamine). Cells were washed 3 times with PBS, and fixed with 3.7% formaldehyde in PBS at room temperature for 10 minutes. The cells were washed 4 times with PBS, and incubated for 30 minutes at room temperature in the presence of PBS/1% bovine serum albumin (BSA). Anti-tubulin antibody (rabbit IgG fraction, whole de-lipidized antsera, Sigma) was diluted 1:200 in PBS/1% BSA and incubated with cells at room temperature for 30 minutes. The cells were washed 5 times with PBS, and incubated with biotinylated goat anti rabbit IgG (Vector Laboratories, Burlingame,CA. stock is 1.5 mg/ml) at a 1:500 dilution in PBS/1% BSA for 30 minutes. The cells were washed 5 times with PBS and incubated with streptavidin conjugated Cy3 (Amersham, 1 mg/ml stock solution) diluted 1:400 in PBS for 30 minutes. The cells were washed 5 times with PBS and the slide was mounted using 50% glycerol in PBS. Cells were imaged using a Nikon fluorescence microscope equipped with a Cy3 and green SCNC filter set. The results indicate that the binding of anti-tubulin is unaffected by the SCNC encoding process or by the presence of intracellular SCNC.

### EXAMPLE 10

### A Reporter Gene Assay for the β2 Adrenergic Receptor Using SCNC-encoded CHO Cells

Chinese hamster ovary (CHO) cells expressing the β2 adrenergic receptor and *renilla* luciferase reporter gene were encoded with green (530 nm) SCNCs using the Chariot reagent as described in Example 1. Encoded cells or unencoded cells were seeded into the wells of a white, clear-bottom, 96-well plate at a seeding density of 100,000 cells per well. The cells were incubated overnight in complete medium (DMEM-F12, 10% fetal bovine serum (FBS), 2 mM L-glutamine, and 1 mg/ml G418 (Gibco BRL)).

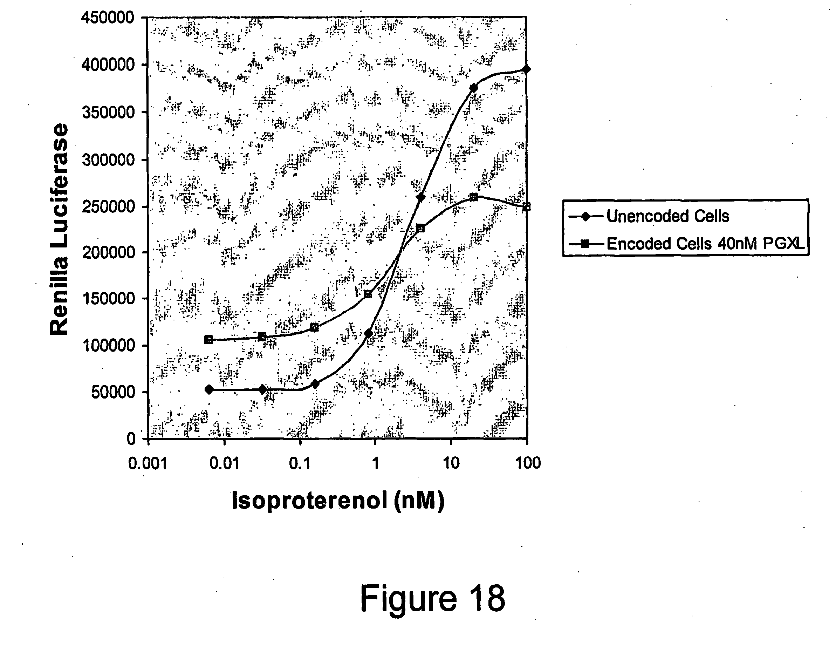

The cells were washed and starved for 20-24 hrs by incubating them in DMEM-F12 medium lacking serum and phenol red. The beta receptor agonist isoproterenol (Sigma) was diluted at various concentrations in DMEM-F12 medium and incubated with cells for four hours. To measure expression of the reporter gene, cells were washed with PBS and assayed for *renilla* luciferase activity using the RenLuc kit (Promega). Luminescence was measured using a Tecan SpectraFluor Plus plate reader. The dose response curves shown in Fig. 18 indicate that the EC₅₀ values for encoded or unencoded cells are nearly identical, but that the encoded cells have a smaller signal dynamic range.

### EXAMPLE 11

### An Fluorescence Competition Binding Assay for the β2 Adrenergic Receptor Using SCNC-Encoded CHO Cells

CHO cells expressing the β2 adrenergic receptor were encoded with green (530 nm) SCNCs using Chariot reagent as described in Example 1. Encoded or unencoded cells were seeded into 8-well chamber slides at a density of 40,000 cells per chamber. The cells were incubated overnight in complete medium.

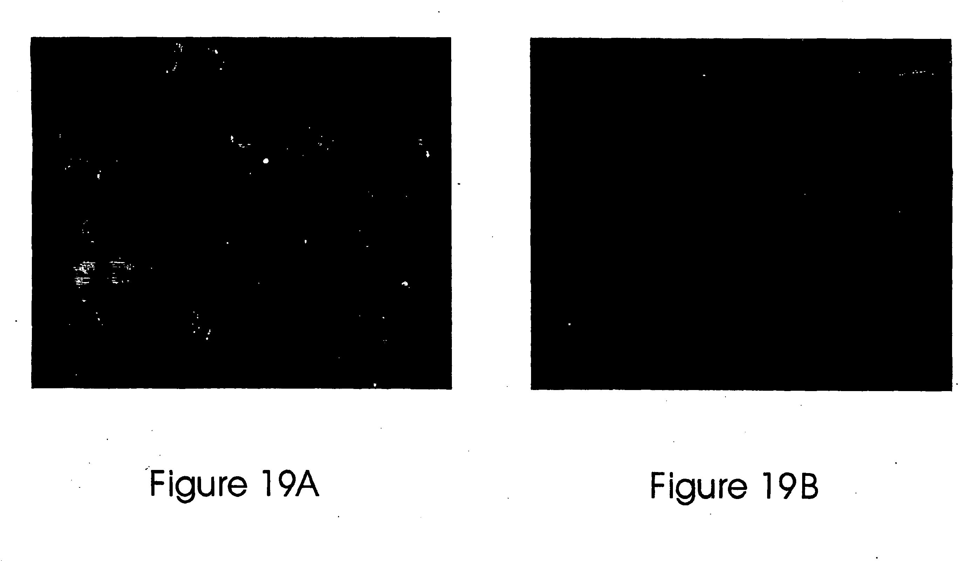

The chamber slides were chilled at 4° C for approximately 20 minutes, and the cells were washed once with cold binding buffer (serum- and phenol red-free DMEM-F12 supplemented with 0.1% BSA). The cells were incubated in binding buffer in the presence or absence of 1 µM unlabeled CGP12177 ligand (Sigma). The slides were incubated at 4° C for 30 minutes. To bind the fluorescent ligand, BODIPY® TMR (±) CGP 12177 (Molecular Probes) was added to a final concentration of 250 nM in binding buffer, and the slides were wrapped in aluminum foil and incubated at 4°C for 1 hour. Each well was washed 4 times with binding buffer and the slides were mounted with 50% glycerol in PBS. Cells were imaged using a Nikon fluorescence microscope equipped with a Cy3 and green SCNC filter set (Fig. 19). Control experiments indicate that competition binding of CGP12177 is essentially the same for either encoded or unencoded cells.

### EXAMPLE 12

### A Calcium Assay for the M1 Muscarinic Receptor Using SCNC-Encoded CHO Cells

CHO cells expressing the M1 muscarinic receptor M1 WT3 (American Type Culture Collection, catalog number CRL-1985) were encoded with green (530 nm) SCNCs using the Chariot reagent as described in Example 1. Encoded or unencoded cells were seeded into the wells of a 96-well assay plate at a density of 10,000 cells per well and grown overnight in complete medium (Ham's F12K, 10% fetal bovine serum (FBS), 2 mM L-glutamine). A calcium dye loading solution using the FLEXstation calcium assay kit (Molecular Devices) was prepared according the manufacturer's directions. The loading buffer was supplemented with 2.5 mM probenecid to inhibit anion-exchange proteins and prevent loss of internalized dye. To load cells with the calcium indicator dye, 100 µl of loading solution is added to 100 µl of medium per well, and the plate was incubated at 37° C, 5% CO₂ for 1 hr.

The plate was removed and placed on the microscope-based system for visualizing fluorescent images described above. Compounds were diluted in complete medium and added to the wells. The plate was incubated at room temperature for 5 minutes, and the cells were imaged as described in Example 5. The results indicate that the agonist carbachol can stimulate the calcium response of either unencoded or encoded cells.

### EXAMPLE 13

### A GPCR Internalization Assay for Multiplex Screening of Agonist or Antagonist Ligands Using SCNC-encoded Cells

A method is described for encoding and multiplexing a GPCR internalization assay. Many, if not all, GPCRs undergo agonist-dependent aggregation on the cell surface and subsequent internalization via clathrin coated pits. The internalized GPCR is contained within an endosome, which is either recycled back to the membrane or targeted to the lysosome for degradation. An assay, based on visualizing the movement of a fluorescent-tagged receptor from the cell surface to an endosomal compartment, has been shown for several GPCRs, including the parathyroid hormone receptor (Conway et al. (1999) J. Biomol. Screening 4(2):75-86), cholecystokinin receptor type A (Tarasova et al. (1997) J. Biol. Chem. 272(23): 14817-24) and β2 adrenergic receptor (Kallal et al. (1998) J. Biol. Chem. 273(1):322-8). A receptor chimera, comprised of green fluorescent protein (GFP) fused to the cytoplamic C-terminal tail of the GPCR, can be used to visualize receptor trafficking. It should also be possible, however, to tag the GPCR with a short epitope sequence displayed on one of its extracellular loops, and to label the receptor with an anti-epitope antibody conjugated to a fluorescent dye molecule or SCNC. Examples of such epitope sequences include the eight amino acid sequence FLAG peptide (Chubet et al. (1996) Biotechniques 20(1): 136-41), or the nine amino acid sequence influenza virus hemagglutinin (HA) peptide (Koller et al. (1997) Anal. Biochem. 250(1):51-60)*.*

To multiplex an internalization assay using epitope-tagged GPCRs, cell lines expressing various GPCRs are encoded as described in, e.g., Example 1, 2 or 3, and mixed. The mixed cells are added to the wells of a clear bottom assay plate. The fluorescent dye-labeled antibody is added, followed by the compound. The cells are incubated at 37° C for 30-60 minutes, and the assay plate and cells are imaged using a fluorescence microscope. Alternatively, the cells can be fixed with paraformaldehyde or some other fixative agent, and the plates are stored at 4° C for imaging at a later time. Binding of an agonist ligand to the GPCR will cause internalization of the GPCR and its bound antibody, which can be visualized under the microscope as a movement of fluorescence from the cell surface to an intracellular compartment. To screen for antagonists, the compounds are screened for their ability to block the agonist-dependent internalization of the receptor. This method can also be used to screen for agonist ligands of orphan GPCRs.

### EXAMPLE 14

### A Method for Encoding and Assaying Cells Grown in a Macroporous Gelatin Microcarrier

A method is described for encoding and screening cells grown on a microcarrier bead surface. An example of such a microcarrier is CultiSpher^{™} from HyClone Laboratories, Inc. CultiSpher^{™} is a macroporous gelatin microcarrier bead that provides a very large interior surface for cell attachment. The large surface-to-area ratio of the beads results in much higher cell yields compared to conventional liquid cell cultures.

Microcarrier beads can be encoded using chemical methods (see*, e.g.,* U.S. Patent No. 6,207,392, PCT Publication No. WO 00/17103, and Han et al. (2001) Nature Biotech. 19:632-635) and then used as a substrate on which to grow cells. An advantage of this method is that encoding is done on the bead scaffold used to grow the cells, and not on individual cells. Methods for encoding the beads include adsorbing a unique SCNC code to the bead surface, or encapsulating the code within the interior of the bead.

To perform a multiplex assay using this method, the microcarrier beads are encoded and stored until ready for use. Cells expressing a receptor target of interest are added to the encoded beads and incubated in culture medium to allow cell attachment. The beads on which each cell line have been grown are combined, and aliquots of the mixture are added to the wells of an assay plate.

There are a variety of assays that can be adapted for use with encoded microcarrier beads and cells. For example, binding of a fluorescent ligand to a cell surface receptor can be measured by flow cytometry of the microcarrier/cell complexes. Fluorescence microscopy can be used to image calcium flux or expression of a reporter gene using cells grown on microcarrier beads.

### EXAMPLE 15

### A Method for Screening 600 GPCRs Using a 10-plex SCNC-encoded Cell Assay

A method is described for screening 600 GPCRs against 96 compounds using a 10-plex encoded cell assay.

The compounds from a 96-well compound plate are replica plated to 60 96-well daughter plates. Alternatively, a 20-plex assay would require 30 compound daughter plates, and a 60-plex assay would only require 10 daughter plates. For a 10-plex assay, the 60 daughter plates are divided into 6 groups of 10 plates each.

The 600 GPCR cell lines are stored as frozen cells, and are thawed as needed. An important advantage of this method is that far fewer cells are used for screening compared to conventional screening methods. For example, fewer than 100 cells per GPCR are screened in a well using encoded cell technology, compared to 50,000 cells per GPCR using a conventional calcium assay such as the Fluorescent Imaging Plate Reader (FLIPR) from Molecular Devices. Therefore, all of the GPCR cell lines required for the encoded cell technology can be grown in 6-well culture plates compared to the large flasks or bioreactors that are required for conventional screening. Growing the cells in 6-well culture plates is also more amenable to automation compared to conventional screening.

To encode the 600 GPCR cell lines, the cells are transferred to 100 6-well culture plates and grown to a cell density of about 0.5-1.0 x 10⁶ cells per well. The 600 wells are organized according to the multiplexing capability of the assay. For example, a 10-plex assay would require that 600 wells be organized into 60 groups, each group comprised of 10 wells, while a 60-plex assay would be organized as 10 groups of 60 wells each. The cell codes are stored as premixed color combinations of SCNCs, and are used as previously described. The number of SCNC colors necessary for a 10-plex, 20-plex, and 60-plex assays using single emission intensity levels are 4,5, and 6, respectively.

For a 10-plex assay, the cell lines growing in 10 different wells are encoded with one of the 10 SCNC codes as described, for example, in Examples 1 and 3. The cells are encoded, lifted from each of the 10 wells, counted, and pooled such that the number of cells comprising each GPCR cell line is approximately equal. For example, pooling 0.5 x 10⁶ cells from each well would result in a mixture containing a total of 5 x 10⁶ cells. Cells from the mix are distributed to all the wells of a 96-well assay plate at a seeding density of 10,000 total cells per well (equivalent to 1000 cells per GPCR cell line). This process is repeated 60 times until all 600 GPCR cell lines are contained within the wells of 60 assay plates.

This screening method can be adapted to a variety of assay formats. One such assay is the GPCR internalization assay described above using epitope-tagged GPCRs. The anti-epitope fluorescent antibody is added to the wells of the 60 assay plates. Compounds from the 60 compound replica plates are transferred to the assay plates, and the plates are incubated at 37° C, 5% CO₂ for 30-60 minutes for receptor internalization to occur. The cells are fixed with paraformaldehyde and stored at 4° C until ready for imaging.

The cells are imaged using an automated high-throughput fluorescence microscope. A nuclear stain such as Hoechst 33258 (350 nm excitation, 461 nm emission) is used to identify single cells within a field of view. To screen for agonist compounds, the cells are screened for the internalization of the fluorescent reporter bound to the antibody. Positive cells are then scanned using multiple filters to determine the SCNC code. This process is repeated at either single or multiple fields of view per well until a statistically significant number of data points are collected. Image and data processing are used to store and analyze the data.

## Claims

1. A method of distinguishably identifying a population of cells in a mixture of different types of cells, comprising:
providing a cell, contacting the cell with a semiconductor nanocrystal (SCNC) under conditions in which the semiconductor nanocrystal is associated with the cell to provide a distinguishably labeled cell, and identifying in a mixture of different types of cells a population of cells derived from the labeled cell via their unique spectral code after it has multiplied in the mixture.

2. A method of identifying a cell in a mixed population of cells, comprising mixing a composition comprising a cell encoded with a semiconductor nanocrystal (SCNC) with a cell distinct therefrom to form a mixed population, culturing the mixed population, such that there is derived from the encoded cell a population of cells after several cell divisions, applying an excitation source to the mixed population, and detecting the SCNC via its unique spectral code to identify the encoded cells.

3. A method of claim 1 which comprises screening modulators of a receptor mediated response in the labeled population of cells, the method comprising:
a) contacting the labeled cells with a predetermined concentration of a compound to be tested;
b) detecting a signal from the cells thereby identifying the cells;
c) detecting the receptor mediated response; and
d) comparing the response in (c) with the response produced in the absence of the compound thereby identifying the compound as a modulator of the receptor mediated response.

4. The method of claim 3, wherein the detecting comprises photochemical means, or spectroscopic means or flow cytometry.

5. The method of any one of claims 1 to 4 wherein said cell is selected from the group consisting of a yeast cell, an amphibian cell, a mammalian cell and a plant cell.

6. The method of claim 5, wherein the cell is a mammalian cell selected from the group consisting of a human cell, a mouse cell, a rat cell, a bovine cell, and a hamster cell.

7. The method of any preceding claim, wherein said cell comprises an intracellular semiconductor nanocrystal.

8. The method of any of claims 1 to 6, wherein said cell comprises an extracellular semiconductor nanocrystal.

9. The method of any of claims 1 to 6, wherein said cell comprises a membrane-associated semiconductor nanocrystal.

10. The method of any preceding claim, wherein the semiconductor nanocrystal comprises a core of a first semiconductor material that is contained within a shell of a second semiconductor material.

11. The method of claim 10, wherein the core is selected from the group consisting of ZnS, ZnSe, ZnTe, CdS, CdSe, CdTe, HgS, HgSe, HgTe, MgS, MgSe, MgTe, CaS, CaSe, CaTe, SrS, SrSe, SrTe, BaS, BaSe, BaTe, GaN, GaP, GaAs, GaSb, InN, InP, InAs, InSb, AlAs, AlP, AlSb, AlS, Ge, Si, Pb, PbS, PbSe, an alloy thereof, and a mixture thereof, and is preferably CdSe.

12. The method of claim 11, wherein the shell is ZnS.

13. The method of claim 1 or claim 3, or the method of any of claims 5 to 12 when directly or indirectly dependent on claim 1, wherein the conditions comprise forming pores in said cell, for example by contacting the cell with a porogen.

14. The method of claim 1 or claim 3, or the method of any of claims 5 to 12 when directly or indirectly dependent on claim 1, wherein the conditions comprise contacting said cell with an SCNC-containing micelle.

15. The method of claim 1 or claim 3, or the method of any of claims 5 to 12 when directly or indirectly dependent on claim 1, wherein the conditions comprise microinjection.

16. The method of any of claims 1 to 12, wherein the semiconductor nanocrystal is linked to a ligand capable of localizing the SCNC to a subcellular component.

17. The method of any of claims 1 to 12, wherein the semiconductor nanocrystal is linked to:
(a) a ligand capable of binding specifically to a cell-surface receptor; or
(b) a conjugating agent which is capable of specifically attaching to a cell-surface molecule.

18. The method of any of claims 1 to 12, wherein the semiconductor nanocrystal is in a micelle.

19. The method of claim 18, wherein said micelle is formed from a phosphatide.

20. The method of claim 19, wherein said phosphatide is a branched glycerol wherein two of the hydroxyl groups of said glycerol are esterified with residues from saturated fatty acids of C10-C20 and at least one of the carbon atoms has an alkyl group.

21. The method of claim 1, wherein the conditions comprise endocytosis.

## Patentansprüche

1. verfahren zum unterscheidbaren Identifizierten einer Population von Zellen in einem Gemisch aus verschiedenen Zelltypen, umfassend:
Bereitstellen einer Zelle, Inkontaktbringen der Zelle mit einem Halbleiternanokristall (SCNC) unter Bedingungen, bei denen der Halbleiternanokristall mit der Zelle assoziiert ist, um eine unterscheidbar markierte Zelle bereitzustellen, und Identifizierten einer von der markierten Zelle erhaltenen Population von Zellen in einem Gemisch aus verschiedenen Zelltypen anhand ihrer einzigartigen Spektralcodes, nachdem diese sich in dem Gemisch vermehrt hat.

2. Verfahren zum Identifizierten einer Zelle in einer gemischten Population von Zellen, umfassend das Mischen einer Zusammensetzung, die eine mit einem Halbleiternanokristall (SCNC) kodierte Zelle umfasst, mit einer davon getrennten Zelle unter Bildung einer gemischten Population, Kultivieren der gemischten Population, sodass von der kodierten Zelle eine Population von Zellen nach mehreren Zellteilungen erhalten wird, Anliegen einer Anregungsquelle an die gemischte Population, und Detektieren des SCNC anhand ihrer einzigartigen Spectralcodes, um die kodierten Zellen zu identifizieren.

3. Verfahren nach Anspruch 1, welches das Durchmustern von Modulatoren einer rezeptorvermittelten Antwort in der markierten Population von Zellen umfasst, wobei das Verfahren Folgendes umfasst:
a) Inkontaktbringen der markierten Zellen mit einer vorherbestimmten Konzentration einer zu untersuchenden Verbindung;
b) Detektieren eines Signals von den Zellen, wodurch die Zellen identifiziert werden;
c) Detektieren der rezeptorvermittelten Antwort; und
d) Vergleichen der Antwort in c) mit der in Abwesenheit der Verbindung erzeugten Antwort, wodurch die Verbindung als Modulator der rezeptorvermittelten Antwort identifiziert wird.

4. Verfahren nach Anspruch 3, wobei das Detektieren fotochemische Mittel oder spektroskopische Mittel oder Durchflusszytometrie umfasst.

5. Verfahren nach einem beliebigen der Ansprüche 1 bis 4, wobei die Zelle ausgewählt ist aus der Gruppe, bestehend aus einer Hefezelle, einer Amphibienzelle, einer Säugerzelle und einer Pflanzenzelle.

6. Verfahren nach Anspruch 5, wobei die Zelle eine Säugerzelle ist, die ausgewählt ist aus der Gruppe, bestehend aus einer humanen Zelle, einer Mäusezelle, einer Rattenzelle, einer Rinderzelle und einer Hamsterzelle.

7. Verfahren nach einem beliebigen vorangehenden Anspruch, wobei die Zelle einen intrazellulären Halbleiternanokristall umfasst.

8. Verfahren nach einem beliebigen der Ansprüche 1 bis 6, wobei die Zelle einen extrazellulären Halbleiternanokristall umfasst.

9. Verfahren nach einem beliebigen der Ansprüche 1 bis 6, wobei die Zelle einen membranassoziierten Halbleiternanokristall umfasst.

10. Verfahren nach einem beliebigen vorangehenden Anspruch, wobei der Halbleiternanokristall einen Kern aus einem ersten Halbleitermaterial umfasst, der in einer Schale aus einem zweiten Halbleitermaterial enthalten ist.

11. Verfahren nach Anspruch 10, wobei der Kern ausgewählt ist aus der Gruppe, bestehend aus ZnS, ZnSe, ZnTe, CdS, CdSe, CdTe, HgS, HgSe, HgTe, MgS, MgSe, MgTe, CaS, CaSe, CaTe, SrS, SrSe, SrTe, BaS, BaSe, BaTe, GaN, GaP, GaAs, GaSb, InN, InP, InAs, InSb, AlAs, AlP, AlSb, AIS, Ge, Si, Pb, PbS, PbSe, einer Legierung davon und einem Gemisch davon, und vorzugsweise CdSe ist.

12. Verfahren nach Anspruch 11, wobei die Schale ZnS ist.

13. Verfahren nach Anspruch 1 oder Anspruch 3, oder Verfahren nach einem beliebigen der Ansprüche 5 bis 12, sofern er direkt oder indirekt von Anspruch 1 abhängig ist, wobei die Bedingungen das Bilden von Poren in der Zelle, zum Beispiel durch Inkontaktbringen der Zelle mit einem Porenbildner, umfasst.

14. Verfahren nach Anspruch 1 oder Anspruch 3 oder Verfahren nach einem beliebigen der Ansprüche 5 bis 12, sofern er direkt oder indirekt von Anspruch 1 abhängig ist, wobei die Bedingungen das Inkontaktbringen der Zelle mit einer SCNC-enthaltenden Mizelle umfassen.

15. Verfahren nach Anspruch 1 oder Anspruch 3 oder Verfahren nach einem beliebigen der Ansprüche 5 bis 12, sofern er direkt oder indirekt von Anspruch 1 abhängig ist, wobei die Bedingungen Mikroinjektion umfassen.

16. Verfahren nach einem beliebigen der Ansprüche 1 bis 12, wobei der Halbleiternanokristall mit einem Liganden verknüpft ist, der in der Lage ist, den SCNC auf eine subzelluläre Komponente zu lokalisieren.

17. Verfahren nach einem beliebigen der Ansprüche 1 bis 12, wobei der Halbleiternanokristall verknüpft ist mit:
a) einem Liganden, der in der Lage ist, spezifisch an einen Zelloberflächenrezeptor zu binden; oder
b) einem Konjugationsmittel, das in der Lage ist, sich spezifisch an ein Zelloberflächenmolekül anzuheften.

18. Verfahren nach einem beliebigen der Ansprüche 1 bis 12, wobei der Halbleiternanokristall eine Mizelle ist.

19. Verfahren nach Anspruch 18 wobei die Mizelle aus einem Phosphatid gebildet wird.

20. Verfahren nach Anspruch 19, wobei das Phosphatid ein verzweigtes Glycerin ist, wobei zwei der Hydroxylgruppen des Glycerins mit Resten von gesättigten Fettsäuren von C₁₀-C₂₀ esterifiziert sind und mindestens eines der Kohlenstoffatome eine Alkylgruppe aufweist.

21. Verfahren nach Anspruch 1, wobei die Bedingungen eine Endocytose umfassen.

## Revendications

1. Procédé pour identifier de manière discernable une population de cellules dans un mélange de différents types de cellule, comprenant les opérations consistant à _{:}
prendre une cellule, mettre en contact la cellule avec un nanocristal semiconducteur (SCNC) dans des conditions dans lesquelles le nanocristal semi-conducteur est associé avec la cellule pour fournir une cellule marquée de manière discernable, et identifier dans un mélange de différents types de cellules une population de cellules issues de la cellule marquée par l'intermédiaire de leur code spectral unique après qu'elle se soit multipliée dans le mélange.

2. Procédé d'identification d'une cellule dans une population mixte de cellules, comprenant les opérations consistant à mélanger une composition comprenant une cellule codée par un nanocristal semiconducteur (SCNC) avec une cellule distincte de celle-ci pour former une population mixte, cultiver la population mixte, de telle sorte qu'une population de cellules est issue de la cellule codée après plusieurs divisions cellulaires, appliquer une source d'excitation à la population mixte, et détecter le SCNC par l'intermédiaire de son code spectral unique pour identifier les cellules codées.

3. Procédé selon la revendication 1, qui comprend le criblage de modulateurs d'une réponse à médiation par récepteur dans la population de cellules marquée, le procédé comprenant les opérations consistant à :
(a) mettre en contact les cellules marquées avec une concentration prédéterminée d'un composé devant être testé ;
(b) détecter un signal provenant des cellules, permettant ainsi d'identifier les cellules ;
(c) détecter la réponse à médiation par récepteur ; et
(d) comparer la réponse en (c) avec la réponse produite en l'absence du composé, permettant ainsi d'identifier le composé comme modulateur de la réponse à médiation par récepteur.

4. Procédé selon la revendication 3, dans lequel la détection comprend des moyens photochimiques, ou des moyens spectroscopiques ou une cytométrie en flux.

5. Procédé selon l'une quelconque des revendications 1 à 4, dans lequel ladite cellule est choisie dans le groupe constitué par une cellule de levure, une cellule d'amphibien, une cellule de mammifère et une cellule végétale.

6. procédé selon la revendication 5, dans lequel la cellule est une cellule de mammifère choisie dans le groupe constitué par une cellule humaine, une cellule de souris, une cellule de rat, une cellule bovine et une cellule de hamster.

7. Procédé selon l'une quelconque des revendications précédentes, dans lequel ladite cellule comprend un nanocristal semiconducteur intracellulaire.

8. Procédé selon l'une quelconque des revendications 1 à 6, dans lequel ladite cellule comprend un nanocristal semiconducteur extracellulaire.

9. Procédé selon l'une quelconque des revendications 1 à 6, dans lequel ladite cellule comprend un nanocristal semiconducteur associé à une membrane.

10. Procédé selon l'une quelconque des revendications précédentes, dans lequel le nanocristal semiconducteur comprend un coeur d'un premier matériau semiconducteur qui est contenu à l'intérieur d'une écorce d'un second matériau semiconducteur.

11. Procédé selon la revendication 10, dans lequel le coeur est choisi dans le groupe constitué par ZnS, ZnSe, ZnTe, CdS, CdSe, CdTe, HgS, HgSe, HgTe, MgS, MgSe, MgTe, CaS, CaSe, CaTe, SrS, SrSe, SrTe, BaS, BaSe, BaTe, GaN, GaP, GaAs, GaSb, InN, InP, InAs, InSb, AlAs, AlP, AlSb, AlS, Ge, Si, Pb, PbS, PbSe, un alliage de ceux-ci et un mélange de ceux-ci, et est, de préférence, du CdSe.

12. Procédé selon la revendication 11, dans lequel l'écorce est du ZnS.

13. Procédé selon la revendication 1 ou la revendication 3, ou procédé selon l'une quelconque des revendications 5 à 12, considérée dans la dépendance directe ou indirecte de la revendication 1, dans lequel les conditions comprennent la formation de pores dans ladite cellule, par exemple par mise en contact de la cellule avec un porogène.

14. Procédé selon la revendication 1 ou la revendication 3, ou procédé selon l'une quelconque des revendications 5 à 12, considérée dans la dépendance directe ou indirecte de la revendication 1, dans lequel les conditions comprennent la mise en contact de ladite cellule avec une micelle contenant un SCNC.

15. Procédé selon la revendication 1 ou la revendication 3, ou procédé selon l'une quelconque des revendications 5 à 12, considérée dans la dépendance directe ou indirecte de la revendication 1, dans lequel les conditions comprennent une micro-injection.

16. Procédé selon l'une quelconque des revendications 1 à 12, dans lequel le nanocristal semiconducteur est lié à un ligand capable de localiser le SCNC à un composant subcellulaire.

17. Procédé selon l'une quelconque des revendications 1 à 12, dans lequel le nanocristal semiconducteur est lié à :
(a) un ligand capable de se lier de façon spécifique à un récepteur de surface cellulaire ; ou
(b) un agent de conjugaison qui est capable de se fixer de façon spécifique à une molécule de surface cellulaire.

18. Procédé selon l'une quelconque des revendications 1 à 12, dans lequel le nanocristal semiconducteur est dans une micelle.

19. Procédé la revendication 18, dans lequel ladite micelle est formée à partir d'un phosphatide.

20. Procédé selon la revendication 19, dans lequel ledit phosphatide est un glycérol ramifié, deux des groupes hydroxyle dudit glycérol étant estérifiés par des restes d'acides gras saturés en C10-C20, et au moins l'un des atomes de carbone ayant un groupe alkyle.

21. Procédé selon la revendication 1, dans lequel les conditions comprennent une endocytose.
